(19) Europäisches Patentamt
European Patent Office
Office européen des brevets

(11) EP 2 013 232 B1

(12) **FASCICULE DE BREVET EUROPEEN**

(45) Date de publication et mention de la délivrance du brevet:
**20.05.2015 Bulletin 2015/21**

(21) Numéro de dépôt: **07731382.3**

(22) Date de dépôt: **27.04.2007**

(51) Int Cl.:
***C07K 14/16*** *(2006.01)*

(86) Numéro de dépôt international:
**PCT/FR2007/000730**

(87) Numéro de publication internationale:
**WO 2007/125210 (08.11.2007 Gazette 2007/45)**

# (54) UTILISATION DE LIGANDS SYNTHETIQUES MULTIVALENTS DE LA NUCLEOLINE DE SURFACE POUR LE TRAITEMENT DU CANCER OU DE L'INFLAMMATION

VERWENDUNG MEHRWERTIGER SYNTHETISCHER LIGANDEN VON OBERFLÄCHENNUKLEOLIN ZUR BEHANDLUNG VON KREBS ODER ENTZÜNDUNGEN

USE OF MULTIVALENT SYNTHETIC LIGANDS OF SURFACE NUCLEOLIN FOR TREATING CANCER OR INFLAMMATION

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **27.04.2006 FR 0603813**

(43) Date de publication de la demande:
**14.01.2009 Bulletin 2009/03**

(60) Demande divisionnaire:
**12185309.7 / 2 540 737**

(73) Titulaire: **Centre National de la Recherche Scientifique (CNRS)**
**75016 Paris (FR)**

(72) Inventeurs:
- **COURTY, José**
  **94440 Villecresnes (FR)**
- **HOVANESSIAN, Ara**
  **92340 Bourg la Reine (FR)**
- **BRIAND, Jean Paul**
  **6700 Strasbourg (FR)**
- **GUICHARD, Gilles**
  **67202 Wolfisheim (FR)**
- **HAMMA, Yamina**
  **94270 Le Kremblin Bicetre (FR)**

(74) Mandataire: **Regimbeau**
**20, rue de Chazelles**
**75847 Paris Cedex 17 (FR)**

(56) Documents cités:
**WO-A-95/29190** **WO-A-2005/035579**
**US-A1- 2004 186 056**

- **NISOLE SEBASTIEN ET AL: "The anti-HIV pentameric pseudopeptide HB-19 binds the C-terminal end of nucleolin and prevents anchorage of virus particles in the plasma membrane of target cells" JOURNAL OF BIOLOGICAL CHEMISTRY, vol. 277, no. 23, 7 juin 2002 (2002-06-07), pages 20877-20886, XP002414667 ISSN: 0021-9258**
- **KRUST B ET AL: "The anti-HIV pentameric pseudopeptide HB-19 is preferentially taken up in vivo by lymphoid organs where it forms a complex with nucleolin" PROCEEDINGS OF THE NATIONAL ACADEMY OF SCIENCES OF THE UNITED STATES OF AMERICA, vol. 98, no. 24, 20 novembre 2001 (2001-11-20), pages 14090-14095, XP002414668 ISSN: 0027-8424**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 013 232 B1

## Description

La présente invention concerne l'utilisation d'un composé synthétique multivalent comprenant ou constitué d'un support sur lequel sont greffés 3 à 8 motifs pseudopeptidiques, ledit composé étant de formule (I) :

$$[Y_1 \overset{\Psi}{-} P - Y_2 - (X)_m]_k - \text{ Support}$$

,

où X représente un acide aminé quelconque ; $Y_1$ et $Y_2$ sont indépendamment choisis parmi l'arginine (R) et la lysine (K) ; P est une proline ; m vaut 0 ou 1 ; k est un entier compris entre 3 et 8 ; Ψ représente une liaison peptidique modifiée, significativement plus résistante à au moins une protéase qu'une liaison peptidique normale, et le support est un peptide linéaire comprenant au moins k lysines, pour la fabrication d'un médicament destiné au traitement d'une maladie impliquant une dérégulation de la prolifération cellulaire et/ou de l'angiogénèse, et de préférence agissant en tant que ligand de la nucléoline de surface.

La division cellulaire, ou mitose, est le processus qui permet aux cellules de se multiplier afin de réparer ou de régénérer les tissus et de remplacer les cellules mortes. Chez les cellules cancéreuses, la régulation de ce processus est défaillante, c'est pourquoi ces cellules se divisent de manière anarchique et créent ainsi des tumeurs. Ainsi, l'une des voies thérapeutiques efficace pour lutter contre le développement des cancers consiste à bloquer la division des cellules cancéreuses en utilisant des molécules ayant des propriétés antimitotiques.

Cependant, les molécules antimitotiques actuelles (paclitaxel, mieux connu sous le non taxol, ou colchicine par exemple) agissent sans spécificité cellulaire, sur toutes les cellules, sans distinction et provoquent par conséquent de nombreux effets secondaires indésirables. Il est donc essentiel de développer des molécules antimitotiques induisant beaucoup moins d'effets délétères.

Toute tumeur pour se développer a besoin de nutriments et d'oxygène. Ces éléments sont apportés par des vaisseaux intra tumoraux qui apparaissent selon un mécanisme appelé angiogénèse. En effet, en absence de vaisseaux, les cellules tumorales sont soumises à un processus de nécrose cellulaire menant à une diminution puis un arrêt de la croissance tumorale. Une autre voie thérapeutique pour lutter contre les cancers consiste donc à bloquer le processus d'angiogénèse en bloquant par exemple les molécules contrôlant ce mécanisme.

La plupart des molécules anti-angiogènes actuelles sont spécifiques d'un type de facteur angiogène. Cette mono spécificité amène des phénomènes de résistance. En effet, l'inhibition d'un type de facteur angiogène induit l'expression d'un autre type par des mécanismes de compensation angiogène. Il serait donc avantageux de disposer des molécules anti-angiogènes ayant un large spectre d'action vis-à-vis des facteurs impliqués.

Cependant, l'inhibition du seul processus d'angiogénèse se révèle généralement insuffisant pour bloquer efficacement la croissance tumorale, et ne permet pas en outre de bloquer la formation de métastases.

Il serait donc très utile de disposer de nouvelles molécules anticancéreuses capables d'inhiber à la fois la prolifération des cellules tumorales et le processus d'angiogénèse au niveau de la tumeur. En effet, une étude récente montre que l'association de deux molécules thérapeutiques, l'une antimitotique et l'autre anti-angiogénique, permet d'observer une synergie et d'augmenter significativement l'efficacité du traitement global par rapport aux traitements par l'une seulement de ces molécules.

Aucune molécule capable d'avoir les deux activités, anti-mitotique et anti-angiogénique, n'a encore été décrite à ce jour.

De plus, la plupart des anticancéreux actuels ne sont pas réellement spécifiques des cellules tumorales et ciblent donc également les cellules saines, provoquant ainsi de nombreux et parfois importants effets secondaires. Ce problème a pu être résolu dans certains cas par le développement d'anticorps ciblant des molécules de surface de certaines tumeurs. Cependant, l'utilisation d'anticorps pose d'autres problèmes sérieux, et le développement d'anticorps thérapeutiques efficaces et non toxiques s'est révélé être une procédure longue, incertaine et coûteuse. De plus, la production d'anticorps à grande échelle et dans des conditions sanitaires sûres est particulièrement difficile. De ce fait, les traitements à base d'anticorps spécifiques sont encore peu nombreux et très coûteux.

Une autre difficulté liée aux anticancéreux classiques, tels que le paclitaxel, est que ces molécules sont souvent très hydrophobes, ce qui rend nécessaire pour parvenir à une biodisponibilité acceptable in vivo de développer des formulations galéniques compliquées et coûteuses. Le problème de la biodisponibilité in vivo se pose également avec beaucoup d'acuité pour les traitements à base d'acides nucléiques, qu'il est très difficile de faire parvenir de façon efficace et spécifique à leurs cellules cibles.

Il serait donc très utile de disposer de nouvelles molécules anticancéreuses présentant à la fois :

- une efficacité fortement améliorée du fait de leur double action inhibitrice sur la prolifération tumorale et sur l'angiogénèse, de telle sorte qu'elles puissent être efficaces seules, sans utilisation d'un traitement de chimiothérapie classique ou de radiothérapie, permettant ainsi de limiter fortement les effets secondaires liés à ces types de traitements,

- un spectre d'action vis-à-vis des facteurs angiogènes assez large pour éviter les phénomènes de résistances aux traitements,
- très peu d'effets secondaires du fait d'une meilleure spécificité pour les cellules tumorales,
- un procédé de synthèse facilement industrialisable,
- une plus grande facilité d'utilisation, notamment grâce à une meilleure biodisponibilité et/ou une demi vie prolongée in vivo, notamment grâce à une spécificité directe pour les cellules tumorales, à une bonne solubilité en milieu aqueux, et à une résistance améliorée aux processus de dégradation in vivo.

La nucléoline (voir structure sur la Figure 1A) a initialement été décrite comme une protéine nucléaire présente dans la plupart des cellules eucaryotes. Plus récemment, il a été démontré que, malgré l'absence de domaine transmembranaire permettant son association à la membrane plasmique, une autre forme moléculaire de cette protéine était présente également à la surface cellulaire (1-4). Cette nucléoline de surface se trouve associée étroitement aux microfilaments d'actine intracellulaire ; cette association étant selon toute vraisemblance indirecte via un partenaire transmembranaire.

Dans une cellule au repos, la nucléoline est localisée principalement au niveau du nucléole, mais également partiellement dans le cytoplasme et à la surface des cellules. A la suite d'une activation de la prolifération cellulaire, la nucléoline cytoplasmique est transloquée vers la surface membranaire par un mécanisme d'un transport actif, non conventionnel, indépendant du réticulum endoplasmique et de l'appareil de Golgi (1).

Ainsi, le niveau d'expression de la nucléoline de surface est fortement augmenté suite à l'activation des cellules, et notamment l'activation de la prolifération cellulaire. La nucléoline de surface constitue donc un marqueur des cellules activées, en prolifération. Dans le cas notamment du virus de l'immunodéficience humaine (VIH) qui cible particulièrement les cellules activées, il a été montré que la nucléoline de surface était impliquée dans l'infection de ces cellules par le VIH (2,5).

Par ailleurs, il a également été montré que la nucléoline de surface est exprimée à la surface des cellules tumorales, comme dans les cellules tumorales dérivées d'hépatocarcinome (6), de leucémie des lymphocytes T (7 et 8) et de cellules du cancer d'utérus (7), ainsi qu'à la surface des cellules endothéliales activées (9), cellules qui sont impliquées dans le processus d'angiogénèse. En outre, la nucléoline de surface constitue un récepteur de faible affinité pour différents ligands, et notamment pour plusieurs facteurs de croissance tels que la midkine (MK), l'heparin affin regulatory peptide (HARP ; également connue sous le nom de pleiotrophin : PTN) et la lactoferrine (10-12).

Récemment, il a été suggéré dans la demande de brevet WO 2005/035579 (31) qu'il était possible de traiter le cancer en utilisant des agents liant la nucléoline, tels que par exemple des anticorps anti-nucléoline, des ARN interférents anti-nucléoline; ou des oligonucléotides antisens anti-nucléoline. Les principaux résultats présentés démontrent que la nucléoline de surface peut être considérée comme un marqueur des cellules cancéreuses, ce qui en soit n'en fait pas une cible efficace. Seul des résultats préliminaires chez la souris montrent que l'ajout en combinaison d'anticorps anti-nucléoline permet d'améliorer la régression tumorale induite par le taxol. Cependant, aucun résultat montrant une efficacité de tels anticorps seuls n'est présenté, et la dose nécessaire pour obtenir l'effet améliorant en combinaison avec le taxol n'est pas indiquée. De plus, l'utilisation d'anticorps in vivo chez l'homme, comme indiqué précédemment, pose de sérieux problèmes de mise en oeuvre.

Les agents anti-nucléoline peuvent agir selon différentes voies. En particulier, de tels agents peuvent ou non agir par leur liaison à la protéine nucléoline. En effet, pour des agents de type ARN interférents ou oligonucléotides antisens anti-nucléoline, l'action éventuelle des agents se situe au niveau des acides nucléiques intracellulaires et non au niveau de la fixation à la nucléoline. Par exemple, dans la demande de brevet US 2005/0026860 (32), des oligonucléotides antisens de la nucléoline sont décrits comme ayant un effet positif sur la régression tumorale in vivo dans un modèle murin. Cependant, l'effet observé chez les souris est partiel, une tumeur moindre se développant malgré tout, et aucun effet sur l'angiogénèse n'est décrit ou suggéré. Ces résultats, bien que suggérant que la nucléoline peut effectivement être considérée comme une cible dans le traitement du cancer, indiquent une efficacité moyenne, peu susceptible de conduire à la possibilité d'un traitement unique par un agent anti-nucléoline, mais plutôt à un simple traitement adjuvant, en sus d'un traitement classique de chimiothérapie. De plus, comme indiqué précédemment, l'utilisation d'oligonucléotides in vivo pose de sérieux problèmes de biodisponibilité.

Un agent anti-nucléoline peut également se lier directement à la protéine nucléoline. Différents ligands de la nucléoline ont été décrits dans la littérature :

- le peptide F3 (ou « tumor homing peptide ») est un peptide correspondant à un fragment de 34 acides aminés de la protéine HMG2N, se liant aux cellules endothéliales activées des vaisseaux de différents types de tumeurs. Récemment, il a été montré que le peptide F3 se lie à la nucléoline exprimée sur les cellules endothéliales, et est

ensuite internalisé et transporté dans le noyau par un processus actif. La liaison à la nucléoline et l'intemalisation sont bloquées par des anticorps anti-nucléoline. Il est décrit que le peptide F3 se lie à la partie N-terminale de la nucléoline qui contient plusieurs régions riches en acides aminés acides (9). Dans la présente demande, les inventeurs montrent que le peptide F3 est incapable d'inhiber la prolifération cellulaire de cellules NIH-3T3 induite par le facteur de croissance HARP (voir Exemple 1.1.1). Le fait de se lier à la nucléoline de surface et d'être internalisé ne suffit donc pas à conférer à ce peptide la capacité à inhiber la prolifération des cellules tumorales;

- le composé multivalent HB19 (voir Figure 2A), synthétisé et décrit par les inventeurs, est également un ligand de la nucléoline de surface (7,8,13,14, 33) qui interagit au niveau du domaine RGG (voir Figure 1B). Il a été montré que ce composé, ainsi que d'autres composés de structure proche, permettaient d'inhiber l'infection des cellules activées par le VIH (13, 34) ainsi que la liaison à la nucléoline d'autres ligands naturels (10-12), mais aucun résultat montrant son éventuelle capacité à réduire et/ou inhiber la croissance tumorale ou l'angiogénèse n'a été décrit ;
- dans la demande de brevet WO00/61597, des oligonucléotides riches en guanine (GROs) sont décrits comme liant une protéine susceptible d'être la nucléoline et inhibant la prolifération des cellules tumorales in vitro à des doses supérieures ou égales à 15 $\mu$M (15). In vivo, seule l'existence d'une certaine synergie avec un traitement chimiothérapique conventionnel est mentionné. Aucun effet sur l'angiogénèse n'est décrit ou suggéré. De plus, il semble que ces GROs se lient principalement à la nucléoline intracellulaire ;
- Dans un article publié par l'équipe ayant déposé la demande WO00/61597, un autre oligonucléotide mixte appelé MIX1, comprenant dans leur séquence sens des bases G et T comme les GROs mais comprenant aussi des bases A et C, est décrit comme se liant à la nucléoline avec la même efficacité que les GROs, mais n'ayant aucun effet sur la prolifération cellulaire, suggérant encore une fois que la simple capacité à se lier à la nucléoline ne suffit pas à conférer une capacité d'inhibition de la prolifération cellulaire (15).
- Récemment, une équipe a montré qu'il était possible d'inhiber l'angiogénèse induite par le VEGF à l'aide d'une préparation d'anticorps polyclonaux anti-nucléoline (16). Cependant, bien que la préparation d'anticorps polyclonaux anti-nucléoline bloque la formation des tubules par les cellules endothéliales, elle ne bloque pas la prolifération des cellules endothéliales. De plus, seule l'angiogénèse induite par le VEGF a été testée, alors qu'il existe de nombreux autres facteurs angiogènes, et aucun résultat montrant une inhibition de la prolifération cellulaire n'est décrit ni suggéré.
- US2004/186056 (35) décrit des peptides ou des peptidomimétiques se liant spécifiquement à la nucléoline et contenant une séquence peptidique particulière, éventuellement conjuguée avec un groupement ayant une activité thérapeutique. L'effet thérapeutique des conjugués est attribuée à la molécule thérapeutique associée au peptide ou peptidomimétique, celui-ci servant seulement au ciblage de la molécule thérapeutique.

Il ressort clairement de la description ci-dessus que tous les ligands de la nucléoline ne présentent pas une activité anti-tumorale, et aucun des documents cités précédemment ne laisse supposer qu'un de ces ligands soit susceptible d'inhiber à la fois la prolifération des cellules tumorales en général et l'angiogénèse induite par différents facteurs.

De plus, il est crucial de noter que les résultats présentés, pris isolément ou en combinaison, ne suggèrent jamais qu'un de ces ligands puisse posséder une activité suffisante pour pouvoir être utilisé seul, sans combinaison avec un traitement anticancéreux classique (radiothérapie ou surtout chimiothérapie classique de type taxol).

Pourtant, les inventeurs ont montré de façon surprenante que le composé peptidique pentavalent HB19 ou d'autres composés présentant au moins 3 motifs pseudopeptidiques du même type greffés sur un support permettent d'inhiber à la fois la prolifération des cellules tumorales en général, qu'elle soit dépendante d'ancrage ou indépendante d'ancrage (prolifération caractéristique des cellules transformées), ou induite par différents facteurs de croissance, mais également l'angiogénèse induite par différents facteurs. De plus et surtout, les inventeurs montrent dans un modèle murin que le composé pentavalent HB19 permet in vivo une telle inhibition et de la prolifération tumorale et de l'angiogénèse mais aussi que l'effet antitumoral du composé HB19, à une dose classique pour un peptide (5 mg/kg), est supérieur à celui du taxol à 10 mg/kg, qui est pourtant une des molécules de référence pour les traitements antitumoraux.

Ainsi, alors que les autres ligands précédemment décrits de la nucléoline de surface ne semblent capables que d'un effet partiel, susceptible de conduire tout au plus à un traitement de type adjuvant, le composé HB19 possède in vivo, chez la souris, une efficacité supérieure au taxol, molécule anticancéreuse de référence, suggérant la possibilité de l'utiliser seul, sans combinaison avec une molécule chimiothérapique classique. Notamment, la dose de taxol administrée permet d'observer une régression tumorale, mais la régression n'est pas totale, une tumeur étant observée et pesée lorsque les souris sont sacrifiées. Au contraire, avec le ligand multivalent HB19, à une dose deux fois plus faible, aucune tumeur n'a pu être prélevée chez les souris lors du sacrifice, démontrant ainsi une régression totale.

Le composé multivalent HB19 apparaît donc comme un composé anticancéreux très puissant. Cet effet est probablement lié à sa double capacité démontrée par les inventeurs à inhiber à la fois la prolifération des cellules tumorales, qu'elle soit induite par plusieurs facteurs de croissance distincts, ou même indépendante d'ancrage, et aussi le processus d'angiogénèse induit par deux facteurs angiogènes distincts.

De plus, aucun effet toxique n'a été observé par les inventeurs, ni sur des cellules cultivées in vitro pendant

plusieurs semaines en présence du composé HB19, ni in vivo sur les souris traitées par le composé HB19. De plus, la purification des protéines liées au composé multivalent HB19 après administration in vivo permet d'obtenir à plus de 90 % la nucléoline, indiquant une forte spécificité de l'interaction entre le composé multivalent HB19 et la nucléoline, ce qui est de nature à fortement limiter la possibilité de survenue d'effets secondaires. Les inventeurs ont de plus montré que, si le peptide HB19 peut être internalisé après sa liaison à la nucléoline de surface, il ne parvient pas jusqu'au noyau, ce qui est un fait important pour expliquer son absence de toxicité sur les cellules saines.

Le composé HB19 et ses dérivés ou analogues sont en outre facilement synthétisables, y compris à l'échelle industrielle, dans des conditions sanitaires facilement maîtrisables.

Enfin, sa spécificité pour la nucléoline, et ainsi pour les cellules tumorales et les cellules endothéliales activées, sa nature pseudopeptidique, et sa solubilité élevée en milieu aqueux, lui permettent de présenter une très bonne biodisponibilité in vivo. En effet, la spécificité du composé HB19 pour la nucléoline de surface ne nécessite aucun couplage avec une molécule de ciblage. En outre, la présence d'une liaison peptidique modifiée (réduite dans le cas du composé HB19) entre la lysine et la proline de chaque motif KPR présenté dans le cas d'HB19 lui confère une bonne résistance aux protéases in vivo et une demi-vie in vivo supérieure à 24h, contrairement à un peptide classique dont la demi-vie in vivo ne dépasse pas une demi heure. De plus, le composé HB19 est complètement soluble en milieu aqueux, ce qui facilite grandement son administration, aucune forme galénique particulière n'étant nécessaire à sa circulation et à son ciblage in vivo.

Ainsi, le composé pentavalent HB19 présente toutes les caractéristiques essentielles pour résoudre les différents problèmes techniques visant à fournir de nouveaux composés anticancéreux :

- capables de présenter seuls une forte efficacité anti-tumorale, par le biais d'une double action sur la prolifération tumorale et l'angiogénèse, efficacité permettant d'envisager un traitement unique, sans combinaison avec une molécule chimiothérapique classique de type taxol ;
- ne présentant pas de spécificité pour un type de cancer particulier, mais ayant un large spectre d'activité sur les cellules tumorales et les cellules endothéliales activées ;
- générant très peu d'effets secondaires in vivo du fait de leur spécificité pour les cellules tumorales et les cellules endothéliales activées par rapport aux cellules saines ;
- possédant un procédé de synthèse facilement industrialisable ; et
- possédant en soi une biodisponibilité in vivo suffisante pour ne pas nécessiter le développement de formes galéniques particulières.

L'invention concerne donc l'utilisation d'un composé synthétique multivalent comprenant ou constitué d'un support sur lequel sont greffés 3 à 8 motifs pseudopeptidiques, ledit composé étant de formule (I) :

$$[Y_1 \overset{\Psi}{-} P - Y_2 - (X)_m]_k - \text{Support}$$ ,

où
X représente un acide aminé quelconque ;
$Y_1$ et $Y_2$ sont indépendamment choisis parmi l'arginine (R) et la lysine (K) ;
P est une proline ;
m vaut 0 ou 1 ;
k est un entier compris entre 3 et 8 ;
Ψ représente une liaison peptidique modifiée, significativement plus résistante à au moins une protéase qu'une liaison peptidique normale, et le support est un peptide linéaire comprenant au moins k lysines,
pour la fabrication d'un médicament destiné au traitement d'une maladie impliquant une dérégulation de la prolifération cellulaire et/ou de l'angiogénèse.

De préférence, un tel composé synthétique multivalent agit en tant que ligand de la nucléoline de surface.

La présente description divulgue également l'utilisation d'un composé synthétique multivalent comprenant ou constitué d'un support sur lequel sont greffés au moins 3 motifs pseudopeptidiques, ledit composé étant de formule (Ibis) :

$$[(X)_n - Y_1 \overset{\Psi}{-} (Z)_i - Y_2 - (X)_m]_k - \text{Support}$$ ,

où

chaque X représente indépendamment un acide aminé quelconque ;
$Y_1$ et $Y_2$ sont indépendamment choisis parmi les acides aminés à chaîne latérale basique ;
Z est choisi parmi :

- une proline, éventuellement substituée en γ, β ou δ par des groupements hydroxyle, aminé, alkyle en $C_1$-$C_{10}$, alcényle en $C_1$-$C_{10}$, alcynyle en $C_1$-$C_{10}$, aryle en $C_5$-$C_{12}$, aralkyle en $C_5$-$C_{14}$, hétéroaryle en $C_5$-$C_{12}$ (avantageusement un hétéroaryle en $C_5$), ces groupes étant eux-mêmes éventuellement substitués par 1 à 6 substituants choisis parmi un atome d'halogène, $NO_2$, OH, un alkyle en $C_1$-$C_4$, $NH_2$, CN, un trihalomèthyle, un acyloxy en $C_1$-$C_4$, un dialkylamino en $C_1$-$C_4$, un groupe guanidino, un groupe thiol;
- un acide aminé, naturel ou non, N-alkylé ;
- un acide aminé dialkylé ;
- un acide aminé dialkylé cyclique ; ou
- l'acide pipécolique ou l'un de ses dérivés ;

n est i sont indépendamment 0 ou 1 ;
m est un entier entre 0 et 3 ;
k est un entier supérieur ou égal à 3 ; et
Ψ représente une liaison peptidique modifiée, significativement plus résistante à au moins une protéase qu'une liaison peptidique normale,
pour la fabrication d'un médicament destiné au traitement d'une maladie impliquant une dérégulation de la prolifération cellulaire et/ou de l'angiogénèse.

De préférence, un tel composé synthétique multivalent agit en tant que ligand de la nucléoline de surface.

On entend par « support » toute molécule pharmaceutiquement acceptable, c'est-à-dire sans toxicité intrinsèque, sur laquelle 3 à 8 motifs pseudopeptidiques de formule (I) peuvent être greffés. Un support acceptable devrait donc posséder une taille suffisante pour permettre de greffer de 3 à 8 motifs pseudopeptidiques de formule (I). Un tel support acceptable devrait aussi de préférence posséder une taille suffisamment petite pour que lesdits 3 à 8 motifs pseudopeptidiques de formule (I) puissent venir ensemble interagir au niveau du domaine RGG d'une ou plusieurs molécules de nucléoline. Le support devrait de surcroît ne pas être immunogène.

La présente description divulgue des composés dans lesquels le support peut être choisi parmi un peptide linéaire ou un peptide cyclique, un peptoïde (oligomère de glycine N-substitué) linéaire ou cyclique, un foldamère (oligomère ou polymère ayant une forte tendance à adopter une conformation compacte bien définie et prévisible en solution), un polymère linéaire ou un dendrimère (macromolécule constituée de monomères qui s'associent selon un processus arborescent autour d'un coeur central plurifonctionnel) sphérique, un sucre, ou une nanoparticule. Dans ces composés décrits, ledit support peut être choisi parmi un peptide linéaire ou cyclique, ou bien un peptoïde linéaire ou cyclique.

L'utilisation d'un peptide linéaire (voir structure de HB19 sur la Figure 2A) permet une synthèse facile du support, et les résultats des inventeurs avec le composé HB19 montrent qu'un tel support permet effectivement de résoudre le problème technique posé dans la présente demande. Un peptide linéaire servant de support peut avantageusement comprendre une proportion de lysine supérieure à 25%. Plus précisément, lorsqu'un peptide linéaire est utilisé comme support, les motifs pseudopeptidiques sont de préférence greffés en position ε d'une lysine.

Un peptide linéaire est utilisé comme support dans l'invention, et il comprend au moins autant de lysines que le nombre de motifs pseudopeptidiques que l'on souhaite greffer dessus.

Par exemple, un peptide linéaire support peut posséder une séquence choisie parmi KKKGPKEKGC (SEQ ID NO :1), KKKKGC (SEQ ID NO :2), KKKKGPKKKKGA (SEQ ID NO :3) ou KKKGPKEKAhx$CONH_2$ (SEQ ID NO :4), où Ahx représente l'acide amino hexanoïque et $CONH_2$ représente le fait que la fonction acide est remplacée par une fonction amide, Ahx$CONH_2$ représentant le (2S)-2-aminohexanamide, ou bien une séquence linéaire constituée de 2 à 4 motifs (KAKPG, SEQ ID NO :12), notamment la séquence AcKAKPGKAKPGKAKPG$CONH_2$ (SEQ ID NO :13, où Ac représente un groupement acétyl $CH_3$-CO-, et $CONH_2$ signifie que la fonction acide COOH de la glycine est remplacée par une fonction amide $CONH_2$). Avantageusement, le peptide linéaire support peut être le peptide KKKGPKEKAhx$CONH_2$ (voir par exemple HB 19 sur la Figure 2A, SEQ ID NO :5, qui possède ce peptide linéaire pour support), ou le peptide AcKAKPGKAKPGKAKPG$CONH_2$ (SEQ ID NO :4, , où Ac représente un groupement acétyl $CH_3$-CO- et $CONH_2$ signifie que la fonction acide COOH de la glycine est remplacée par une fonction amide $CONH_2$, voir par exemple Nucant 7 sur la Figure 2F, SEQ ID NO :17, qui possède ce peptide linéaire pour support).

Parmi les peptides linéaires, certains sont connus pour adopter une structure hélicoïdale. Ces peptides linéaires peuvent également être utilisés comme support dans l'invention. De tels supports peptidiques linéaires formant une structure hélicoïdale comprennent notamment des supports constitués d'un nombre entier supérieur ou égal à 3, notamment de 3 à 8, de répétitions de motifs peptidiques de séquence Aib-Lys-Aib-Gly (SEQ ID NO:6) ou Lys-Aib-Gly (SEQ ID NO :7) respectivement, où Aib représente l'acide 2-amino-isobutyrique. Chacun de ces motifs comprenant un

seul résidu lysine (Lys), il faudra autant de répétitions de ces motifs qu'on souhaite pouvoir greffer de motifs pseudopeptidiques de formule (I).

Par exemple, pour obtenir un composé pentavalent avec 5 motifs pseudopeptidiques de formule (I), on peut utiliser comme support un peptide linéaire formant une structure hélicoïdale de formule Aib-Lys-Aib-Gly-Aib-Lys-Aib-Gly-Aib-Lys-Aib-Gly-Aib-Lys-Aib-Gly-Aib-Lys-Aib-Gly (SEQ ID NO:8) ou Lys-Aib-Gly-Lys-Aib-Gly-Lys-Aib-Gly-Lys-Aib-Gly-Lys-Aib-Gly (SEQ ID NO:9). Avantageusement, un peptide linéaire formant une structure hélicoïdale de formule dérivée de SEQ ID NO :8 et 9 est utilisé, dont la formule est choisie parmi Ac-Aib-Lys-Aib-Gly-Aib-Lys-Aib-Gly-Aib-Lys-Aib-Gly-Aib-Lys-Aib-Gly-Aib-Lys-Aib-Gly-$CONH_2$ (SEQ ID NO :18, où Ac représente un groupement acétyl $CH_3$-CO- et $CONH_2$ signifie que la fonction acide COOH de la glycine est remplacée par une fonction amide $CONH_2$, voir par exemple Nucant 2 sur la Figure 2C, SEQ ID NO :20, qui possède ce peptide pour support) ou Ac-Lys-Aib-Gly-Lys-Aib-Gly-Lys-Aib-Gly-Lys-Aib-Gly-Lys-Aib-Gly-$CONH_2$ (SEQ ID NO :19, où Ac représente un groupement acétyl $CH_3$-CO- et $CONH_2$ signifie que la fonction acide COOH de la glycine est remplacée par une fonction amide $CONH_2$, voir par exemple Nucant 3 sur la Figure 2D, SEQ ID NO :21, qui possède ce peptide pour support).

Alternativement, pour obtenir un composé hexavalent avec 6 motifs pseudopeptidiques de formule (I), on peut utiliser comme support un peptide linéaire formant une structure hélicoïdale de formule Ac-Aib-Lys-Aib-Gly-Aib-Lys-Aib-Gly-Aib-Lys-Aib-Gly-Aib-Lys-Aib-Gly-Aib-Lys-Aib-Gly-Aib-Lys-Aib-Gly-$CONH_2$ (SEQ ID NO :14, où Ac représente un groupement $CH_3$-CO- et $CONH_2$ signifie que la fonction acide COOH de la glycine est remplacée par une fonction amide $CONH_2$) ou Ac-Lys-Aib-Gly-Lys-Aib-Gly-Lys-Aib-Gly-Lys-Aib-Gly-Lys-Aib-Gly-Lys-Aib-Gly-$CONH_2$ (SEQ ID NO :15, où Ac représente un groupement $CH_3$-CO- et $CONH_2$ signifie que la fonction acide COOH de la glycine est remplacée par une fonction amide $CONH_2$, voir par exemple Nucant 6 sur la Figure 2E, SEQ ID NO :17, qui possède ce peptide pour support).

Dans les composés de formule (Ibis) décrits ci-dessus, un peptide ou peptoïde cyclique peut également être avantageusement utilisé comme support. Cela permet notamment de restreindre la flexibilité du squelette. Un peptide ou peptoïde cyclique support peut notamment être choisi parmi un hexa-, octa-, déca- ou dodéca- peptide cyclique constitué préférentiellement de résidus d'acides aminés de configuration L (lévogyre) et D (dextrogyre) en alternance (D,L-cyclopeptide) ou bien d'un enchaînement de résidus N-alkyl Glycine (peptoïde cyclique). Un exemple de composé avec comme support un hexapeptide cyclique constitué en alternance de résidus alanine (A) de configuration D et de résidus lysine (K) de configuration L présentant 3 motifs KPR avec une liaison $\Psi(\Psi = (-CH_2N-))$ entre K et P est montré sur la Figure 2B (composé Nucant 01).

Avantageusement, le support d'un composé de formule (I) selon l'invention est support est choisi parmi un peptide linéaire de séquence SEQ ID NO :1, SEQ ID NO :2, SEQ ID NO :3, SEQ ID NO :4, SEQ ID NO :8, SEQ ID NO :9, SEQ ID NO :13, SEQ ID NO :14, SEQ ID NO :15, SEQ ID NO :18, ou SEQ ID NO :19.

Au sens de l'invention, on entend par « greffés » pour les motifs pseudopeptidiques le fait d'être liés au support par une liaison covalente, soit directement, soit par l'intermédiaire d'un composé espaceur entre les motifs pseudopeptidiques et le support. De ce fait, dans un mode de réalisation particulier, les motifs pseudopeptidiques sont greffés directement sur le support, sans composé espaceur entre eux et le support. Dans un autre mode de réalisation, les motifs pseudopeptidiques sont greffés sur le support par l'intermédiaire d'un espaceur. Des exemples d'espaceurs acceptables comprennent des composés de type éthylène glycol, pipérazine, ou un acide aminé de type acide aminohexanoïque ou beta-alanine.

Dans le cas où le support est un peptide linéaire ou cyclique et où les motifs pseudopeptidiques sont greffés directement sur le peptide, la liaison entre le peptide et les motifs pseudopeptidiques est de préférence réalisée au niveau d'un résidu lysine du peptide support, au niveau d'un groupe amino en position $\alpha$ ou $\varepsilon$, de préférence au niveau du groupe amino en position $\varepsilon$ (sur la chaîne latérale) de la lysine. Ainsi, le greffage direct des motifs pseudopeptidiques sur un support peptidique se fait avantageusement par une liaison amide entre une fonction acide COOH de l'acide aminé en position C-terminale du motif pseudopeptidique et un groupement amino d'un résidu lysine, de préférence le groupement amino en position $\varepsilon$ (sur la chaîne latérale) de la lysine.

Dans les composés selon l'invention, 3 à 8 motifs pseudopeptidiques sont greffés sur le support. En effet, les résultats des inventeurs montrent l'importance de la liaison au domaine RGG de la nucléoline (voir Figure 1) pour l'efficacité anti-tumorale exceptionnelle du composé HB19 et des composés dérivés ou analogues. Or la liaison au domaine RGG de la nucléoline est réalisée grâce à la présentation multivalente de plusieurs motifs pseudopeptidiques tels que ceux incorporés dans la formule (I). Pour des composés dont le support est un peptide linéaire de séquence KKKGPKEKGC, KKKKGC, KKKKGPKKKKGA ou KKKGPKEKAhx$CONH_2$, les inventeurs ont montré qu'en dessous de 3 motifs ($k < 3$), l'efficacité de liaison à la nucléoline est moins forte et l'efficacité anti-tumorale en est vraisemblablement amoindrie. Les composés selon l'invention comprennent donc de 3 à 8 motifs pseudopeptidiques ($3 \leq k \leq 8$) greffés sur le support. De plus, les inventeurs ont montré que l'activité était optimale avec 5 ou 6 motifs pseudopeptidiques greffés sur le support ($k = 5$), l'efficacité de liaison à la nucléoline n'augmentant plus pour un nombre plus élevé de motifs pseudopeptidiques. Avantageusement, dans les composés de formule (I), k est donc compris entre 4 et 7, entre 4 et 6, entre 4 et 5 ou entre 5 et 6. Plus avantageusement encore, dans les composés de formule (I), k vaut 5 ou mieux encore 6.

Au sens de l'invention, on entend par « acide aminé quelconque » tout acide aminé naturel ou de synthèse, éventuellement modifié par la présence d'un ou plusieurs substituants. Plus précisément, on entend par acide aminé un acide alpha aminé répondant à la structure générale :

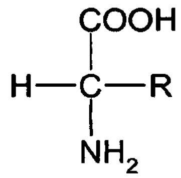
,

où R représente la chaîne latérale de l'acide aminé. Au sens de l'invention, R représente donc la chaîne latérale d'un acide aminé naturel ou non. Par « acide aminé naturel », on entend tout acide aminé pouvant se trouver naturellement in vivo chez un être vivant. Les acides aminés naturels comprennent donc notamment les acides aminés codés par les ARNm incorporés dans les protéines lors de la traduction, mais également d'autres acides aminés trouvés naturellement in vivo pouvant être un produit ou un sous-produit d'un processus métabolique, comme par exemple l'ornithine qui est générée dans le processus de production de l'urée par l'arginase à partir de L-arginine. Dans l'invention, les acides aminés utilisés peuvent donc être naturels ou non. Notamment, les acides aminés naturels sont généralement de configuration L, mais au sens de l'invention, un acide aminé peut aussi bien être de configuration L ou D. De plus, R n'est bien entendu pas limité aux chaînes latérales des acides aminés naturels, mais peut être librement choisi.

Dans les motifs pseudopeptidiques des composés de formule (Ibis), Z est soit absent (i = 0), soit présent (i=1) et alors choisi parmi

- une proline, éventuellement substituée en $\gamma$, $\beta$ ou $\delta$ par des groupements hydroxyle, amine, alkyle en $C_1$-$C_{10}$, alcényle en $C_1$-$C_{10}$, alcynyle en $C_1$-$C_{10}$, aryle en $C_5$-$C_{12}$, aralkyle en $C_5$-$C_{14}$, hétéroaryle en $C_5$-$C_{12}$ (avantageusement un hétéroaryle en $C_5$), ces groupes étant eux-mêmes éventuellement substitués par 1 à 6 substituants choisis parmi un atome d'halogène, $NO_2$, OH, un alkyle en $C_1$-$C_4$, $NH_2$, CN, un trihalométhyle, un acyloxy en $C_1$-$C_4$, un dialkylamino en $C_1$-$C_4$, un groupe guanidino, un groupe thiol;
- un acide aminé, naturel ou non, N-alkylé ;
- un acide aminé dialkylé (par exemple l'acide amino isobutyrique) ;
- un acide aminé dialkylé cyclique ; ou
- l'acide pipécolique ou l'un de ses dérivés ;

Par « alkyle en $C_1$-$C_i$ », on entend un radical hydrocarboné saturé linéaire ou ramifié de formule $-C_jH_{2j+1}$ où $1 \leq j \leq i$. Les alkyles en $C_1$-$C_{10}$ comprennent donc les alkyles en $C_1$ (méthyle), $C_2$ (éthyle), $C_3$ (n-propyle, ou isopropyle), $C_4$ (n-butyle, isobutyle, sec-butyle ou tert-butyle), $C_5$ (ex : n-pentyle, néopentyle, isopentyle, tert-pentyle), et $C_6$ à $C_{10}$. Par « alcényle en $C_1$-$C_{10}$ » on entend un radical hydrocarboné insaturé linéaire ou ramifié comprenant de 1 à 10 atomes de carbones et comprenant au moins une double liaison C=C. Par « alcynyle en $C_1$-$C_{10}$ » on entend un radical hydrocarboné insaturé linéaire ou ramifié comprenant de 1 à 10 atomes de carbones et comprenant au moins une triple liaison C≡C. Par « aryle en $C_5$-$C_{12}$ », on entend un radical hydrocarboné monocyclique ou polycyclique aromatique de 5 à 12 atomes de carbones. Par « aralkyle en $C_5$-$C_{14}$ », on entend la combinaison d'un alkyle et d'un aryle possédant au total 5 à 14 atomes de carbone. Par « hétéroaryle en $C_5$-$C_{12}$ », on entend un groupement aryle dont au moins un atome de carbone de la chaîne hydrocarbonée comportant normalement 5 à 12 carbones est substitué par un autre atome choisi parmi N, O, ou S. Par « hétéroaryle en $C_5$ », on entend donc un groupement aryle dont au moins un des 5 atomes de carbone de la chaîne hydrocarbonée est substitué par un autre atome choisi parmi N, O, ou S. Par « acyloxy en $C_1$-$C_4$ », on entend un groupement de formule -O(O)C-(alkyle en $C_1$-$C_4$), —O(O)C-(cycloalkyle en $C_4$-$C_{12}$), -O(O)C-(aryle en $C_4$-$C_{12}$), -O(O)C-(arylalkyle en $C_4$-$C_{12}$), ou -O(O)C-(hétéroaryle en $C_4$-$C_{12}$). Avantageusement, dans un composé de formule (I), un tel « acyloxy en $C_1$-$C_4$ » est choisi parmi un groupement de formule -O(O)C-(alkyle en $C_1$-$C_4$), -O(O)C-(cycloalkyle en $C_4$), -O(O)C-(aryle en $C_4$), -O(O)C-(arylalkyle en $C_4$), ou -O(O)C-(hétéroaryle en $C_4$). Par « dialkylamino en $C_1$-$C_4$ » on entend un radical de formule - N(alkyle en $C_1$-$C_4$)$_2$ où chaque alkyle est identique ou différent.

Par « acide aminé N-alkylé », on entend un acide aminé quelconque dont un des atomes d'hydrogène du groupement aminé est substitué par une chaîne alkyle $C_1$-$C_{10}$ ou un groupement aralkyle en $C_5$-$C_{14}$, de préférence en $C_5$-$C_{10}$, notamment en $C_{10}$, éventuellement substitués. Des exemples d'acides aminés N-alkylés comprennent l'acide N-méthylglycine ou sarcosine, l'acide N-méthylisoleucine, l'acide N-méthylvaline etc... Par « acide aminé dialkylé », on entend tout acide aminé dont deux des atomes d'hydrogène (sur le carbone central ou sur le groupement amine) sont substitués par une chaîne alkyle en $C_1$-$C_{10}$ ou un groupement aralkyle en $C_5$-$C_{14}$, de préférence en $C_5$-$C_{10}$, notamment en $C_{10}$, éventuellement substitués. Des exemples d'acides aminés dialkylés comprennent l'acide 2-amino-isobutyrique

(Aib), l'acide aminocyclopropane carboxylique etc...

Avantageusement, Z est présent et donc i vaut 1. Avantageusement également, quand Z est présent (i=1), alors Z est une proline, éventuellement substituée en $\gamma$, $\beta$ ou $\delta$ comme décrit précédemment.

Dans les motifs pseudopeptidiques des composés de formule (I) selon l'invention, P est une proline.

Dans les motifs pseudopeptidiques des composés de formule (Ibis), $Y_1$ et $Y_2$ sont choisis parmi les acides aminés à chaîne latérale basique. Par « acide aminé à chaîne latérale basique » on entend tout acide aminé naturel ou non, dont la chaîne latérale R possède une valeur de pKa supérieure à 7 (pKa(R)>7). Ainsi, tout acide aminé peut être utilisé pour $Y_1$ et $Y_2$, sous réserve que sa chaîne latérale possède une valeur de pKa supérieure à 7, de préférence supérieure à 7,5 ; supérieure à 8 ; supérieure à 8,5 ; ou supérieure à 9. Notamment, parmi les acides aminés naturels, ceux dont la chaîne latérale possède une valeur de pKa supérieure à 7 comprennent la lysine (K, pKa(R) $\approx$ 10,5) l'arginine (R, pKa(R) $\approx$ 12,5), et l'ornithine (homologue inférieur de la lysine, pKa(R) $\approx$ 10,8), considérés généralement comme les acides aminés naturels basiques. Ainsi, dans un mode de réalisation avantageux, $Y_1$ et $Y_2$ sont indépendamment choisis parmi l'arginine (R), la lysine (K) et l'ornithine. Encore avantageusement, $Y_1$ est une lysine (K) et $Y_2$ est une arginine (R). Mais d'autres acides aminés non naturels peuvent leur être substitués, du moment que la valeur de pKa de leur chaîne latérale R soit supérieure à 7, de préférence supérieure à 7,5 ; supérieure à 8 ; supérieure à 8,5 ; ou supérieure à 9.

Dans les motifs pseudopeptidiques des composés de formule (I) selon l'invention, $Y_1$ et $Y_2$ sont indépendamment choisis parmi l'arginine (R) et la lysine (K).

Dans les composés selon l'invention, le motif pseudopeptidique essentiel à la liaison au domaine RGG de la nucléoline est le sous-motif de formule (II) $Y_1 \overset{\Psi}{\text{———}} (Z)i — Y_2 \quad (II)$, où $Y_1$ et $Y_2$ sont tels que définis précédemment. Cependant, la présence à l'une ou l'autre extrémité de ce sous-motif essentiel de quelques acides aminés quelconques, tels que définis précédemment, n'est pas de nature à empêcher la liaison à la nucléoline.

C'est pourquoi, dans les motifs pseudopeptidiques des composés de formule (I) selon l'invention, m vaut 0 ou 1, avantageusement m vaut 0.

C'est également pourquoi, dans les motifs pseudopeptidiques des composés de formule (Ibis), le sous-motif essentiel de formule (II) peut comprendre à l'une et/ou l'autre de ses extrémités de 0 à 3 acides aminés quelconques, représentés dans la formule (I) par (X)n et (X)m respectivement, où n vaut 0 ou 1 et m est un entier de 0 à 3. Avantageusement, le nombre d'acides aminés quelconques présents à l'une et/ou l'autre des extrémités du sous-motif essentiel de formule (II) est faible, c'est-à-dire que n vaut avantageusement 0 et m est un entier avantageusement entre 0 et 2, avantageusement 0 ou 1, avantageusement 0. Ainsi, dans un mode de réalisation avantageux, n et m sont égaux à 0.

Dans les composés de formule (I) selon l'invention, le sous-motif de formule (II) comprend une liaison peptidique $\Psi$ modifiée, significativement plus résistante à au moins une protéase qu'une liaison peptidique normale.

Par « liaison peptidique normale », on entend une liaison amide de formule (-CONH-) qui est normalement présente entre deux acides aminés au sein d'une protéine naturelle. Une telle liaison est sensible à l'action des protéases. Par « liaison peptidique $\Psi$ modifiée », on entend une liaison chimique entre deux acides aminés de formule chimique distincte de la liaison peptidique normale de formule (-CONH-) Cette liaison $\Psi$ modifiée est telle qu'elle est significativement plus résistante à au moins une protéase qu'une liaison peptidique normale de formule (-CONH-), par « protéase », encore appelée « peptidase » ou « enzyme protéolytique », on entend toute enzyme qui clive les liaisons peptidiques normales des protéines. Ce processus est appelé clivage protéolytique. Cela implique l'utilisation d'une molécule d'eau ce qui classe les protéases parmi les hydrolases. Parmi les protéases, on distingue notamment les protéases appelées N-peptidases qui effectuent le clivage à l'extrémité N-terminale des protéines. Ces protéases sont particulièrement gênantes pour la stabilité in vivo de peptides sans liaisons peptidiques modifiées. C'est la raison pour laquelle les motifs pseudopeptidiques des composés de formule (I) comprennent une liaison $\Psi$ modifiée entre $Y_1$ et Z (si i=1) ou $Y_1$ et $Y_2$ (si i=0) de façon à augmenter significativement la résistance du sous-motif de formule (II), qui est essentiel à la liaison à la nucléoline, notamment à ces N-peptidases. La liaison $\Psi$ devrait donc permettre d'augmenter significativement la résistance à au moins une N-peptidase. Cela permet d'augmenter significativement la demi-vie des composés de formule (I) in vivo et in vitro. Notamment, le composé HB19, qui présente cette liaison $\Psi$ modifiée, possède dans du sérum humain ou dans du sérum de veau fétal à 37°C une demi-vie supérieure à 24 heures, tandis que le même composé avec une liaison peptidique normale à la place de la liaison $\Psi$ ne possède dans les même conditions qu'une demi-vie d'une heure.

De plus, les inventeurs on trouvé que la présence de cette liaison $\Psi$ modifiée permet également d'augmenter significativement l'efficacité de liaison à la nucléoline. Ce phénomène pourrait être dû au fait que cela permet au compose HB19 de former un complexe irréversible avec la nucléoline.

Différentes liaisons chimiques susceptibles d'augmenter significativement la résistance à au moins une protéase sont connues. Ainsi, dans un mode de réalisation avantageux, $\Psi$ représente une liaison réduite ($-CH_2NH-$) (ou bien

($-CH_2N-$) dans le cas où la liaison se fait au niveau d'un groupement amine secondaire comme dans le cas d'une liaison avec une proline), une liaison rétro inverso (-NHCO-), une liaison méthylène oxy ($-CH_2-O-$), une liaison thiométhylène ($-CH_2-S-$), une liaison carba ($-CH_2CH_2-$), une liaison cetomethylene ($-CO-CH_2-$), une liaison hydroxyéthylène ($-CHOH-CH_2-$), une liaison (-N-N-), une liaison E-alcène ou une liaison (-CH=CH-). Notamment, les inventeurs ont montré que l'utilisation d'une liaison réduite ($-CH_2NH-$) permet d'augmenter significativement la résistance a au moins une protéase. Avantageusement, Ψ représente donc une liaison réduite ($-CH_2NH-$).

Bien que seule la liaison Ψ entre $Y_1$ et P soit systématiquement présente dans les composés de formule (I) selon l'invention, il est également possible que d'autres liaisons peptidiques des motifs pseudopeptidiques soient modifiées tel que décrit précédemment. En particulier, au sens de l'invention, les liaisons entre acides aminés pour lesquelles rien n'est précisé peuvent être indifféremment des liaisons peptidiques normales ou des liaisons Ψ modifiées tel que décrit précédemment. La présence de liaisons Ψ additionnelles peut permettre d'augmenter encore la résistance aux protéases des composés de formules (I). Cependant, l'augmentation liée à la présence de la première liaison Y entre P est déjà très significative, et l'ajout d'autres liaisons Ψ complique la synthèse des motifs pseudopeptidiques et donc des composés de formule (I). La présence de liaisons Ψ additionnelles est donc possible mais facultative. De même, dans les composés de formule (Ibis) décrits ici, d'autres liaisons peptidiques des motifs pseudopeptidiques peuvent être modifiées tel que décrit précédemment.

Des exemples de composés utilisables dans l'invention incluent notamment les composés (voir Figure 2 et exemples 1, 2, 3 et 4) :

- HB19 (Figure 2A, SEQ ID NO : 5, composé possédant pour support un peptide linéaire de séquence SEQ ID NO:4 sur lequel 5 motifs pseudopeptidiques KΨPR (avec $\Psi = CH_2-N$) sont liés covalamment sur le ε amino groupe de chacun des 5 résidus lysine),
- Nucant 2 (Figure 2C, SEQ ID NO : 20, composé possédant pour support un peptide linéaire adoptant une structure hélicoïdale de séquence SEQ ID NO :18 sur lequel 5 motifs pseudopeptidiques KΨPR (avec $\Psi = CH_2-N$) sont liés covalamment sur le ε amino groupe de chacun des 5 résidus lysine),
- Nucant 3 (Figure 2D, SEQ ID NO : 21, composé possédant pour support un peptide linéaire adoptant une structure hélicoïdale de séquence SEQ ID NO :19 sur lequel 5 motifs pseudopeptidiques KΨPR (avec $\Psi = CH_2-N$) sont liés covalamment sur le ε amino groupe de chacun des 5 résidus lysine),
- Nucant 6 (Figure 2E, SEQ ID NO : 16, composé possédant pour support un peptide linéaire adoptant une structure hélicoïdale de séquence SEQ ID NO :15 sur lequel 6 motifs pseudopeptidiques KΨPR (avec $\Psi = CH_2-N$) sont liés covalamment sur le ε amino groupe de chacun des 6 résidus lysine), et
- Nucant 7 (Figure 2F, SEQ ID NO : 17, composé possédant pour support un peptide linéaire de séquence SEQ ID NO:13 sur lequel 6 motifs pseudopeptidiques KΨPR (avec $\Psi = CH_2-N$) sont liés covalamment sur le ε amino groupe de chacun des 6 résidus lysine).

Un composé de formule (Ibis) décrit ici est le composé Nucant 01 (Figure 2B, composé possédant pour support un hexapeptide cyclique constitué en alternance de résidus alanine (A) de configuration D et de résidus lysine (K) de configuration L, sur lequel trois motifs pseudopeptidiques KΨPR (avec $\Psi = CH_2-N$) sont liés covalamment sur le ε amino groupe de chacun des 3 résidus lysine (K) ; voir Figure 2B).

Les composés décrits précédemment sont utilisés pour la fabrication d'un médicament destiné au traitement d'une maladie impliquant une dérégulation de la prolifération cellulaire et/ou de l'angiogénèse. Par « maladie impliquant une dérégulation de la prolifération cellulaire et/ou de l'angiogénèse », on entend au sens de l'invention toute maladie humaine ou animale affectant un ou plusieurs organes dans lesquels sont observées une ou plusieurs proliférations anormales de cellules, groupes de cellules ou de tissu et/ou une néovascularisation anormale. De telles maladies comprennent bien évidemment les cancers de tout type, tels que les adénomes, les sarcomes ; les carcinomes ; les lymphomes ; et notamment les cancers de l'ovaire, du sein, du pancréas, des ganglions lymphatiques, de la peau, du sang, du poumon, du cerveau, du rein, du foie, de la cavité nasopharyngée, de la thyroïde, du système nerveux central, de la prostate, du côlon, du rectum, du col utérin, des testicules, ou de la vessie. Elles comprennent également des maladies non cancéreuses de la peau telles que les kystes épidermiques et dermiques, le psoriasis, les angiomes ; des maladies oculaires telles que la dégénérescence maculaire liée à l'age (DMLA), la rétinopathie diabétique, ou le glaucome néovasculaire ; des maladies neurodégénératives comme la sclérose en plaque, Parkinson et Alzheimmer ; ou des maladies auto-immunes telles que le lupus ou la polyarthrite rhumatoïde ainsi que les maladies liées à l'athérosclérose.

Avantageusement, ladite maladie impliquant une dérégulation de la prolifération cellulaire et/ou de l'angiogénèse est un cancer, notamment l'un de ceux cités précédemment.

La présente description décrit en outre un procédé de criblage de molécules inhibant à la fois la prolifération cellulaire et l'angiogénèse, comprenant :

a) la mise en présence de cellules exprimant la nucléoline de surface avec une molécule à tester, et

b) la détermination de la capacité de ladite molécule à tester à se lier au domaine RGG de la nucléoline.

Il est possible de produire un domaine RGG synthétique de 60 acides aminés de la nucléoline par une synthèse chimique ou par une approche de génie génétique via l'expression de sa séquence d'ADN nucléique. La détermination de la capacité de ladite molécule à tester à se lier au domaine RGG de la nucléoline peut alors être réalisée par différentes technologies connues de l'homme du métier.

Notamment, elle peut être réalisée par mesure de la liaison à un domaine RGG synthétique de 60 acides aminés par la technique de résonance plasmonique de surface, notamment sur un appareil Biacore® 3000. Le système BIACORE® est un biocapteur utilisant le principe physique de la résonance plasmonique de surface (SPR). Il permet de mesurer en temps réel, et sans marquage spécifique, les constantes cinétiques d'interaction (Ka et Kd) entre deux molécules sur une surface biospécifique. Pour cela, une des molécules (le ligand) est immobilisée sur la surface " sensor " et l'autre (l'analyte) est injectée. Le principe de détection par SPR quantifie des changements de l'indice de réfraction près de la surface, reliés à la variation de masse à la surface du biocapteur, due à la formation et à la dissociation des complexes moléculaires. En effet, lorsqu'une lumière monochromatique et polarisée arrive à l'interface entre deux milieux d'indice de réfraction différents, et que cette interface est recouverte d'une fine couche métallique, l'intensité de la lumière réfléchie est nettement réduite pour un angle d'incidence particulier. Ceci provient du fait qu'une composante électromagnétique de la lumière, l'onde évanescente, se propage perpendiculairement à l'interface, jusqu'à 1 $\mu$m. L'angle de résonance varie notamment en fonction de l'indice de réfraction, donc en fonction de la masse des molécules situées au voisinage de la surface. Par conséquent, un suivi de l'angle SPR en fonction du temps permet de suivre en temps réel l'association et la dissociation entre le ligand et l'analyte. Le signal obtenu est enregistré: c'est un sensorgramme. Il est quantifié en unités de résonance (RU). Une variation de 1000 RU correspond à un déplacement de l'angle de 0.1° et équivaut à une fixation de 1 ng de protéine par $mm^2$. L'utilisation de cette technologie permet donc de déterminer non seulement la capacité d'une molécule à tester à se lier au domaine RGG, mais aussi plus précisément l'efficacité de liaison (constante d'affinité) de ladite molécule à tester au domaine RGG de la nucléoline.

Il est également possible de générer un domaine RGG synthétique marqué avec la biotine ou fusionné avec un peptide ou une protéine comme la GST (Glutathione S-transferase). La présence de la biotine ou de la GST permet alors une détection grâce à l'utilisation d'un ligand marqué (fluorescent, luminescent, radioactif etc), l'avidine/streptavidine si le domaine RGG est biotinylé ou des anticorps anti-GST si le domaine RGG est fusionné à la GST. Bien que moins précises, ces technologies permettent un criblage plus facile et rapide d'un nombre plus important de molécules pour leur capacité à se lier au domaine RGG de la nucléoline, la détermination plus précise de l'efficacité de liaison au domaine RGG de la nucléoline pouvant ensuite être réalisée par la technique de résonance plasmonique de surface précédemment décrite.

De préférence, on réalise donc d'abord par une méthode simple et facile à mettre en oeuvre un criblage sur la capacité à se lier au domaine RGG de la nucléoline, puis on affine l'analyse en déterminant l'efficacité de liaison au domaine RGG des candidats capables de se lier au domaine RGG par la technique de résonance plasmonique de surface. Cependant, dans le cas où seulement un petit nombre de composés sont à tester, on peut directement mesurer leur efficacité de liaison au domaine RGG par la technique de résonance plasmonique de surface.

L'invention concerne en outre un composé de formule (I) tel que défini précédemment, à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I).

Notamment, le support (à l'exception de ceux exclus au paragraphe précédent), la façon dont les motifs pseudopeptidiques sont greffés sur le support, les acides aminés X, $Y_1$ et $Y_2$, n et m, ou Y peuvent être sous la forme de tout mode de réalisation précédemment décrit.

Notamment, le support peut être choisi parmi un peptide linéaire comprenant une séquence d'acides aminés choisie parmi Aib-K-Aib-G (SEQ ID NO : 6) ou K-Aib-G (SEQ ID NO : 7), un peptide cyclique, un peptoïde linéaire ou cyclique, un foldamère, un polymère linéaire ou un dendrimère sphérique, un sucre, ou une nanoparticule.

Dans certains cas avantageux, le support peut être un peptide linéaire constitué d'une séquence d'acides aminés choisie parmi SEQ ID NO :8 et SEQ ID NO :9, SEQ ID NO : 14, SEQ ID NO : 15, SEQ ID NO :18, et SEQ ID NO :19.

Les composés selon l'invention incluent notamment les composés avec comme support un peptide linéaire de structure hélicoïdale tel que Nucant 2 (SEQ ID NO : 20 et Figure 2C) et Nucant 3 (SEQ ID NO : 21 et Figure 2D) décrits précédemment. Ils incluent également le composé Nucant 6 (SEQ ID NO : 16, voir exemple 4 et Figure 2E).

L'invention concerne également comme composé le composé Nucant 7 (SEQ ID NO : 17 et Figure 2F).

La présente description décrit en outre un composé de formule (Ibis) tel que défini précédemment, à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I). Notamment, le support (à l'exception de ceux exclus au paragraphe précédent), la façon dont les motifs

pseudopeptidiques sont greffés sur le support, les acides aminés X, $Y_1$ et $Y_2$, n et m, ou Y peuvent être sous toute forme précédemment décrite.

La présente description décrit également un composé de formule (Ibis) tel que défini précédemment, à l'exception des composés dont le support est un peptide linéaire. Notamment, le support (à l'exception des peptides linéaires), la façon dont les motifs pseudopeptidiques sont greffés sur le support, les acides aminés X, $Y_1$ et $Y_2$ ,n et m, ou Y peuvent être sous toute forme précédemment décrite.

La présente description décrit également le composé avec comme support un peptide cyclique Nucant 01 (Figure 2B).

L'invention concerne en outre un composé de formule (I) tel que défini précédemment, à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I), pour son utilisation en tant que médicament.

Notamment, le support (à l'exception de ceux exclus au paragraphe précédent), la façon dont les motifs pseudopeptidiques sont greffés sur le support, les acides aminés X, $Y_1$ et $Y_2$, n et m, ou Y peuvent être sous la forme de tout mode de réalisation précédemment décrit.

Notamment, le support peut être choisi parmi un peptide linéaire comprenant une séquence d'acides aminés choisie parmi Aib-K-Aib-G (SEQ ID NO : 6) ou K-Aib-G (SEQ ID NO : 7), un peptide cyclique, un peptoïde linéaire ou cyclique, un foldamère, un polymère linéaire ou un dendrimère sphérique, un sucre, ou une nanoparticule.

Dans certains cas avantageux, le support peut être un peptide linéaire constitué d'une séquence d'acides aminés choisie parmi SEQ ID NO :8, et SEQ ID NO :9, SEQ ID NO : 14, SEQ ID NO : 15, SEQ ID NO :18, et SEQ ID NO :19.

De tels composés, en tant que médicaments, incluent notamment les composés avec comme support un peptide linéaire de structure hélicoïdale tel que Nucant 2 (SEQ ID NO : 20 et Figure 2C) et Nucant 3 (SEQ ID NO : 21 et Figure 2D) décrits précédemment. Ils incluent également le composé Nucant 6 (SEQ ID NO : 16, et voir exemple 4 et Figure 2E).

L'invention concerne également le composé Nucant 7 (SEQ ID NO : 17 et Figure 2F), pour son utilisation en tant que médicament.

La présente description décrit en outre un composé de formule (Ibis) tel que défini précédemment, à l'exception des composés dont le support est un peptide non cyclique comprenant une séquence d'acides aminés choisie parmi KPG, KGP, KGC, ou $KX_1$ $KX_4KX_1$ K, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I), pour son utilisation en tant que médicament. Notamment, le support (à l'exception de ceux exclus au paragraphe précédent), la façon dont les motifs pseudopeptidiques sont greffés sur le support, les acides aminés X, $Y_1$ et $Y_2$, n et m, ou Y peuvent être sous toute forme précédemment décrite.

La présente description décrit également un composé de formule (Ibis) tel que défini précédemment, à l'exception des composés dont le support est un peptide linéaire, pour son utilisation en tant que médicament. Notamment, le support (à l'exception des peptides linéaires), la façon dont les motifs pseudopeptidiques sont greffés sur le support, les acides aminés X, $Y_1$ et $Y_2$, n et m, ou Y peuvent être sous la forme de tout mode de réalisation précédemment décrit.

La présente description décrit également le composé avec comme support un peptide cyclique Nucant 01 (Figure 2B), pour son utilisation en tant que médicament.

L'invention concerne en outre une composition pharmaceutique comprenant un composé de formule (I) tel que défini précédemment, à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I).

Notamment, le support (à l'exception de ceux exclus au paragraphe précédent), la façon dont les motifs pseudopeptidiques sont greffés sur le support, les acides aminés X, $Y_1$ et $Y_2$, n et m, ou Y peuvent être sous la forme de tout mode de réalisation précédemment décrit.

Notamment, le support peut être choisi parmi un peptide linéaire comprenant une séquence d'acides aminés choisie parmi Aib-K-Aib-G (SEQ ID NO : 6) ou K-Aib-G (SEQ ID NO : 7), un peptide cyclique, un peptoïde linéaire ou cyclique, un foldamère, un polymère linéaire ou un dendrimère sphérique, un sucre, ou une nanoparticule.

Dans certains cas avantageux, le support peut être un peptide linéaire constitué d'une séquence d'acides aminés choisie parmi SEQ ID NO : 8 et SEQ ID NO : 9, SEQ ID NO : 14, SEQ ID NO : 15, SEQ ID NO :18, et SEQ ID NO :19.

Les compositions pharmaceutiques selon l'invention peuvent notamment comprendre les composés avec comme support un peptide linéaire de structure hélicoïdale tels que Nucant 2 (SEQ ID NO : 20 et Figure 2C) et Nucant 3 (SEQ ID NO : 21 et Figure 2D) décrits précédemment. Elles peuvent également comprendre le composé Nucant 6 (SEQ

ID NO : 16, et voir exemple 4 et Figure 2E).

L'invention concerne également une composition pharmaceutique comprenant le composé Nucant 7 (SEQ ID NO : 17 et Figure 2F).

De telles compositions pharmaceutiques associent le ou les composés selon l'invention à un support pharmaceutiquement acceptable.

La présente description décrit en outre une composition pharmaceutique comprenant un composé de formule (Ibis) tel que défini précédemment, à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I). Notamment, le support (à l'exception de ceux exclus au paragraphe précédent), la façon dont les motifs pseudopeptidiques sont greffés sur le support, les acides aminés X, $Y_1$ et $Y_2$, n et m, ou Y peuvent être sous toute forme précédemment décrite.

La présente description décrit également une composition pharmaceutique comprenant un composé de formule (Ibis) tel que défini précédemment, à l'exception des composés dont le support est un peptide linéaire. Notamment, le support (à l'exception des peptides linéaires), la façon dont les motifs pseudopeptidiques sont greffés sur le support, les acides aminés X, $Y_1$ et $Y_2$, n et m, ou Y peuvent être sous la forme de tout mode de réalisation précédemment décrit.

La présente description décrit également une composition pharmaceutique comprenant le composé avec comme support un peptide cyclique Nucant 01 (Figure 2B).

La présente description décrit également l'utilisation d'un composé synthétique multivalent comprenant ou constitué d'un support sur lequel sont greffés au moins 3 motifs pseudopeptidiques, ledit composé étant de formule (Ibis) : $[(X)_n—Y_1\overset{\Psi}{—}(Z)_i—Y_2\text{-}(X)_m]_k$—Support, où chaque X représente indépendamment un acide aminé quelconque ; $Y_1$ et $Y_2$ sont indépendamment choisis parmi les acides aminés à chaîne latérale basique ; Z est choisi parmi une proline, éventuellement substituée en $\gamma$, $\beta$ ou $\delta$ ; un acide aminé naturel ou non N-alkylé ; un acide aminé dialkylé ; un acide aminé dialkylé cyclique ; l'acide pipécolique ou l'un de ses dérivés ; n et i sont indépendamment 0 ou 1 ; m est un entier entre 0 et 3 ; k est un entier supérieur ou égal à 3 ; et $\Psi$ représente une liaison peptidique modifiée, significativement plus résistante à au moins une protéase qu'une liaison peptidique normale, pour la fabrication d'un médicament destiné au traitement d'une maladie inflammatoire.

Il est maintenant bien connu que les maladies chroniques inflammatoires, et notamment les maladies auto-immunes à médiation cellulaire sont en partie induites par des cytokines. Notamment, les résultats obtenus dans différents modèles animaux expérimentaux ont souligné le rôle des cytokines dans la pathogenèse de la maladie (17). Par exemple, les cytokines pro-inflammatoires telles que le facteur de nécrose tumorale $\alpha$ (TNF-$\alpha$), l'interleukine 1(IL-1), l'IL-6, l'IL-15 et l'IL-18 régulent les réponses immunitaires et inflammatoires chez les patients souffrant de polyarthrite rhumatoïde. En particulier, le TNF-$\alpha$ et l'IL-1$\beta$ favorisent la destruction du cartilage et de la moelle osseuse.

En outre, au cours du processus inflammatoire, l'endothélium vasculaire exprime différentes chimiokines et molécules d'adhésion qui participent au recrutement sélectif des leucocytes au niveau du foyer inflammatoire (18).

IL-8 est une chimiokine C-X-C qui initie l'activation et le recrutement sélectif des leucocytes au niveau des sites tissulaires de l'inflammation. Lorsqu'elle est exprimée à des niveaux élevés, l'IL-8 peut avoir des conséquences pathologiques pour l'organisme. Le Lipopolysaccharide (LPS) et les cytokines pro-inflammatoires telles que le TNF-$\alpha$ et l'IL-1 induisent la sécrétion d' IL-8 par de nombreux types cellulaires, en particulier les cellules endothéliales (19).

La molécule d'adhésion cellulaire intercellulaire-1 (ICAM-1) est une protéine de type immunoglobuline exprimée à la surface de plusieurs types cellulaires incluant les cellules endothéliales et les cellules impliquées dans la réponse immunitaire. Elle joue un rôle important dans l'adhésion et la migration des leucocytes vers les sites d'inflammation (20).

Les cytokines pro-inflammatoires sont également impliquées dans la réponse inflammatoire généralisée (choc sceptique) induite par les infections bactériennes. Le Lipopolysaccharide (LPS) est un composant intégral de la membrane externe des bactéries gram-négatives. Cette molécule immuno-stimulatrice est un facteur qui contribue de façon majoritaire à l'initiation d'une réponse inflammatoire généralisée appelée choc sceptique, qui accompagne souvent une bactériémie gram-négative. Le LPS a notamment comme propriété biologique de stimuler la production de cytokines telles que le TNF-$\alpha$, l'IL-1 et l'IL-6 par les cellules lymphoréticulaires. L'induction de ces cytokines joue un rôle pivot dans le développement du syndrome sceptique, puisque l'administration de TNF-$\alpha$ seul peut conduire à un état sceptique et à la mort, le TNF-$\alpha$ pouvant induire la production d'IL-1 et d'IL-6 in vivo. En outre, dans des modèles animaux, un pré-traitement avec un anticorps anti-TNF-$\alpha$ et un antagoniste du récepteur de l'IL-1 permettent de protéger les animaux contre les effets létaux du LPS (21).

La septie sévère est associée avec une réaction inflammatoire explosive et à des déficiences d'organes, et également souvent associée avec un fort taux de mortalité. Elle peut être consécutive à une infection bactérienne, fongique ou virale. Cette réponse est marquée par la sécrétion séquentielle de cytokines pro-inflammatoires puis anti-inflammatoires. Parmi les cytokines pro-inflammatoires les plus importantes, se trouvent le TNF-$\alpha$ et l'IL-$\beta$. Jusqu'à

présent, aucun des essais cliniques impliquant des réactifs anti-LPS ou anti-cytokine n'a été couronné de succès (22, 23).

Les cytokines pro-inflammatoires semblent également jouer un rôle physiopathologique chez les patients souffrant de cardite ou inflammation du coeur, qui se manifeste par une inflammation de l'endocarde (endocardite) ou une inflammation du péricarde (péricardite) ou une inflammation du muscle cardiaque (myocardite). Par exemple, le niveau sérique d'IL-6 est significativement augmenté chez des patients souffrant d'endocardite infectieuse qui peut être induite notamment par une infection par *Staphylococcus aureus.* De cette façon, un niveau sérique élevé d'IL-6 peut suggérer l'existence d'une péricardite infectieuse et être utilisé comme une aide au diagnostic et au suivi du traitement de la maladie (24,25)

Au vu de l'importance du rôle des cytokines pro-inflammatoires telles que le TNF-$\alpha$, l'IL-1, et l'IL-6 dans les maladies inflammatoires, des thérapies anti-cytokines inflammatoires comprenant des réactifs anti-TNF-$\alpha$, anti-IL-1 et anti-IL-6 ont été développées pour le traitement de patients souffrant de maladies inflammatoires (26).

Différents essais cliniques impliquant des réactifs anti-cytokine inflammatoire dans le traitement de maladies inflammatoires chroniques telles que la polyarthrite rhumatoïde et la maladie inflammatoire abdominale ont obtenu un certain succès : Etanercept (protéine de fusion TNF récepteur-P75 Fc), Infliximab (anticorps monoclonal chimérique anti-TNF-$\alpha$ humain), Adalimumab (anticorps monoclonal recombinant humain anti-TNF-$\alpha$ humain) et Anakinra (forme recombinante de l'antagoniste du récepteur IL-1$\beta$ humain)(22).

Cependant, tous les réactifs anti-cytokine inflammatoire disponibles jusqu'à présent sont des protéines, et souffrent donc des inconvénients associés de façon générale avec les médicaments protéiques. En particulier, ces médicaments ont un coût très élevé et sont difficiles à produire en grande quantité.

Par conséquent, il existe un réel besoin pour des molécules de petit poids moléculaire capables de cibler spécifiquement les voies de synthèse des cytokines pro-inflammatoires.

De façon surprenante, les inventeurs ont trouvé que les composés de formule (Ibis) tels que décrits précédemment possèdent une activité anti-inflammatoire, et notamment inhibent la production de TNF-$\alpha$, d'IL-6 et d'IL-8, ainsi que l'expression d' ICAM-1 par différents types cellulaires stimulés par du LPS. Ces composés sont très intéressants puisque, comme indiqué précédemment :

- aucun effet toxique de ces composés n'a été observé par les inventeurs, ni in vitro, ni in vivo ;
- ces composés sont facilement synthétisables, y compris à l'échelle industrielle, dans des conditions facilement maîtrisables ;
- ces composés possèdent en eux-mêmes une biodisponibilité in vivo suffisante pour ne pas nécessiter le développement de forme galénique particulière.

La présente description décrit donc l'utilisation d'un composé synthétique multivalent de formule (Ibis) tel que défini précédemment, pour la préparation d'un médicament destiné au traitement des maladies inflammatoires.

Notamment, dans la formule (Ibis), le support ; le nombre de motifs pseudopeptidiques k ; la façon dont les motifs pseudopeptidiques sont greffés sur ce support ; les acides aminés X, Y1 et Y2 ; n et m, ou $\Psi$ peuvent être sous toute forme précédemment décrite.

Par « maladie inflammatoire », on entend toute maladie dans laquelle une réaction inflammatoire a des conséquences pathologiques pour l'organisme. Notamment, les maladies inflammatoires incluent les maladies autoimmunes (telle que le lupus ou la polyarthrite rhumatoïde), la septicémie, le choc septique, les maladies inflammatoires cardiaques (cardites, et notamment endocardite, péricardite, myocardite, en particulier les endocardites d'origine infectieuse, telles que celles induites par *Staphylococcus aureus*), le rejet de greffe, les traumatismes, les maladies inflammatoires articulaires (notamment les différents types d'arthrite), les maladies inflammatoires du système gastro-intestinal (notamment les colites, entérites, gastrites, gastro-entérites, et les maladies inflammatoires chroniques de l'intestin (MICI) telles que la maladie de Crohn et la recto-colite hémorragique (RCH)), les maladies inflammatoires de la peau (eczéma, dermatite de contact allergique, psoriasis, dermatose), les maladies inflammatoires des voies respiratoires (asthme, bronchite chronique, en particulier la bronchite chronique obstructive (COPD)), et les allergies.

La maladie inflammatoire peut notamment être une maladie autoimmune, en particulier le lupus ou la polyarthrite rhumatoïde. La maladie inflammatoire peut également être le choc septique. La maladie inflammatoire peut en outre être une endocardite, en particulier une endocardite d'origine infectieuse, notamment du type de celles induites par *Staphylococcus aureus.*

Les avantages de la présente invention sont illustrés dans les figures et les exemples ci-après.

**DESCRIPTION DES FIGURES**

**Figure 1. A.** Structure de la protéine nucléoline. La nucléoline humaine est constituées de 707 acides-aminés. On

peut décomposer la nucléoline en 2 domaines majeurs (3,4) : N-terminal (aa 1-308) et C-terminal (309-706). Le domaine N-terminal comporte 4 longs domaines acides, constitués d'une répétition ininterrompue d'acides glutamiques et d'acides aspartiques (A1, A2, A3, A4). Le domaine C-terminale constitué d'une alternance de régions hydrophobes et hydrophiles formant 4 domaines de liaison à l'ARN nommés RBDs (pour « RNA Binding Domains » : I, II, III, IV) et à son extrémité (aa 644-707) se trouve le domaine RGG fortement basique composé de répétitions Arg-Gly-Gly. **B.** identification du domaine de liaison du composé HB19 à la nucléoline : le domaine RGG. Des constructions de la nucléoline correspondant aux parties N- et C- terminales ont été réalisées par la transcription/traduction *in vitro* dans le système employant les lysats des réticulocytes du lapin. Ainsi, la nucléoline complète et les parties N- et C- terminales contenant les acides aminés 1-707, 1-308 et 309-707 respectivement marqués à la [$^{35}$S] Met/Cys ont été produits. Les produits bruts marqués ont ensuite été incubés avec HB 19-biotinylé, et les complexes purifiés sur une colonne d'avidine-agarose. Comme attendu, la nucléoline complète interagit avec HB19. En revanche, la partie N-terminale de la nucléoline riche en résidus acides n'interagit pas du tout avec le composé, alors que la partie C-terminale interagit avec HB19 (14). Ayant identifié que la partie C-terminale de la nucléoline contient la cible d'HB19, différentes constructions (N° 1 à 9) de cette région de la nucléoline ont été réalisées. La première construction correspond à l'ADNc codant pour la partie C-terminale de la nucléoline humaine, comprenant les 4 RBDs et le domaine RGG, en fusion avec la protéine GST (Glutathione S-Transferase) pour permettre la détection avec des anticorps dirigés contre la GST. Les autres constructions, également en fusion avec la GST, correspondent à cette même partie mais tronquée d'un ou plusieurs domaines. Toutes ces protéines sont générées chez *E.coli.* La capacité d'HB19 d'interagir avec chaque construction a été testée en incubant les extraits bruts des bactéries, exprimant les différentes constructions de la nucléoline, avec HB19-biotinylé qui ensuite a été purifié par fixation sur Avidine-agarose. Ces échantillons ont ensuite été analysés par gel de polyacrylamide et la GST a été révélée par immunodétection (Western Blot) en utilisant des anticorps anti-GST. Les résultats démontrent que la présence du domaine RGG est nécessaire pour l'interaction avec HB19 avec la partie C-terminale de la nucléoline. De plus, le domaine RGG seul est suffisant pour cette interaction.

**Figure 2. A.** Structure du composé HB-19. **B.** Structure du composé trivalent Nucant 01 ayant pour support un hexapeptide cyclique constitué en alternance de résidus alanine (A) de configuration D et de résidus lysine (K) de configuration L. Trois motifs pseudopeptidiques K$\Psi$PR (avec $\Psi$ = $CH_2$-N) sont liés covalamment sur le $\varepsilon$ amino groupe de chacun des 3 résidus lysine. **C.** Structure du composé pentavalent Nucant 2 (SEQ ID NO:10) ayant pour support un peptide linéaire adoptant une structure hélicoïdale de séquence SEQ ID NO :8 sur lequel 5 motifs pseudopeptidiques K$\Psi$PR (avec $\Psi$ = $CH_2$-N) sont liés covalamment sur le $\varepsilon$ amino groupe de chacun des 5 résidus lysine, Ac représente un groupement $CH_3$-CO-. **D.** Structure du composé pentavalent Nucant 3 (SEQ ID NO :11) ayant pour support un peptide linéaire adoptant une structure hélicoïdale de séquence SEQ ID NO:9 sur lequel 5 motifs pseudopeptidiques K$\Psi$PR (avec $\Psi$ = $CH_2$-N) sont liés covalamment sur le $\varepsilon$ amino groupe de chacun des 5 résidus lysine, Ac représente un groupement $CH_3$-CO-. **E.** Structure du composé hexavalent Nucant 6 (SEQ ID NO :16) ayant pour support un peptide linéaire adoptant une structure hélicoïdale de séquence SEQ ID NO :15 sur lequel 6 motifs pseudopeptidiques K$\Psi$PR (avec $\Psi$ = $CH_2$-N) sont liés covalamment sur le $\varepsilon$ amino groupe de chacun des 6 résidus lysine, Ac représente un groupement $CH_3$-CO-. **F.** Structure du composé hexavalent Nucant 7 (SEQ ID NO :17) ayant pour support un peptide linéaire de séquence SEQ ID NO:13 sur lequel 6 motifs pseudopeptidiques K$\Psi$PR (avec $\Psi$ = $CH_2$-N) sont liés covalamment sur le $\varepsilon$ amino groupe de chacun des 6 résidus lysine, Ac représente un groupement $CH_3$-CO-.

**Figure 3.** Effet de **A.** l'anti-nucléoline (anti Nu), **B.** des IgG isotypiques , **C.** du peptide F3, et **D.** d'HB-19 sur la prolifération des cellules NIH-3T3 induite par l'HARP. Des cellules NIH-3T3 quiescentes sont stimulées ou non par 4 nM d'HARP en présence ou non d'HB19 aux concentrations indiquées. Après 24 heures d'incubation, la prolifération cellulaire est déterminée par mesure de l'incorporation de thymidine tritiée. Les résultats sont présentés en pourcentage par rapport au témoin stimulé par l'HARP (100%). SEM (barre), (**p<0.01 et ***p<0.001).

**Figure 4.** Effet d'un pré-traitement d'une heure d'HB-19 sur la prolifération induite par HARP sur les cellules NIH-3T3. Des cellules NIH-3T3 sont traitées avec HB19 pendant 1 heure puis lavées et stimulées ou non avec 3,6 nM d'HARP. Après 24 heures d'incubation, la prolifération cellulaire est estimée par mesure de l'incorporation de thymidine tritiée. Les résultats sont présentés en pourcentage par rapport au témoin stimulé par l'HARP (100%). SEM (barre), (**p<0.01 et ***p<0.001).

**Figure 5.** Effet de HB-19 sur la prolifération des cellules NIH-3T3 stimulées par 0.2 nM FGF-2 (**A**) ou par 5% de sérum de veau foetal (**B**). Des cellules NIH-3T3 quiescentes sont stimulées par du FGF-2 ou par 5% de sérum en présence ou non d'HB19 aux concentrations indiquées. Après 24 heures d'incubation, la prolifération cellulaire est déterminée par mesure de l'incorporation de thymidine tritiée. Les résultats sont présentés en pourcentage par rapport au témoin stimulé par l'HARP (100%). SEM (barre), (**p<0.01 et ***p<0.001).

**Figure 6.** Effet de l'anti-nucléoline (anti Nu) (**A**), des IgG isotypiques (**B**) et d'HB-19 (**C**) sur la croissance des MDA-MB231 sur agar mou. Les cellules MDA-MB231 sont ensemencées dans le milieu de culture à 0.35% d'agar sur une matrice d'agar à 0.6%. Après 10 jours de culture les colonies ayant un diamètre supérieur à 50 $\mu$m sont

comptées. 5 champs par puits et chaque point est réalisée en triplicate, (**p<0.01).

**Figure 7.** Effet de l'anti-nucléoline (anti Nu) (**A**) et d'HB-19 (**B**) sur la croissance des B16-BL6 sur agar mou. Les cellules B16-BL6 sont ensemencées dans le milieu de culture à 0.35% d'agar sur une matrice d'agar à 0.6%. Après 10 jours de culture les colonies ayant un diamètre supérieur à 50 μm sont comptées. 5 champs par puits et chaque point est réalisé en triplicate, (*p<0.05 et **p<0.01).

**Figure 8.** Effet d'HB-19 sur l'angiogenèse. **A.** Effet d'HB19 testé sur la prolifération in vitro de cellules HUVEC. 20000 cellules HUVEC ont été mises en culture par puits et le composé HB19 a été ajouté tous les jours à différentes concentrations. Les cellules ont été comptées après 6 jours de traitement. **B.** Effet d'HB19 (1 μM) sur la différenciation des cellules HUVEC dans un gel tridimensionnel de collagène en culture en présence des facteurs angiogènes HARP (1 nM), VEGF (1 nM) et FGF-2 (3 nM) a également été testé. Au bout de 4 jours, les structures de réseaux tubulaires ont été comptées. Les résultats sont présentés en unités arbitraires. C. Effet d'HB-19 sur l'angiogenèse induite par HARP ou par FGF-2 dans un modèle d'angiogenèse in vivo (/matrigel « plug assay »). Le matrigel (300 μl) contenant les molécules indiquées est injecté en sous-cutané chez la souris. Après une semaine, les souris sont sacrifiées, le matrigel est prélevé et des coupes de 8 μm d'épaisseur sont réalisées. Après coloration, le nombre de cellules endothéliales est estimé par analyse d'image. Pour chaque matrigel, 5 coupes sont analysées et 4 souris (matrigel) par point expérimentale.

**Figure 9.** Effet de HB-19 sur la croissance tumorale dans un modèle de xénogreffe de MDA-MB231. Les cellules MDA-MB231 de carcinome mammaire humain sont injectées en sous cutané à des souris athymique nude. Quand la tumeur atteint un volume de 200 mm3, les souris sont traitées par voie sous-cutanée comme indiqué sur le graphique en **A. B.** Observation et mesure des tumeurs sur les souris sacrifiées à jour 40. **C.** Poids des tumeurs chez les souris sacrifiées à jour 40.

**Figure 10.** Action d'HB-19 sur la croissance tumorale dans un modèle de xénogreffe de MDA-MB231. Évolution de la croissance tumorale. Injections intrapéritonéales (IP) ou sous-cutanées (SC).

**Figure 11.** Effet d'HB-19 sur des cellules tumorales métastasique dans un modèle de xénogreffe de MDA-MB231. La recherche des cellules MDA-MB231 dans le sang de souris xénogreffées et traitées ou non avec HB-19 a été réalisé par cytométrie de flux (FACS) à l'aide d'anticorps dirigé contre le HLA-DR. Les cellules $HLA\text{-}DR^+$ sont entourées et le pourcentage de cellules $HLA\text{-}DR^+$ parmi les cellules du sang périphérique est indiqué. **A.** Sang de souris sans xénogreffe de MDA-MB231, non traitées, pourcentage de cellules $HLA\text{-}DR^+$ parmi les cellules du sang périphérique : 0,36% **B.** Sang de souris avec xénogreffe de MDA-MB231, non traitées, pourcentage de cellules $HLA\text{-}DR^+$ parmi les cellules du sang périphérique : 22,2% **C.** Sang de souris avec xénogreffe de MDA-MB231, traitées par HB19 en sous-cutané, pourcentage de cellules $HLA\text{-}DR^+$ parmi les cellules du sang périphérique : 0,1% **D.** Sang de souris avec xénogreffe de MDA-MB231, traitées par HB19 en intrapéritonéal, pourcentage de cellules $HLA\text{-}DR^+$ parmi les cellules du sang périphérique : 0,31 %.

**Figure 12.** Effet d'HB-19 et de Nucant 01 sur la prolifération de cellules NIH-3T3 induite par l'HARP. Des cellules NIH-3T3 quiescentes ont été stimulées ou non par 4 nM d'HARP en présence d'HB19 ou de Nucant 01 aux concentrations indiquées. Après 24 heures d'incubation, la prolifération cellulaire a été déterminée par mesure de l'incorporation de thymidine tritiée. Les résultats (moyenne de 3 points) sont présentés en pourcentage de la prolifération cellulaire par rapport au témoin stimulé par l'HARP en absence de HB 19 et Nucant 01 (prolifération cellulaire 100%).

**Figure 13.** Effet d'HB-19 de Nucant 2 et Nucant 3 sur la prolifération de cellules NIH-3T3 induite par l'HARP. Des cellules NIH-3T3 quiescentes ont été stimulées ou non par 4 nM d'HARP en présence d'HB19, Nucant 2, ou Nucant 3 aux concentrations indiquées (0,1 ; 0,25 ; et 0,5 μM). Après 24 heures d'incubation, la prolifération cellulaire des cellules NIH-3T3 a été déterminée par mesure de l'incorporation de thymidine tritiée. Les résultats (moyenne de 3 points) sont présentés en pourcentage de la prolifération cellulaire par rapport au témoin stimulé par l'HARP (prolifération cellulaire 100%). Les concentrations IC50 (concentration induisant une inhibition de 50% de la prolifération cellulaire par rapport au témoin stimulé par l'HARP) sont également indiquées.

**Figure 14.** Effet de NUCANT 3, 6 et 7 sur la prolifération des cellules NIH-3T3 induite par 5% de SVF. Des cellules NIH-3T3 rendues quiescentes par privation de sérum sont stimulées par 5 % de SVF en présence ou non de différentes concentrations de NUCANT 3, 6 ou 7 allant de 0,125 à 2 μM. Après 24 heures d'incubation, la prolifération cellulaire est déterminée par mesure de l'incorporation de thymidine tritiée. Les résultats sont présentés en pourcentage par rapport aux cellules témoins stimulées par 5 % de SVF. L'$ID_{50}$ est indiqué par une ligne pointillée.

**Figure 15.** Nucant 6 et Nucant 7 présentent une activité anti-nucléoline de surface supérieure à HB-19. $ID_{50}$: la concentration en μM qui inhibe 50% la nucléoline de surface. $ID_{95}$: la concentration en μM qui inhibe 95% la nucléoline de surface.

**Figure 16.** Effet inhibiteur d'HB-19, de Nucant 3, de Nucant 6 et de Nucant 7 sur l'expression cellulaire de la nucléoline par des cellules de MDA-MB 231. Des cellules MDA-MB 231 ont été cultivées dans des flacons de 75 $cm^2$ dans du DMEM contenant du sérum de veau foetal à 10%. Après 2 jours de culture, les cellules subconfluentes (environ 3 x $10^6$ cellules par flacon) ont été traitées avec 10 μM d'HB-19 (piste 1), de Nucant 3 (piste 2), de Nucant

6 (piste 3) ou de Nucant 7 (piste 4) pendant 24 ou 48 heures. Les pistes C, représentent les cellules non traitées. Après 24 ou 48 heures de traitement, les cellules ont été lavées par du PBS et incubées avec 10 ml de DMEM contenant 1% de sérum de veau foetal et HB-19 biotinylé (5 $\mu$M) pendant 45 minutes à la température ambiante. Après un lavage intensif du tapis cellulaire avec du PBS contenant 1mM EDTA (PBS-EDTA), des extraits cytoplasmiques ont été préparés à l'aide d'un tampon de lyse contenant 20 mM Tris HC1, pH 7.6, 150 mM de NaCl, 5 mM de $MgCl_2$, 0.2 mM de fluorure de phenylmethylsulfonyl, 5 mM de P-mercaptoéthanol, aprotinin (1000 U/ml) et 0.5% Triton X-100. Le complexe formé entre la nucléoline de surface et HB-19 biotinylé a été isolé par purification des extraits en utilisant de l'avidine-agarose (100 $\mu$l; ImmunoPure Immobilized Avidin, Pierce Chemical Company, USA) dans PBS-EDTA. Après 2 h d'incubation à 4°C, les échantillons d'avidine-agarose ont été lavés intensivement avec PBS-EDTA. Ces échantillons contenant la nucléoline de surface purifiée (A; matériel correspondant à 2 x $10^6$ cellules) et des extraits bruts de cellules (B et C; matériel correspondant à 4 x $10^5$ cellules) ont été dénaturés par chauffage dans le tampon d'électrophorèse contenant du SDS et analysés par SDS-PAGE. La présence de nucléoline de surface a été révélée par immunoblotting en utilisant un anticorps monoclonal D3 (A et B). L'analyse électrophorétique après coloration au bleu de Coomassie est montrée en C. La piste M correspond aux marqueurs de poids moléculaire.

**Figure 17.** Inhibition de l'angiogénèse dans un modèle ex vivo de la CAM. 20 $\mu$l d'eau contenant ou non (contrôle) HB-19 (10 $\mu$M; 0,6 $\mu$g) ou Nucant 7 (10 $\mu$M; 0,8 $\mu$g) sont déposés à la surface de la CAM. L'observation des vaisseaux est réalisée après 48 h d'incubation.

**Figure 18.** Inhibition par HB-19 de la production de TNF-$\alpha$ par des cellules mononucléées du sang périphérique (PBMC) humaines primaires stimulées par différentes préparations de LPS. Des PBMC ont été isolés par centrifugation sur gradient de densité Ficoll à partir de sang total humain EDTA-potassium, et re-suspendus dans du milieu RPMI 1640 contenant 1% de sérum humain AB (Invitrogen). Des cellules à une concentration de $10^6$ cellules/0,5 ml, en absence (0) ou en présence (1 et 5 $\mu$M) de HB-19, ont été stimulées avec 100 ng/ml de LPS d'*Escherichia coli* de type 0111 :B4 et 055 :B5, et de LPS *Salmonella enterica* de sérotype Re 595. Les mêmes PBMC ont été stimulés avec de la PMA :Ionomycin (Phorbol 12-myristate 13-acetate :Ionomycin) à 20 ng/ml :1 $\mu$M. Les cultures de PBMC ont été incubées à 37 °C dans un incubateur à 5% de $CO_2$ et le niveau de protéine TNF-$\alpha$ a été mesuré par ELISA dans des sumageants de culture récoltés après 20 heures d'incubation.

**Figure 19.** Inhibition par HB-19 de la production de TNF-$\alpha$ et d'IL-6 par des macrophages murins du péritoine primaires stimulés par du LPS. Des macrophages murins du péritoine, en l'absence (-) ou en présence (+) de 4 $\mu$M de HB-19 ont été soit non stimulés (B4 0) ou stimulés avec du LPS d'Escherichia coli de sérotype 0111:B4 à 100 ng/ml (B4 100) et 1000 ng/ml (B4 1000). Les cultures cellulaires ont été incubées à 37 °C dans un incubateur à 5% de $C0_2$ pendant 20 heures et les niveaux de TNF-$\alpha$ (A) et d'IL-6 (**B**) ont été mesurés par ELISA.

**Figure 20.** Inhibition par Nucant 7 de la production de TNF-$\alpha$ et d'IL-6 par des macrophages du péritoine murin primaires stimulés par du LPS. Des macrophages du péritoine murin, en l'absence (-) ou en présence (+) de 10 $\mu$M de Nucant 7 ont été soit non stimulés (-) ou stimulés (+) avec du LPS d'*Escherichia Coli* de sérotype 0111 :B4 à 10 ng/ml, 100 ng/ml et 1000 ng/ml. Les cultures cellulaires ont été incubées à 37 °C dans un incubateur à 5% de CO2 pendant 20 heures, et les niveaux de protéine TNF-$\alpha$ (**A**) et d'IL-6 (**B**) ont été mesurés par ELISA.

**Figure 21.** Inhibition par HB-19 de la production d'IL-8 et de l'expression d'ICAM-1 par des cellules endothéliales vasculaires ombilicales humaines (HUVEC) stimulées par du LPS. Des cellules HUVEC, à 10 000 cellules/$cm^2$, ont été mises en culture dans des plaques 96-puit dans du milieu EBM-2 contenant 2% de sérum de veau foetal. Les cellules en absence ou en présence de 5 $\mu$M de HB-19, ont été stimulées par du LPS d'*Escherichia coli* de sérotype 055 :B5 à 100 ng/ml. Les cultures cellulaires ont été incubées à 37 °C dans un incubateur à 5% de $CO^2$ pendant 20 heures et les niveaux de protéine IL-8 et ICAM-1 ont été mesurés par ELISA. Des cellules HUVEC en absence ou en présence de 5 $\mu$m de HB-19 ont été utilisées comme contrôle pour montrer les niveaux de base.

**Figure 22.** Inhibition par HB-19 de la production de TNF-$\alpha$ et d'IL-6 par des cellules mononucléées du sang périphérique (PBMC) humaines primaires stimulées par des des bactéries *Staphylococcus aureus* inactivées par la chaleur (HKSA, pour « heat-killed *Staphylococcus aureus* »)._Des PBMC ont été isolés par centrifugation sur gradient de densité Ficoll à partir de sang total humain EDTA-potassium, et resuspendus dans du milieu RPMI 1640 contenant 1% de sérum humain AB (Invitrogen). Des cellules à une concentration de $10^6$ cellules/0,5 ml, en absence (Contrôle) ou en présence (10 $\mu$M) de HB-19, Nucant 3, Nucant 6, ou Nucant 7, ou de dexamethasone (Dex. 1 $\mu$g/ml)) ont été stimulées avec $10^8$ particules HKSA/ml (InvivoGen, San Diego, USA). Les cultures de PBMC ont été incubées à 37 °C dans un incubateur à 5% de $CO_2$ et le niveau de protéine TNF-$\alpha$ (**A**) et d'IL-6 (**B**) a été mesuré par ELISA dans des surnageants de culture récoltés après 20 heures d'incubation

## EXEMPLES

### *EXEMPLE 1. Activité anti-tumorale du composé pentavalent HB19*

### 1.1 Effet du composé pentavalent HB19 sur la croissance des cellules tumorales in vitro

#### 1.1.1 Rôle de la nucléoline de surface dans la prolifération cellulaire dépendante d'ancrage et activité inhibitrice d'HB-19 sur cette prolifération

Dans une série d'expériences le rôle de la nucléoline dans l'activité biologique de la molécule HARP a été étudié : l'activité mitogène de l'HARP, testée par mesure de l'incorporation de thymidine tritiée par des cellules NIH 3T3, a été évaluée en présence ou en absence d'un anticorps monoclonal qui reconnaît spécifiquement la nucléoline. Les résultats montrent que cet anticorps inhibe d'une façon dose dépendante l'activité mitogène de l'HARP sur des cellules NIH 3T3 (Figure 3A).

En effet, l'adjonction de 50 nM d'anticorps anti-nucléoline inhibe complètement l'activité mitogène induite par 4 nM d'HARP, tandis que des anticorps non spécifiques de la nucléoline du même isotype n'ont aucun effet sur l'induction de la prolifération induite par HARP et ceci quelque soit la concentration d'immunoglobulines utilisées, montrant la spécificité de l'inhibition observée (Figure 3B).

Le peptide F3 et le composé HB19 sont deux ligands de la nucléoline. Le peptide F3 se lie à la partie N-terminale de la nucléoline qui contient plusieurs régions riches en acides aminés acides (9), contrairement au composé HB19 qui se lie au domaine RGG localisé dans la partie C-terminale de la nucléoline (voir Figure 1). Il a par ailleurs été montré que la liaison spécifique du composé HB19 à la surface des cellules n'est pas affectée par la présence du peptide F3 (expérience realisée par FACS par les inventeurs).

Les inventeurs ont recherché si le peptide F3 et le composé pentavalent HB19 sont capables d'inhiber la prolifération cellulaire de cellules NIH-3T3 induite par le facteur de croissance HARP. Dans la même série d'expérience, les effets du composé HB-19 et du peptide F3, qui lient spécifiquement la nucléoline de surface, ont donc été testés.

Les résultats concernant le peptide F3 sont présentés sur la Figure 3C et indiquent sans ambiguïté que le peptide F3, comme les anticorps IgG contrôles, ne provoque pas d'inhibition de la prolifération des NIH 3T3 induite par HARP.

Les résultats concernant HB19 sont présentés sur la Figure 3D et indiquent au contraire qu'HB-19 inhibe de façon dose dépendante la stimulation de la prolifération des NIH 3T3 induite par HARP. L'adjonction de 0,5 μM d'HB-19 inhibe 81% de l'effet induit par 4 nM d'HARP. Cela démontre clairement qu'il ne suffit pas de disposer d'un ligand de la nucléoline capable d'être internalisé pour induire une inhibition de la prolifération, et suggère que pour être efficace, un ligand de la nucléoline devrait être multivalent et se lier à un ou plusieurs motifs RGG du domaine C-terminal d'une ou plusieurs molécules de nucléoline.

De plus, il est intéressant de noter qu'une inhibition similaire est observée lorsque les cellules sont pré-traitées pendant 1 heure par des concentrations variables d'HB-19, lavées puis stimulées par HARP (Figure 4). Ce résultat démontre qu'HB-19 se lie à la nucléoline de surface présent sur les NIH 3T3 bloquant ainsi la stimulation de la prolifération cellulaire induite par HARP 1 heure après.

Dans le but d'étudier si l'effet de HB-19 sur l'inhibition de la prolifération cellulaire est spécifique d'un facteur de croissance donné comme l'HARP, deux séries d'expérimentation ont été réalisées consistant à étudier l'effet d'HB-19 sur des cellules NIH 3T3 stimulées par:

- du FGF-2, un autre facteur de croissance, ou
- 5% de sérum de veau foetal, qui contient un mélange de différents facteurs de croissance.

Les résultats de ces expériences sont présentées sur la Figure 5 et montrent qu'HB-19, à une concentration de 0,5 μM, est capable d'inhiber la prolifération de cellules stimulées par du FGF-2 (A) ou par du sérum de veau foetal (B).

Ainsi, les résultats obtenus montrent globalement qu'HB-19 est capable d'inhiber in vitro la prolifération cellulaire de cellules tumorale et ceci, quelque soit l'inducteur de la prolifération cellulaire utilisé.

#### 1.1.2 Rôle de la nucléoline dans la prolifération cellulaire indépendante d'ancrage et activité inhibitrice d'HB-19 sur cette prolifération

Parallèlement à ces études portant sur la prolifération cellulaire, le rôle de la nucléoline sur la croissance indépendante d'ancrage, caractéristique phénotypique des cellules transformées, a été testé dans un modèle de croissance sur agar mou en utilisant la lignée de carcinome mammaire humain MDA-MB-231 ainsi qu'une lignée de mélanomes de souris B16-BL6.

Dans ces expériences, les cellules sont ensemencées sur un gel d'agar en présence ou en absence de concentration variable d'anticorps anti-nucléoline, d'immunoglobulines témoins ou du composé HB-19. Après 10 jours d'incubation à 37°C, le nombre de colonies présentes dans chaque boite de culture est compté. Comme le montre la Figure 6, le nombre de colonies est diminué de 60 % pour les cultures traitées par 0,1 μM d'anti-nucléoline (A) alors qu'aucun effet n'est observé lorsque les cultures sont traitées avec des immunoglobulines du même isotype (B).

Surtout, une inhibition du nombre de colonies par rapport au témoin est également observée pour les cultures traitées par HB-19 et ceci d'une façon dose dépendante. Ainsi, 59 % de diminution du nombre de colonies pour les cultures traitées par 1 μM d'HB-19 (Figure 6C) est observée.

Des résultats similaires ont été obtenus en utilisant des mélanomes murins comme les B16-BL6 comme cellules cibles. Les résultats sont présentés sur la Figure 7. L'examen des résultats montre qu'aussi bien l'anticorps anti-nucléoline (A) que la molécule HB-19 (B) inhibe la croissance sur agar mou des B16-BL6 et ceci d'une façon dose dépendante. Une inhibition de plus de 50 % du nombre de clones est observée en présence de 1 μM d'HB-19.

L'ensemble de ces résultats démontre que le composé HB-19 a un effet inhibiteur sur la croissance cellulaire indépendante d'ancrage et ceci dans deux modèles cellulaires, sur des carcinomes humains de glandes mammaires et des mélanomes de souris.

**1.2 <u>Effet du composé pentavalent HB19 sur l'angiogénèse induite pair des facteurs angiogènes</u>**

Etant donné que la nucléoline de surface est présente à la surface des cellules endothéliales activées (9), l'effet d'HB-19 sur la différenciation des cellules endothéliales a été testé.

Cet effet a d'abord été testé sur la prolifération in vitro de cellules endothéliales (cellules HUVEC : Human umbilical vein endothelial cells). 20000 cellules HUVEC ont été mises en culture par puits et le composé HB19 a été ajouté tous les jours à différentes concentrations. Les cellules ont été comptées après 6 jours de traitement. Les résultats sont présentés sur la Figure 8A et montrent que, contrairement à la préparation d'anticorps polyclonaux anti-nucléoline utilisée dans l'article de Huang et al (16), HB19 permet d'inhiber la prolifération des cellules endothéliales.

L'effet de la présence d'HB19 (1 μM) sur la différenciation des cellules HUVEC dans un gel tridimensionnel de collagène en culture en présence des facteurs angiogènes HARP (1 nM), VEGF (1 nM) et FGF-2 (3 nM) a également été testé. Au bout de 4 jours, les structures de réseaux tubulaires ont été comptées. Les résultats sont présentés sur la Figure 8B et montrent qu'HB19 inhibe également la différenciation des cellules HUVEC dans un gel tridimensionnel de collagène en culture en présence de facteurs angiogènes.

L'effet d'HB-19 sur la différenciation des cellules endothéliales a enfin été testé dans un modèle d'angiogénèse *in vivo.* Ce test mime les premières étapes de ce processus menant à la formation des vaisseaux sanguins.

Ce modèle d'angiogénèse expérimental consiste à injecter chez la souris, en sous cutané du matrigel contenant une substance à analyser pour ses propriétés stimulatrice ou inhibitrice de l'angiogénèse. Après une semaine, le matrigel est prélevé, des coupes histologiques sont réalisées et le nombre de cellules endothéliales (CD31+, facteur VIII +) est quantifié par analyse d'image après immunohistochimie. Comme le montre la Figure 8, HB-19 seul n'a pas d'effet sur le recrutement des cellules endothéliales dans le matrigel. Par contre il inhibe l'angiogénèse induite par HARP ou par le FGF-2.

L'analyse des résultats obtenus montre qu'HB-19 est capable d'inhiber l'angiogénèse induite par des facteurs proangiogénique comme le FGF-2 ou HARP. Ceci démontre un effet angiostatique général d'HB-19 qui cible spécifiquement les cellules endothéliales engagées dans l'angiogénèse.

Ainsi, le composé HB19 inhibe de façon drastique la prolifération et la différenciation des cellules HUVEC induite par HARP, VEGF et FGF-2, et possède donc un effet beaucoup plus prononcé que la préparation d'anticorps polyclonaux anti-nucléoline utilisée dans l'article de Huang et al (16).

Il existe plusieurs avantages à utiliser les cellules endothéliales comme cible anticancéreuse. Contrairement aux cellules tumorales présentant une instabilité génétique, les cellules endothéliales sont très stables génétiquement limitant ainsi les mécanismes de résistance. De plus la cible moléculaire étant la nucléoline de surface, HB-19 cible principalement les cellules endothéliales activées donc entrées dans un programme de néo-angiogénèse. Il est à noter que les cellules endothéliales issues des tumeurs se divisent 70 fois plus vite que les cellules endothéliales normales d'où un ciblage majoritaire sur les cellules endothéliales tumorales limitant ainsi les effets secondaires possibles.

**1.3 <u>Effet anti-tumoral du composé pentavalent HB19 in vivo</u>**

L'effet du composé pentavalent HB-19 sur la croissance tumorale *in vivo,* a été testé dans un modèle de croissance tumorale chez la souris athymique. Dans cette expérience, les cellules cibles sont des cellules issues du cancer de la glande mammaire humaine : les MDA-MB231.

Des lots de 4 souris athymiques (nude/nude) sont injectés au niveau de leur flanc avec $2x10^6$ cellules. Lorsque le volume des tumeurs ont atteint au moins 200 $mm^3$, les souris sont traitées ou non par injection au niveau de la tumeur

(voie péritumorale sous-cutanée) de 100 μl tous les deux jours d'une solution de PBS (lot contrôle) d'une solution d'HB-19 (5 mg/kg) ou d'un agent classiquement utilisé en clinique le tamoxifène (encore appelé taxol, 10 mg/kg). Aux jours 7, 14, 21, 28, 34 et 40, la taille de la tumeur est mesurée à l'aide d'un pied à coulisse.

Les résultats sont présentés sur la Figure 8 et indiquent que le peptide HB-19, utilisé à une dose de 5 mg/kg, induit une inhibition de la croissance des tumeurs par rapport aux souris contrôles non traitées qui présentent un volume tumoral 7 fois plus important.

De plus, alors que le volume tumoral des souris traitées au tamoxifène n'a pas significativement évolué depuis le début du traitement, les tumeurs des souris traitées par HB-19 sont indétectables après 21 jours de traitement (Figure 9A). Ainsi, alors que le tamoxifène à 10 mg/kg n'induit qu'une stabilisation du volume tumoral ou une régression partielle, le composé pentavalent HB-19 à 5 mg/kg induit quant à lui une régression tumorale apparemment totale.

Afin de contrôler ces résultats, après 40 jours de traitement, les souris sont sacrifiées, les tumeurs sont prélevées puis pesées. Alors que le poids moyen des tumeurs des souris non traitées est de 0,22 g (distribution 0,083 - 0,34 g), celles traitées au tamoxifène 10 mg/kg est de 0,06 g (distribution 0,006 - 0,22 g), et aucune tumeur n'est détectée chez les souris traitées par HB-19 (Figure 9B et C), confirmant ainsi l'estimation du volume tumoral faite par mesure extracorporelle de la taille.

Dans une autre expérience, HB19 (5 mg/kg) est administré par voie intrapéritonéale (IP) et par la voie péritumorale (SC) (Figure 10). Les résultats montrent que l'action anti-tumorale d'HB-19 est aussi efficace par voie intrapéritonéale (IP) que par voie péritumorale, démontrant ainsi non seulement l'efficacité inattendue du composé pentavalent HB19 , mais aussi sa capacité à être biodisponible in vivo au site de la tumeur, y compris en cas d'administration systémique.

Il est également à noter qu'au cours du traitement avec HB-19 aucun signe physiologique ou comportemental anormal n'a été décelé chez les souris traitées. D'autre part, l'examen anatomique des organes à la fin de l'expérience n'a révélé aucun signe visible de toxicité tissulaire, ni aucun changement dans la formule sanguine ou dans le nombre de plaquettes.

Par ailleurs, il n'a pas été possible de détecter de cellules humaines HLA-$DR^+$ (donc de cellules tumorales MDA-MB231) parmi les cellules du sang périphérique des souris xénogreffées et traitées par HB-19 (Figure 11). En effet, contrairement aux souris non traitées (PBS) où la proportion de cellules MDA-MB231 (HLA-$DR^+$) représente 22,2% des cellules du sang périphérique, cette proportion n'est respectivement que de 0,1% et 0,31% dans le cas des souris traitées par HB-19 par voie sous-cutanée ou intrapéritonéale. Ces résultats suggèrent qu'HB-19 est également capable d'empêcher la circulation sanguine des cellules tumorales, et ainsi de prévenir un événement métastasique.

### 1.4 <u>Conclusion</u>

Les résultats présentés dans l'exemple 2 montrent que le composé pentavalent HB-19 est un puissant inhibiteur de la prolifération cellulaire en associant à la fois des effets sur la croissance et l'angiogénèse tumorale. Ces observations ont été confirmées dans un modèle *in vivo* montrant qu'un traitement par injection péritumorale d'HB-19 est capable d'induire, en absence de toxicité tissulaire, une inhibition et même une régression de la tumeur dans un modèle de xénogreffe de cellules PC3 à des souris athymique.

En comparaison avec des traitements classiquement utilisés en thérapeutique cancéreuse comme le taxol, dans le modèle expérimental utilisé HB-19 apparaît beaucoup plus efficace. Cette plus grande efficacité d'HB-19 comparé au tamoxifène peut être la conséquence de l'effet inhibiteur d'HB-19 à la fois sur la croissance tumorale et également sur la formation des vaisseaux qui sont nécessaires à la prolifération tumorale. En effet que ce soit sur les cellules tumorales ou sur les cellules endothéliales activées, HB-19 cible et bloque la prolifération de ces deux types de cellules.

Il existe un grand nombre de molécules qui ont la propriété d'inhiber la prolifération des cellules tumorales. Ces molécules ont souvent une action sans véritablement avoir de ciblage cellulaire. En effet, pour de nombreux agents chimiothérapeutiques, la cible moléculaire présente dans une cellule tumorale se retrouve également dans une cellule normale ce qui explique les nombreux effets secondaires de ces traitements. Les agents biologiques ciblés ont moins d'effets secondaires car ils bloquent généralement une cible peu ou non présente dans les cellules normales. La nucléoline présente à la surface des cellules activées répond à cette propriété et constitue donc une cible thérapeutique de choix dans le traitement des cancers. Il semble également important de remarquer que nous ciblons non seulement les cellules tumorales mais également les cellules endothéliales activées. De plus, la nucléoline de surface ne semble pas limitée à un type de cancer particulier.

L'efficacité d'un double ciblage (cellules tumorales proprement dites et cellules endothéliales activées) est à comparer avec un résultat issu d'une étude réalisée par Genentech montrant dans une étude randomisée un effet très efficace dans les cancers colorectaux humain de l'association d'un antiprolifératif (5-fluorouracile) associé à un antiangiogéne (Avastin) (28). En fait, dans une étude de phase III, un traitement complémentaire d'Avastin à la chimiothérapie (irinotécan/5-fluorouracile/leucovorin) a prolongé la survie de façon très significative de cinq mois, en moyenne (20,3 mois par rapport à 15,6 mois) pour les personnes atteintes d'un cancer colorectal métastatique non traité auparavant.

Chez ces patients, il a été observé que la durée pendant laquelle la tumeur ne grossissait pas, passait de 6,2 mois à 10,6 mois par rapport aux patients qui ne recevaient que de la chimiothérapie (29).

Cette dualité d'action vis-à-vis des cellules tumorales et des cellules endothéliales fait d'HB-19 une molécule de choix dans le traitement des cancers.

***EXEMPLE 2. Activité anti-tumorale du composé trivalent Nucant 01***

**2.1 Synthèse du composé trivalent Nucant 01**

La structure chimique du composé trivalent Nucant 01 est présentée sur la Figure 2B. Ce composé possède pour support un hexapeptide cyclique constitué en alternance de résidus alanine (A) de configuration D et de résidus lysine (K) de configuration L. Trois motifs pseudopeptidiques KΨPR (avec Ψ = CH2-NH) sont liés covalamment sur le ε amino groupe de chacun des 3 résidus lysine (K).

La synthèse du composé Nucant 01 a été réalisée par couplage covalent du motif KΨPR sur une molécule "coeur " cyclique de symétrie C3. La synthèse de la molécule coeur a été décrite par S Fournel et coll (30). Le motif KΨPR protégé a été assemblé sur une résine de type chlorotrityl par la technique classique de synthèse en phase solide selon une chimie de type Fmoc puis clivé de la résine en condition acide faible. Le motif KΨPR protégé a ensuite été couplé sur la fonction epsilon NH2 de chaque résidu lysine (K) de la molécule coeur selon une stoechiométrie 1,1 KΨPR/1 molécule cyclique. Le couplage a été réalisé selon le procédé d'activation BOP/HoBt pendant 48H. A la fin de la réaction, les groupements protecteurs des motifs KΨPR ont été clivés en acide trifluoroacétique et le composé final précipité à l'éther. La molécule Nucant 01 finalement obtenue a été purifiée par HPLC et son intégrité caractérisée par spectrométrie de masse.

**2.2 Activité inhibitrice de Nucant 01 sur la prolifération de cellules tumorales in vitro**

L'effet de Nucant 01 sur la prolifération des cellules NIH-3T3 induite par l'HARP a été comparé à celui du composé pentavalent HB-19. Des cellules NIH-3T3 quiescentes ont été stimulées ou non par 4 nM d'HARP en présence d'HB19 ou de Nucant 01 à différentes concentrations (0,1 ; 0,2 ; 0,4 ; 1 ; 2 et 4 μM). Après 24 heures d'incubation, la prolifération cellulaire des cellules NIH-3T3 a été déterminée par mesure de l'incorporation de thymidine tritiée comme décrit précédemment.

Par rapport au témoin stimulé par l'HARP en absence de HB19 et Nucant 01 (prolifération cellulaire 100%), on observe que le composé Nucant 01, qui est seulement trivalent pour les motifs KPR, est également capable d'inhiber de 50% la prolifération cellulaire de cellules NIH-3T3 induite par l'HARP pour une concentration de 2μM. Ce résultat démontre donc que des composés synthétiques multivalents présentant au moins 3 motifs pseudopeptidiques de formule (I) greffé sur un peptide cyclique permettent également, comme le composé pentavalent HB-19 dont le support est un peptide linéaire, d'inhiber la prolifération de cellules tumorales induite par HARP.

La concentration nécessaire est 10 fois supérieure à celle nécessaire pour obtenir la même inhibition avec HB-19 (0,2 μM), mais le composé Nucant 01 présente que 3 motifs pseudopeptidiques de formule (I), tandis que le composé HB-19 en présente 5.

Il est donc très probable que l'augmentation à 4 ou 5 du nombre de motifs pseudopeptidiques de formule (I) greffés sur un peptide cyclique du type de celui utilisé dans Nucant 01 permette d'augmenter encore l'efficacité du composé, possiblement jusqu'à 100 fois.

**2.3 Conclusion**

Ces résultats démontrent clairement l'importance de la présentation multivalente de motifs pseudopeptidiques de formule (Ibis) pour l'activité des composés utilisés, le support utilisé pouvant varier, aussi bien un peptide linéaire que cyclique pouvant notamment être utilisés sans affecter l'efficacité des composés.

D'autres supports acceptables peuvent donc être utilisés indifféremment, du moment qu'ils permettent de greffer au moins 3, de préférence entre 3 et 8, de préférence entre 4 et 6, de préférence 5 ou 6 motifs pseudopeptidiques de formule (Ibis).

***EXEMPLE 3. Activité anti-tumorale des composés pentavalents Nucant 2 et Nucant 3***

**3.1 Synthèse des composés pentavalents Nucant 2 et Nucant 3**

Deux supports peptidiques connus pour adopter une structure hélicoïdale, sur lesquels ont été ancrés les motifs KΨPR ont été assemblés en synthèse-phase solide. Ces supports sont construits à partir de motifs répétitifs de séquence

Aib-Lys-Aib-Gly pour NUCANT 2 et Lys-Aib-Gly pour NUCANT 3 enchaînés cinq fois, où Aib représente l'acide 2-amino-isobutyrique. L'assemblage a été réalisé en chimie de type Boc. Les groupements Fmoc protecteurs de la chaîne latérale des résidus Lys ont ensuite été clivés par traitement pipéridine (3 fois 5 minutes) dans la DMF. Les cinq groupements εNH2 des lys ont ensuite servi à ancrer les motifs KΨPR (avec Ψ = $CH_2$-N). Le clivage acide final a été réalisé en acide fluorhydrique. Après précipitation des peptides à l'éther, dissolution en conditions aqueuses et lyophilisation, les analogues NUCANT 2 et NUCANT 3 ont été purifiés en HPLC analysés en spectrométrie de masse et lyophilisés.

**3.2 <u>Activité inhibitrice de Nucant 2 et Nucant 3 sur la prolifération de cellules tumorales in vitro</u>**

L'effet de Nucant 2 et Nucant 3 sur la prolifération des cellules NIH-3T3 induite par l'HARP a été comparé à celui du composé pentavalent HB-19. Des cellules NIH-3T3 quiescentes ont été stimulées ou non par 4 nM d'HARP en présence d'HB19, Nucant 2, ou Nucant 3 aux concentrations indiquées (0,1 ; 0,25 ; et 0,5 μM). Après 24 heures d'incubation, la prolifération cellulaire des cellules NIH-3T3 a été déterminée par mesure de l'incorporation de thymidine tritiée comme décrit précédemment.

Dans cette expérience, HB19 inhibe avec une IC50 à 0.1 μM. Nucant 2 et Nucant 3 inhibent d'une façon dose dépendante la prolifération cellulaire, avec une IC50 à 1,5 μM.

Les composés pentavalents Nucant 2 et Nucant 3 sont donc capables d'inhiber d'une façon dose dépendante la prolifération cellulaire, avec une concentration IC50 (concentration induisant une inhibition de 50% de la prolifération cellulaire) très proche du composé HB 19.

L'effet de Nucant 3 sur la prolifération des cellules NIH-3T3 induite par 5% de SVF a également été comparé à celui du composé HB-19. Des cellules NIH-3T3 rendues quiescentes par privation de sérum sont stimulées par 5 % de SVF en présence ou non de différentes concentrations de NUCANT 3, 6 ou 7 allant de 0,125 à 2 μM. Après 24 heures d'incubation, la prolifération cellulaire est déterminée par mesure de l'incorporation de thymidine tritiée comme décrit précédemment.

Les résultats sont présentés sur la Figure 14 en pourcentage par rapport aux cellules témoins stimulées par 5 % de SVF. Les résultats indiquent que NUCANT 3 présente un effet d'inhibition sur la prolifération cellulaire. L'analyse des courbes indique que NUCANT 3 possède une valeur d'$ID_{50}$ (Inhibitory Dose à 50%) comparable légèrement plus faible que la molécule HB-19 et est donc un peu plus efficace que HB-19. Aucun effet n'est observé pour NUCANT 3 sur des cellules non stimulées indiquant l'absence de toxicité de ces molécules.

**3.3 <u>Conclusion</u>**

Ces résultats démontrent également l'importance de la présentation multivalente de motifs pseudopeptidiques de formule (I) pour l'activité des composés utilisés dans l'invention, le support utilisé pouvant être aussi bien un peptide linéaire renfermant ou non des éléments de structuration (coude β, feuillet β), un peptide linéaire adoptant une structure hélicoïdale ou encore un composé cyclique, sans que soit affectée l'efficacité de la construction.

Différents supports acceptables peuvent donc être utilisés indifféremment, du moment qu'ils permettent de greffer au moins 3, de préférence entre 3 et 8, de préférence entre 4 et 6, de préférence 5 ou 6 motifs pseudopeptidiques de formule (I).

***EXEMPLE 4. Activité anti-tumorale des composés hexavalents Nucant 6 et Nucant 7***

**4.1 <u>Synthèse des composés hexavalents Nucant 6 et Nucant 7</u>**

Ces composés hexavalents ont été obtenus en utilisant le même procédé de synthèse que celui utilisé pour les composés pentavalents Nucant 2 et 3.

**4.2 <u>Activité inhibitrice de Nucant 6 et Nucant 7 sur la prolifération de cellules tumorales in vitro</u>**

L'effet des composés hexavalents Nucant 6 et Nucant 7 sur la prolifération des cellules NIH-3T3 induite par 5% de SVF a été comparé à celui du composé pentavalent HB-19. Des cellules NIH-3T3 rendues quiescentes par privation de sérum sont stimulées par 5 % de SVF en présence ou non de différentes concentrations de NUCANT 3, 6 ou 7 allant de 0,125 à 2 μM. Après 24 heures d'incubation, la prolifération cellulaire est déterminée par mesure de l'incorporation de thymidine tritiée comme décrit précédemment.

Les résultats sont présentés sur la Figure 14 en pourcentage par rapport aux cellules témoins stimulées par 5 % de SVF. Les résultats indiquent que NUCANT 6 et 7 présentent un effet d'inhibition sur la prolifération cellulaire. L'analyse des courbes indique que NUCANT 6 et 7 possèdent une valeur d'$ID_{50}$ (Inhibitory Dose à 50%) comparable légèrement plus faible que la molécule HB-19 et sont donc un peu plus efficace que HB-19. Aucun effet n'est observé

pour NUCANT 6 ou 7 sur des cellules non stimulées indiquant l'absence de toxicité de ces molécules.

**4.3 Conclusion**

Ces résultats démontrent encore une fois l'importance de la présentation multivalente de motifs pseudopeptidiques de formule (I) pour l'activité des composés utilisés dans l'invention, le support utilisé pouvant être aussi bien un peptide linéaire renfermant ou non des éléments de structuration (coude $\beta$, feuillet $\beta$), un peptide linéaire adoptant une structure hélicoïdale ou encore un composé cyclique, sans que soit affectée l'efficacité de la construction.

En particulier, la présentation de 6 motifs pseudopeptidiques semble très efficace pour obtenir les effets anti-prolifératifs et anti-angiogéniques désirés.

***EXEMPLE 5. Nucant 6 et Nucant 7 sont des inhibiteurs plus puissants de la nucléoline de surface que HB-19***

**5.1 Nucant 6 et Nucant 7 sont des inhibiteurs plus puissants qu'HB-19 et inhibent l'activité de la nucléoline de surface**

L'activité de la nucléoline de surface a été testée dans des cellules HeLa P4 selon la technique que nous avons décrite auparavant (13).

Les résultats, présentés dans la Figure 15, indiquent que l'inhibition de l'activité de la nucléoline de surface par Nucant 3 est comparable à celle qu'exerce HB-19. En revanche, Nucant 6 et Nucant 7 présentent une activité anti-nucléoline de surface supérieure à HB-19. En effet, HB-19 et Nucant 3 manifestent une valeur $ID_{50}$ (Inhibitory Dose à 50%) qui est entre 0,1 - 0,2 $\mu$M, tandis que Nucant 6 et Nucant 7 présentent une valeur $ID_{50}$ qui est inférieure à 0,1 $\mu$M. De plus, Nucant 6 et Nucant 7 utilisés à 0,8 $\mu$M induisent une inhibition de l'activité de la nucléoline de surface de plus que 95%.

**5.2 HB-19, Nucant 3, 6, and 7 induisent une inhibition de l'expression de la nucléoline de surface dans les cellules humaines issues d'un cancer du sein, les MDA-MB 231**

La nucléoline de surface joue un rôle important dans la prolifération et l'angiogénèse tumorale. HB-19 ainsi que les molécules apparentées Nucant 3, Nucant 6 et Nucant 7 se lient spécifiquement à la nucléoline de surface bloquant ainsi la croissance et l'angiogénèse tumorale. Après liaison de ces pseudopeptides à la nucléoline de surface, le complexe [pseudopeptide-nucleolin] est rapidement internalisé selon un processus actif. Les résultats sont présentés sur la Figure 16A et montrent que le traitement des cellules avec HB-19 (piste 1), Nucant 3 (piste 2), Nucant 6 (piste 3) ou Nucant 7 (piste 4), ont comme conséquence une diminution de la présence de la nucléoline de surface en comparaison avec les cellules non traitées. Cette diminution est observée après 24 heures de traitement mais également après 48 heures de traitement où dans ce cas la nucléoline devient indétectable.

Il est intéressant de noter que l'on observe une réduction sensiblement plus importante de la nucléoline de surface lorsque les cellules sont traitées pendant 24 heures avec les pseudopeptides Nucant 6 et Nucant 7 (pistes 3 et 4) comparés aux cellules traitées avec HB-19 et Nucant 3 (pistes 1 et 2). La même observation peut être faite après 48 heures de traitement. Il est important de noter que la réduction de la nucléoline de surface n'est pas la conséquence d'une diminution de la quantité intracellulaire de la nucléoline de surface. En effet, des quantités identiques de nucléoline sont retrouvées dans les extraits cellulaires des cellules traitées, ou non, par HB-19 ou par les différents Nucant (Figure 16B). Il est a noté que d'une façon équivalente, aucune différence n'est observée concernant les profils électrophorétiques des protéines extraites de cellules traitées, ou non, par HB-19 ou les différents Nucants. Ce résultat illustre que la synthèse de protéine n'est pas affectée par HB-19 ou les différents Nucants étudiés. D'autre part, il est intéressant de noter qu'aucun effet cytotoxique d'HB-19 ou des différents Nucants étudiés n'a été observé pouvant expliquer l'effet sur la diminution de la nucléoline de surface observé.

**5.3 Conclusions**

L'ensemble de ces résultats montre que :

a) la nucléoline est exprimée abondamment à la surface des cellules tumorales, par exemple les cellules humaines de cancer du sein (MDA-MB231).
b) le traitement par HB-19, Nucant 3, Nucant 6 et Nucant 7 induit une diminution marquée du « pool » de la nucléoline présente à la surface des cellules.
c) les pseudopeptides Nucant 6 et Nucant 7 sont plus efficaces dans l'effet de diminution du « pool » de la nucléoline présente à la surface des cellules ainsi que dans l'inhibition de l'activité de la nucléoline de surface.

***EXEMPLE 6. Effet d'HB-19 et Nucant 7 sur l'angiogenèse***

Méthodes

L'effet d'HB-19 et Nucant 7 sur l'angiogenèse a été testé dans un modèle ex vivo d'angiogénèse, la membrane chorio-allantoïdienne (CAM) de l'embryon de poulet.

20 μl d'eau contenant ou non (contrôle) HB-19 (10 μM; 0,6 μg) ou Nucant 7 (10 μM; 0,8 μg) sont déposés à la surface de la CAM. L'observation des vaisseaux est réalisée après 48 h d'incubation

Résultats

Les résultats sont présentés sur la Figure 17 et montrent qu'après 48 heures d'incubation, HB-19 et Nucant 7 induisent clairement une inhibition de l'angiogénèse. Une étude par analyse d'images qui tient compte non seulement de la longueur des capillaires mais également du nombre de ramifications indique que l'inhibitions est d'environ 50 % par rapport au témoin

***EXEMPLE* 7. *Activité anti-inflammatoire des composés HB19 et Nucant 7***

**7.1 Inhibition par HB-19 de la production de TNF-α par des PBMC humains primaires stimulés par du LPS.**

Méthodes

Des PBMC ont été isolés par centrifugation sur gradiant de densité Ficoll à partir de sang total humain EDTA-potassium, et resuspendus dans du milieu RPMI 1640 contenant 1% de sérum humain AB (Invitrogen). Des cellules à une concentration de $10^6$ cellules/0,5 ml, en absence (0) ou en présence (1 et 5 μM) de HB-19, ont été stimulées avec 100 ng/ml de LPS d'*Escherichia coli* de type 0111 :B4 et 055 :B5, et de LPS *Salmonella enterica* de serotype Re 595. Les mêmes PBMC ont été stimulés avec de la PMA :Ionomycin (Phorbol 12-myristate 13-acetate :Ionornycin) à 20 ng/ml :1 μM. Les cultures de PBMC ont été incubées à 37 °C dans un incubateur à 5% de $CO_2$ et le niveau de protéine TNF-α a été mesuré par ELISA dans des surnageants de culture récoltés après 20 heures d'incubation.

Résultats

Les résultats (voir Figure 18) montrent que les PBMC fraîchement isolés produisent du TNF-α, et que cette production constitutive de TNF-α n'est pas affectée par HB-19. En revanche, HB-19 inhibe de façon dose dépendante la production de TNF-α par les PBMC en réponse à une stimulation par différentes préparations de LPS d'Escherichia Coli ou de Salmonella Enterica. Cet effet est spécifique, puisque HB-19 n'a pas d'effet sur la production de TNF-α par les PBMC en réponse à une stimulation par la PMA-Ionomycine.

A 5 μM de HB-19, la production de TNF-α par des PBMC humains en réponse à la stimulation par différentes préparations de LPS est inhibée à un niveau comparable au niveau de base observé en absence de stimulation par le LPS.

**7.2 Inhibition par HB-19 et Nucant 7 de la production de TNF-α et d'IL-6 par des macrophages murins du péritoine primaires stimulés par du LPS**

Méthode

Pour obtenir des macrophages stimulés, des souris balb/c de 7 à 8 semaines ont été injectées en intra-péritonéal 4 jours avant l'expérience avec 1,5 ml de solution de thioglycolate (solution saline à 3%). Les macrophages ont été récoltés dans la cavité péritonéale par lavage du péritoine avec 5 ml de milieu RPMI contenant 1% de sérum de veau foetal. Les cellules ont ensuite été mises en plaques à une densité de $10^6$ cellules/0,5 ml dans du milieu RPMI 1640, incubées à 37 °C dans un incubateur à 5% de $CO^2$, et les cellules non adhérentes ont été enlevées 2 heures plus tard.

Les macrophages, en absence (-) ou en présence (+) de 4 μM de HB-19 ou 10 μM de Nucant 7 ont été soit non stimulés soit stimulés avec du LPS d'*Echerichia Coli* de sérotype 0111:B4 à 10 ng/ml, 100 ng/ml et 1000 ng/ml. Les cultures cellulaires ont été incubées à 37 °C dans un incubateur à 5% de $CO^2$ pendant 20 heures, et les niveaux de protéine TNF-α et IL-6 ont été mesurés par ELISA.

Résultats

Les résultats obtenus avec HB-19 sont représentés sur la Figure 19. A 4 μM de HB-19, la production de TNF-α et d'IL-6 par des macrophages du péritoine murin en réponse à une stimulation par du LPS est significativement inhibée. Si l'on tient compte du niveau de base observé en absence de stimulation par le LPS, le degré net d'inhibition est de 72-75 % pour le TNF-α et de 68-71 % pour l'IL-6.

Les résultats obtenus avec Nucant 7 sont représentés sur la Figure 20. A 10 μM de Nucant 7, la production de TNF-α et d'IL-6 par les macrophages du péritoine murin en réponse à une stimulation par le LPS est inhibée quasiment complètement, puisque le niveau de production de cytokine observé dans les cultures traitées par Nucant 7 en réponse à la stimulation par le LPS est comparable à celui observé en absence de LPS.

Si l'on tient compte du niveau de base observé en absence de stimulation par le LPS, le degré d'inhibition à 10 μM de Nucant 7 est de plus de 95% dans les cultures stimulées à 10, 100 et 1000 ng/ml de LPS. Le fait que le degré d'inhibition n'est pas modifié en présence d'une concentration de LPS 100 fois supérieur suggère que le mécanisme d'inhibition par Nucant 7 est principalement dû à la liaison à la nucléoline de surface. En effet, si le mécanisme d'inhibition par Nucant 7 était une conséquence de l'interaction avec le LPS, alors l'effet d'inhibition serait plus faible à 100 ng/ml de LPS qu'à 10 ng/ml de LPS.

### 7.3 Inhibition par HB-19 de la production d'IL-8 et de l'expression de ICAM-1 par des cellules HUVEC stimulées par du LPS.

Méthode

Des cellules HUVEC, à 10 000 cellules/$cm^2$, ont été mises en culture dans des plaques 96-puit dans du milieu EBM-2 contenant 2% de sérum de veau foetal. Les cellules en absence ou en présence de 5 μM de HB-19, ont été stimulées par du LPS d'*Echerichia coli* de sérotype 055 :B5 à 100 ng/ml. Les cultures cellulaires ont été incubées à 37 °C dans un incubateur à 5% de $CO_2$ pendant 20 heures et les niveaux de protéine IL-8 et ICAM-1 ont été mesurés par ELISA. Des cellules HUVEC en absence ou en présence de 5 μm de HB-19 ont été utilisées comme contrôle pour montrer les niveaux de base.

Résultats

Si l'on tient compte du niveau de base observé en absence de stimulation par le LPS, le degré d'inhibition de la production d'IL-8 et de ICAM-1 à 5 μM de HB-19 est d'environ 50%. Ces résultats montrent donc l'efficacité potentielle de HB-19 et des composés apparentés du type Nucant en tant qu'inhibiteur de la production d'IL-8 et de ICAM-1 (voir Figure 21).

### 7.4 Inhibition par HB-19 de la production de TNF-α par des PBMC humains primaires stimulés par des bactéries *Staphylococcus aureus* inactivées.

L'infection par *Staphylococcus aureus* a été montrée comme étant une des causes majeures dans la pathogenèse de l'endocardite (24, 25).

Les inventeurs ont donc mesuré les niveaux de TNF-α et d'IL-6 dans des cultures de PBMC humains primaires en réponse à des bactéries *Staphylococcies aureus* inactivées par la chaleur (HKSA, pour « heat-killed *Staphylococcus aureus* »), en absence (contrôle) ou en présence de 10 μM des composés HB-19, Nucant 3, Nucant 6, ou Nucant 7. En contrôle positif (Dex.), des PBMC ont également été traités avec de la dexaméthasone, qui est un glucocorticoïde avec une activité anti-inflammatoire et immunosuppressive connue.

Méthode

Des PBMC ont été isolés par centrifugation sur gradient de densité Ficoll à partir de sang total humain EDTA-potassium, et re-suspendus dans du milieu RPMI 1640 contenant 1% de sérum humain AB (Invitrogen). Des cellules à une concentration de $10^6$ cellules/0,5 ml, en absence (Contrôle) ou en présence (10 μM) de HB-19, Nucant 3, Nucant 6, Nucant 7, et dexamethasone (1 μg/ml)) ont été stimulées avec $10^8$ particules HKSA/ml (InvivoGen, San Diego, USA). Les cultures de PBMC ont été incubées à 37 °C dans un incubateur à 5% de $CO_2$ et le niveau de protéine TNF-α et IL-6 a été mesuré par ELISA dans des surnageants de culture récoltés après 20 heures d'incubation.

Résultats

Les résultats sont présentés sur la Figure 22 et montrent que tous les pseudopeptides testés (HB-19, Nucant 3, Nucant 6, et Nucant 7) permettent d'obtenir une inhibition significative de la production par les PBMC de TNF-α et d'IL-6 en réponse à une stimulation par des bactéries *Staphylococcus aureus* inactivées par la chaleur. Les pseudopeptides sont aussi efficaces que le médicament anti-inflammatoire classique qu'est la dexaméthasone.

Ainsi, les pseudopeptides de formule (I), tels que HB-19, Nucant 3, Nucant 6, et Nucant 7, peuvent également être utilisés en thérapie de l'inflammation cardiaque, et en particulier dans le traitement de l'endocardite d'origine infectieuse.

### 7.5 Conclusion

Ainsi, les résultats obtenus par les inventeurs montrent que les composés HB-19 et Nucant 7 sont capables d'inhiber la production de cytokines pro-inflammatoires telles que le TNF-$\alpha$, l'IL-6, ainsi que la production de la chimiokine IL-8 et de la molécule d'adhésion ICAM-1 par différents types cellulaires en réponse à une stimulation par le LPS.

En outre, ces composés permettent également d'inhiber significativement la production de cytokines pro-inflammatoires telles que le TNF-$\alpha$, l'IL-6 en réponse à une stimulation par des bactéries *Staphylococcus aureus,* un des agents responsables de nombreuses endocardites d'origine infectieuse.

Par conséquent, ces composés, ainsi que les composés apparentés de formule (I) décrits dans la présente demande, sont capables d'inhiber la production de cytokines pro-inflammatoires et de molécules impliquées dans le recrutement des leucocytes dans les foyers d'inflammation. Ces composés peuvent donc être utilisés dans des applications anti-inflammatoires, notamment dans le traitement des différentes maladies mentionnées dans la description générale.

## BIBLIOGRAPHIE

1. Hovanessian, A.G., Puvion-Dutilleul, F., Nisole, S., Svab, J., Perret, E., Deng, J. S., and Krust, B. The cell-surface-expressed nucleolin is associated with the actin cytoskeleton. (2000) Exp. Cell Res. 261, 312-328

2. Nisole, S., Krust, B., Callebaut, C., Guichard, G., Muller, S., Briand, J. P., and Hovanessian, A. G. The anti-HIV composé HB-19 forms a complex with the cell-surface-expressed nucleolin independent of heparan sulfate proteoglycans. (1999) J. Biol. Chem. 274, 27875-27884

3. Ginisty, H., Sicard, H., Roger, B., and Bouvet, P. Structure and functions of nucleolin. (1999) J. Cell Science 112, 761-772

4. Srivastava, M., and Pollard, H. B. Molecular dissection of nucleolin's role in growth and cell prolifération: new insights. (1999) FASEB J. 13, 1911-1922

5. Nisole, S., Krust, B., and Hovanessian, A. G. Anchorage of HIV on permissive cells leads to coaggregation of viral particles with surface nucleolin at membrane raft microdomains. (2002) Exp. Cell Res. 276, 155-173

6. Semenkovich, C. F., Ostlund, R. E. J., Olson, M. O., and Yang, J. W. A protein partially expressed on the surface of HepG2 cells that binds lipoproteins specifically is nucleolin. (1990) Biochemistry 29, 9708-9713

7. Callebaut, C., Jacotot, E., Krust, B., Guichar, G., Blanco, J., Svab, J., Muller, S., Briand, J. P., and Hovanessian, A. G. Composé TASP inhibitors of HIV entry bind specifically to a 95-kDa cell surface protein. (1997) J. Biol. Chem. 272, 7159-7166

8. Callebaut, C., Blanco, J., Benkirane, N., Krust, B., Jacotot, E., Guichard, G., Seddiki, N., Svab, J., Dam, E., Muller, S., Briand, J. P., and Hovanessian, A. G. Identification of V3 loop-binding proteins as potential receptors implicated in the binding ofHIV particles to CD4(+) cells. (1998) J. Biol. Chem. 273, 21988-2199

9. Christian, S., Pilch, J., Akerman, M. E., Porkka, K., Laakkonen, P., and Ruoslahti, E. Nucleolin expressed at the cell surface is a marker of endothelial cells in angiogenic blood vessels. (2003) J. Cell Biol. 163, 871-878

10. Said, A. E., Krust, B., Nisole, S., Briand, J. P., and Hovanessian, A. G. The anti-HIV cytokine midkine binds the cell surface-expressed nucleolin as a low affinity receptor. (2002) J. Biol. Chem. 277, 37492-37502

11. Said, E. A., Courty, J., Svab, J., Delbé, J., Krust, B., and Hovanessian, A. G. Pleiotrophin inhibits HIV infection by binding the cell surface-expressed nucleolin. (2005) FEBS J. 272,4646-4659

12. Legrand, D., Vigie, K., Said, E. A., Elass, E., Masson, M., Slomianny, M. C., Carpentier, M., Briand, J. P., Mazurier, J., and Hovanessian, A. G. Surface nucleolin participates in both the binding and endocytosis of lactoferrin in target cells. (2004) Eur. J. Biochem. 271, 303-317

13. Nisole, S., Krust, B., Dam, E., Blanco, A., Seddiki, N., Loaec, S., Callebaut, C., Guichard, G., Muller, S., Briand, J. P., and Hovanessian, A. G. The HB-19 composé 5[Kpsi(CH2N)PR]-TASP inhibits attachment of T lymophocyte- and macrophage-tropic HIV to permissive cells. (2000) AIDS Res. Hum. Retroviruses 16, 237-249

14. Nisole, S., Said, E. A., Mische, C., Prevost, M. C., Krust, B., Bouvet, P., Bianco, A., Briand, J. P., and Hovanessian, A. G. The anti-HIV pentameric composé HB-19 binds the C-terminal end of nucleolin and prevents anchorage of virus particles in the plasma membrane of target cells. (2002) J. Biol. Chem. 277, 20877-20886

15. Bates, P. J., Kahlon, J. B., Thomas, S. D., Trent, J. O., and Miller, D. M. Antiproliferative activity of G-rich

oligonucleotides correlates with protein binding. (1999) J. Biol. Chem. 274, 26369-26377
16. Huang Y, Shi H, Zhou H, Song X, Yuan S, Luo Y. The angiogenesis function of nucleolin is mediated by vascular endothelial growth factor and nonmuscle myosin. (2006) Blood 107, 3564-3571.
17. Seko Y, Cole S, Kasprzak W, Shapiro BA, Ragheb JA. The role of cytokine mRNA stability in the pathogenesis of autoimmune disease. Autoimmun Rev 2006;5:299-305.
18. Rot A. Neutrophil attractant/activation protein-1 (intrleukin-8) induces in vitro neutrophil migration by haptotactic mechanism. Eur J Immunol 1993;23:303-06.
19. Elass E, Masson M, Mazurier J, Legrand D. Lactoferrin inhibits the lipposaccharaide-induced expression and proteoglycan-binding ability of IL-8 in human entothelial cells. Infect Immun 2002;70:1860-66.
20. Kevil CG, Patel RP, Bullard DC. Essential role of ICAM-1 in mediating monocyte adhesion to aortic endothelial cells. Am J Physiol Cell Physiol 2001;281:C1442-47.
21. Bucklin SE, Silverstein R, Morrison DC. An interleukin-6-induced acute-phase response does not confer protection against lipopolysaccharide lethality. Infect Immun 1993;61:3184-89.
22. Karima R, Matsumoto S, Higashi H, Matsushima K. The molecular pathogenesis of endotoxic shock and organ failure. Mol Med Today 1999;5:123-32.
23. Zanotti S, Kulmar A, Kumar A. Cytokine modulation in sepsis and septic shock. Expert Opin Investig Drugs 2002;11:1061-75.
24. Alter, P, Hoeschen, J., Ritter, M., Maisch, B. Usefulness of cytokines interleukin-6 and interleukin-2R concentrations in diagnosing active infective endocarditis involving native valves. (2002) Am. J. Cardiol. 89, 1400-1404
25. Shun, C.T., Lu, S.Y., Yeh, C.Y., Chaing, C.P., Chia, J.S., Chen, J.Y. Glucosyltransferases of viridans streptococci are modulins of interleukin-6 induction in infective endocarditis. (2005) Infec. Immun. 73, 3261-3270.
26. Kannan K, Ortmann RA, Kimpel D. Animal models of rheumatoid arthritis and their relevance to human disease. Pathophysiology 2005;12:167-81.
27. Peifer C, Wagner G, Laufer S. New approaches to the treatment of inflammatory disorders small molecule inhibitors of p38 MAP kinase. Curr Top Med Chem 2006;6(2):113-49 2006;6:113-49.
28. Kabbinavar, F., Hurwitz, H. I., Fehrenbacher, L., Meropol, N. J., Novotny, W. F., Lieberman, G., Griffing, S., and Bergsland, E. Phase II, randomized trial comparing bevacizumab plus fluorouracil (FU)/leucovorin (LV) with FU/LV alone in patients with metastatic colorectal cancer. (2003) J Clin Oncol 21, 60-65.
29. Hurwitz, H. I. New agents in colon cancer. (2003) Clin Adv Hematol Oncol 1, 404-405
30. Fournel, S. et al. C3-symmetric peptide scaffolds are functional mimetics of trimeric CD40L. (2005) Nat Chem Biol 1, 377-382.
31. WO 2005/035579.
32. US 2005/0026860.
33. Krust B. et al. The anti-HIV pentameric pseudopeptide HB-19 is preferentially taken up in vivo by lymphoid organs where it forms a complex with nucleolin. (2001). PNAS. 98(24) : 14090-14095.
34. WO95/29190.
35. US2004/186056.

LISTE DE SEQUENCE

<110> Centre National de la Recherche Scientifique (CNRS)
COURTY José
HOVANESSIAN Ara
BRIAND Jean Paul
GUICHARD Gilles
HAMA Yamina

<120> Utilisation de ligands synthétiques multivalents de la nucléoline de surface pour le traitement du cancer ou de l'inflammation

<130> 351183-D24262

<150> FR0603813
<151> 2006-04-27

<160> 21

<170> PatentIn version 3.3

<210> 1
<211> 10
<212> PRT
<213> Séquence artificielle

<220>
<223> Peptide synthétique

<400> 1

Lys Lys Lys Gly Pro Lys Glu Lys Gly Cys
1 5 10

<210> 2
<211> 6
<212> PRT
<213> Séquence artificielle

<220>
<223> Peptide synthétique

<400> 2

Lys Lys Lys Lys Gly Cys
1 5

<210> 3
<211> 12
<212> PRT
<213> Séquence artificielle

<220>
<223> Peptide synthétique

<400> 3

Lys Lys Lys Lys Gly Pro Lys Lys Lys Lys Gly Ala

1 5 10

<210> 4
<211> 9
<212> PRT
<213> Séquence artificielle

<220>
<223> Peptide synthétique

<220>

<221> MOD_RES
<222> (9)
<223> Xaa représente le (2S)-2-aminohexanamide

<400> 4

Lys Lys Lys Gly Pro Lys Glu Lys Xaa
1 5

<210> 5
<211> 11
<212> PRT
<213> Séquence artificielle

<220>
<223> Peptide synthétique

<220>
<221> BINDING
<222> (1)
<223> Lysinyl proline

<220>
<221> BINDING
<222> (3)
<223> N6-Arg-Pro-Lysinyl

<220>
<221> BINDING
<222> (5)
<223> N6-Arg-Pro-Lysinyl

<220>
<221> BINDING
<222> (8)
<223> N6-Arg-Pro-Lysinyl

<220>
<221> BINDING
<222> (10)
<223> N6-Arg-Pro-Lysinyl

<220>
<221> MOD_RES
<222> (11)
<223> Xaa représente le (2S)-2-aminohexanamide

<400> 5

Pro Arg Lys Lys Lys Gly Pro Lys Glu Lys Xaa
1 5 10

<210> 6
<211> 4
<212> PRT
<213> Séquence artificielle

<220>
<223> Peptide synthétique

<400> 6

Aib Lys Aib Gly
1

<210> 7
<211> 3
<212> PRT
<213> Séquence artificielle

<220>
<223> Peptide synthétique

<400> 7

Lys Aib Gly
1

<210> 8
<211> 20
<212> PRT
<213> Séquence artificielle

<220>
<223> Peptide synthétique

<400> 8

```
Aib Lys Aib Gly Aib Lys Aib Gly Aib Lys Aib Gly Aib Lys Aib Gly Aib Lys Aib Gly
1               5                   10                  15                  20
```

<210> 9
<211> 15
<212> PRT
<213> Séquence artificielle

<220>
<223> Peptide synthétique

<400> 9

```
Lys Aib Gly Lys Aib Gly Lys Aib Gly Lys Aib Gly Lys Aib Gly
1               5                   10                  15
```

<210> 10
<211> 20
<212> PRT
<213> Séquence artificielle

<220>

<223> Peptide synthétique

<220>
<221> BINDING
<222> (2)
<223> N6-Arg-Pro-Lysinyl

<220>
<221> BINDING
<222> (6)
<223> N6-Arg-Pro-Lysinyl

<220>
<221> BINDING
<222> (10)
<223> N6-Arg-Pro-Lysinyl

<220>
<221> BINDING
<222> (14)
<223> N6-Arg-Pro-Lysinyl

<220>
<221> BINDING
<222> (18)
<223> N6-Arg-Pro-Lysinyl

<400> 10

```
Aib Lys Aib Gly Aib Lys Aib Gly Aib Lys Aib Gly Aib Lys Aib Gly Aib Lys Aib Gly
1               5                   10                  15                  20
```

<210> 11
<211> 15
<212> PRT
<213> Séquence artificielle

<220>
<223> Peptide synthétique

<220>
<221> BINDING
<222> (1)
<223> N6-Arg-Pro-Lysinyl

<220>
<221> BINDING
<222> (4)
<223> N6-Arg-Pro-Lysinyl

<220>
<221> BINDING
<222> (7)
<223> N6-Arg-Pro-Lysinyl

<220>
<221> BINDING

<222> (10)
<223> N6-Arg-Pro-Lysinyl

<220>
<221> BINDING
<222> (13)
<223> N6-Arg-Pro-Lysinyl

<400> 11

Lys Aib Gly Lys Aib Gly Lys Aib Gly Lys Aib Gly Lys Aib Gly
1 5 10 15

<210> 12
<211> 5
<212> PRT
<213> Séquence artificielle

<220>
<223> Peptide synthétique

<400> 12

Lys Ala Lys Pro Gly
1 5

<210> 13
<211> 15
<212> PRT
<213> Séquence artificielle

<220>
<223> Peptide synthétique

<220>
<221> MOD_RES
<222> (1)
<223> Acétyl lysine

<220>
<221> MOD_RES
<222> (15)
<223> Amide glycine

<400> 13

Lys Ala Lys Pro Gly Lys Ala Lys Pro Gly Lys Ala Lys Pro Gly
1 5 10 15

<210> 14
<211> 24
<212> PRT
<213> Séquence artificielle

<220>

<223> Peptide synthétique

<220>
<221> MOD_RES
<222> (1)
<223> Acétyl lysine

<220>
<221> MOD_RES
<222> (24)
<223> Amide glycine

<400> 14

```
Aib Lys Aib Gly Aib Lys Aib Gly Aib Lys Aib Gly Aib Lys Aib Gly
1               5                   10                  15
Aib Lys Aib Gly Aib Lys Aib Gly
            20
```

<210> 15
<211> 18
<212> PRT
<213> Séquence artificielle

<220>
<223> Peptide synthétique

<220>
<221> MOD_RES
<222> (1)
<223> Acétyl lysine

<220>
<221> MOD_RES
<222> (18)
<223> Amide glycine

<400> 15

```
Lys Aib Gly Lys Aib Gly Lys Aib Gly Lys Aib Gly Lys Aib Gly Lys
1               5                   10                  15
Aib Gly
```

<210> 16
<211> 18
<212> PRT
<213> Séquence artificielle

<220>
<223> Peptide synthétique

<220>
<221> MOD_RES
<222> (1)
<223> Acétyl lysine

<220>
<221> BINDING
<222> (1)
<223> N6-Arg-Pro-Lysinyl

<220>
<221> BINDING
<222> (4)
<223> LYS-Ψ-PRO-ARG-LYS. Ψ représente la liaison ($-CH_2NH-$)

<220>
<221> BINDING
<222> (7)
<223> N6-Arg-Pro-Lysinyl

<220>
<221> BINDING
<222> (10)
<223> N6-Arg-Pro-Lysinyl

<220>
<221> BINDING
<222> (13)
<223> N6-Arg-Pro-Lysinyl

<220>
<221> BINDING
<222> (16)
<223> N6-Arg-Pro-Lysinyl

<220>
<221> MOD_RES
<222> (18)
<223> Amide glycine

<400> 16

```
Lys Aib Gly Lys Aib Gly Lys Aib Gly Lys Aib Gly Lys Aib Gly Lys
1               5                   10                  15
Aib Gly
```

<210> 17
<211> 15
<212> PRT
<213> Séquence artificielle

<220>
<223> Peptide synthétique

<220>
<221> MOD_RES
<222> (1)
<223> Acétyl lysine

<220>
<221> BINDING
<222> (1)

<223> N6-Arg-Pro-Lysinyl

<220>
<221> BINDING
<222> (3)
<223> N6-Arg-Pro-Lysinyl

<220>
<221> BINDING
<222> (6)
<223> N6-Arg-Pro-Lysinyl

<220>
<221> BINDING
<222> (8)
<223> N6-Arg-Pro-Lysinyl

<220>
<221> BINDING
<222> (11)
<223> N6-Arg-Pro-Lysinyl

<220>
<221> BINDING
<222> (13)
<223> N6-Arg-Pro-Lysinyl

<220>
<221> MOD_RES
<222> (15)
<223> Amide glycine

<400> 17

```
Lys Ala Lys Pro Gly Lys Ala Lys Pro Gly Lys Ala Lys Pro Gly
1               5                   10                  15
```

<210> 18
<211> 20
<212> PRT
<213> Séquence artificielle

<220>
<223> Peptide synthétique

<220>
<221> MOD_RES
<222> (1)
<223> Acétyl lysine

<220>
<221> MOD_RES
<222> (20)
<223> Amide glycine

<400> 18

Aib Lys Aib Gly Aib Lys Aib Gly Aib Lys Aib Gly Aib Lys Aib Gly Aib Lys Aib Gly
1 5 10 15 20

<210> 19
<211> 15
<212> PRT
<213> Séquence artificielle

<220>
<223> Peptide synthétique

<220>
<221> MOD_RES
<222> (1)
<223> Acétyl lysine

<220>
<221> MOD_RES
<222> (15)
<223> Amide glycine

<400> 19

Lys Aib Gly Lys Aib Gly Lys Aib Gly Lys Aib Gly Lys Aib Gly
1 5 10 15

<210> 20
<211> 20
<212> PRT
<213> Séquence artificielle

<220>
<223> Peptide synthétique

<220>
<221> MOD_RES
<222> (1)
<223> Acétyl lysine

<220>
<221> BINDING
<222> (2)
<223> N6-Arg-Pro-Lysinyl

<220>
<221> BINDING
<222> (6)
<223> N6-Arg-Pro-Lysinyl

<220>
<221> BINDING
<222> (10)
<223> N6-Arg-Pro-Lysinyl

<220>
<221> BINDING

<222> (14)
<223> N6-Arg-Pro-Lysinyl

<220>
<221> BINDING
<222> (18)
<223> N6-Arg-Pro-Lysinyl

<220>
<221> MOD_RES
<222> (20)
<223> Amide glycine

<400> 20

```
Aib Lys Aib Gly Aib Lys Aib Gly Aib Lys Aib Gly Aib Lys Aib Gly Aib Lys Aib Gly
1               5                   10                  15                  20
```

<210> 21
<211> 15
<212> PRT
<213> Séquence artificielle

<220>
<223> Peptide synthétique

<220>
<221> MOD_RES
<222> (1)
<223> Acétyl lysine

<220>
<221> BINDING
<222> (1)
<223> N6-Arg-Pro-Lysinyl

<220>
<221> BINDING
<222> (4)
<223> N6-Arg-Pro-Lysinyl

<220>
<221> BINDING
<222> (7)
<223> N6-Arg-Pro-Lysinyl

<220>
<221> BINDING
<222> (10)
<223> N6-Arg-Pro-Lysinyl

<220>
<221> BINDING
<222> (13)
<223> N6-Arg-Pro-Lysinyl

<220>

<221> MOD_RES
<222> (15)
<223> Amide glycine

<400> 21

```
Lys Aib Gly Lys Aib Gly Lys Aib Gly Lys Aib Gly Lys Aib Gly
1               5                   10                  15
```

## Revendications

1. Utilisation d'un composé synthétique multivalent comprenant ou constitué d'un support sur lequel sont greffés 3 à 8 motifs pseudopeptidiques, ledit composé étant de formule :

$$[Y_1 \overset{\Psi}{-} P - Y_2 - (X)_m]_k - \text{Support}$$

,

où
X représente un acide aminé quelconque ;
$Y_1$ et $Y_2$ sont indépendamment choisis parmi l'arginine (R) et la lysine (K) ;
P est une proline ;
m vaut 0 ou 1 ;
k est un entier compris entre 3 et 8 ;
$\Psi$ représente une liaison peptidique modifiée, significativement plus résistante à au moins une protéase qu'une liaison peptidique normale, et
le support est un peptide linéaire comprenant au moins k lysines,
pour la fabrication d'un médicament destiné au traitement d'une maladie impliquant une dérégulation de la prolifération cellulaire et/ou de l'angiogénèse.

2. Utilisation selon la revendication 1, **caractérisée en ce que** ledit support est un peptide linéaire choisi parmi :

    • SEQ ID NO :1,
    • SEQ ID NO :2,
    • SEQ ID NO :3,
    • SEQ ID NO :4,
    • une séquence linéaire constituée de 2 à 4 motifs KAKPG (SEQ ID NO :12), et
    • un peptide linéaire de structure hélicoïdale constitué de 3 à 8 répétitions de motifs peptidiques de séquence Aib-Lys-Aib-Gly (SEQ ID NO :6) ou Lys-Aib-Gly (SEQ ID NO :7) respectivement.

3. Utilisation selon la revendication 2, **caractérisée en ce que** ledit support est un peptide linéaire choisi parmi les peptides linéaires de séquence SEQ ID NO :1, SEQ ID NO :2, SEQ ID NO :3, SEQ ID NO :4, SEQ ID NO :8, SEQ ID NO :9, SEQ ID NO :13, SEQ ID NO :14, SEQ ID NO :15, SEQ ID NO :18, ou SEQ ID NO :19.

4. Utilisation selon l'une quelconque des revendications 1 à 3, **caractérisée en ce que** lesdits motifs pseudopeptidiques sont greffés directement sur ledit support.

5. Utilisation selon l'une quelconque des revendications 1 à 4, **caractérisée en ce que** k est compris entre 5 et 6.

6. Utilisation selon l'une quelconque des revendications 1 à 5, **caractérisée en ce que** $Y_1$ est une lysine (K) et $Y_2$ est une arginine (R).

7. Utilisation selon l'une quelconque des revendications 1 à 6, **caractérisée en ce que** m est égal à 0.

**8.** Utilisation selon l'une quelconque des revendications 1 à 7, **caractérisée en ce que** Ψ représente une liaison réduite ($-CH_2NH-$) , une liaison rétro inverso (-NHCO-), une liaison méthylène oxy ($-CH_2-O-$) , une liaison thiométhylène ($-CH_2-S-$), une liaison carba ($-CH_2CH_2-$), une liaison cétométhylène ($-CO-CH_2-$), une liaison hydroxyéthylène ($-CHOH-CH_2-$), une liaison (-N-N-), une liaison E-alcène ou une liaison (-CH=CH-).

**9.** Utilisation selon la revendication 1, **caractérisée en ce que** le composé est choisi parmi les composés dont la structure est décrite sur la Figure 2A (SEQ ID NO :5), la Figure 2C (SEQ ID NO :20), la Figure 2D (SEQ ID NO :21), la Figure 2E (SEQ ID NO :16), ou la Figure 2F (SEQ ID NO :17).

**10.** Utilisation selon l'une quelconque des revendications 1 à 9, **caractérisée en ce que** ladite maladie impliquant une dérégulation de la prolifération cellulaire et/ou de l'angiogénèse est un cancer.

**11.** Utilisation selon l'une quelconque des revendications 1 à 9, **caractérisée en ce que** ladite maladie impliquant une dérégulation de la prolifération cellulaire et/ou de l'angiogénèse est :

- une maladie non cancéreuse de la peau choisie parmi les kystes épidermiques et dermiques, le psoriasis, et les angiomes, ou
- une maladie oculaire choisie parmi la dégénérescence maculaire liée à l'age (DMLA), la rétinopathie diabétique, et le glaucome néovasculaire.

**12.** Composé synthétique multivalent comprenant ou constitué d'un support sur lequel sont greffés 3 à 8 motifs pseudopeptidiques, ledit composé étant de formule :

$$[Y_1 \overset{\Psi}{-} P - Y_2 - (X)_m]_k - \text{Support}$$

,

où
X représente un acide aminé quelconque ;
$Y_1$ et $Y_2$ sont indépendamment choisis parmi l'arginine (R) et la lysine (K) ;
P est une proline ;
m vaut 0 ou 1 ;
k est un entier compris entre 3 et 8 ;
Ψ représente une liaison peptidique modifiée, significativement plus résistante à au moins une protéase qu'une liaison peptidique normale,
et le support est un peptide linéaire comprenant au moins k lysines,
à l'exception des composés dont le support est un peptide non cyclique comprenant une séquence d'acides aminés choisie parmi KPG, KGP, KGC, ou $KX_1KX_4KX_1K$, où $X_1$ est facultatif et choisi parmi la lysine (K), la valine (V), l'alanine (A), l'acide glutamique (E), et l'isoleucine (I), et $X_4$ est facultatif et choisi parmi la valine (V), l'alanine (A), l'acide glutamique (E), et l'isoleucine (I).

**13.** Composé selon la revendication 12, **caractérisé en ce que** le support est un peptide linéaire constitué de 3 à 8 répétitions de motifs peptidiques de séquence Aib-K-Aib-G (SEQ ID NO :6) ou K-Aib-G (SEQ ID NO :7) respectivement.

**14.** Composé selon la revendication 13, **caractérisé en ce que** le support est constitué d'une séquence d'acides aminés choisie parmi SEQ ID NO : 8, SEQ ID NO : 9, SEQ ID NO : 14, SEQ ID NO : 15, SEQ ID NO :18, et SEQ ID NO :19.

**15.** Composé selon l'une quelconque des revendications 12 à 14, **caractérisé en ce que** Ψ représente une liaison réduite ($-CH_2NH-$), une liaison retro inverso (-NHCO-), une liaison méthylène oxy ($-CH_2-O-$), une liaison thiométhylène ($-CH_2-S-$), une liaison carba ($-CH_2CH_2-$), une liaison cétométhylène ($-CO-CH_2-$), une liaison hydroxyéthylène ($-CHOH-CH_2-$), une liaison (-N-N-), une liaison E-alcène ou une liaison (-CH=CH-).

**16.** Composé selon l'une quelconque des revendications 12 à 15, **caractérisé en ce que** m est égal à 0.

**17.** Composé selon la revendication 12, **caractérisé en ce qu'**il est choisi parmi les composés dont la structure est

décrite sur la Figure 2C (SEQ ID NO :20), la Figure 2D (SEQ ID NO :21), la Figure 2E (SEQ ID NO :16).

**18.** Composé dont la structure est décrite sur la Figure 2F (SEQ ID NO :17).

**19.** Composé selon l'une quelconque des revendications 12 à 18, pour son utilisation en tant que médicament.

**20.** Composition pharmaceutique comprenant un composé selon l'une quelconque des revendications 12 à 18.

## Patentansprüche

**1.** Verwendung einer multivalenten synthetischen Verbindung, welche einen Träger, auf welchen 3 bis 8 pseudopeptidische Motive aufgepfropft sind, umfasst oder daraus gebildet wird, wobei die Verbindung die Formel aufweist:

$$[Y_1 \overset{\Psi}{-} P - Y_2 - (X)_m]_k - \textbf{Träger} ,$$

worin
X für eine beliebige Aminosäure steht;
$Y_1$ und $Y_2$ unabhängig voneinander unter Arginin (R) und Lysin (K) ausgewählt sind;
P ein Prolin ist;
m einen Wert von 0 oder 1 aufweist;
k eine ganze Zahl zwischen 3 und 8 eingeschlossen ist;
$\Psi$ für eine modifizierte Peptidbindung, die signifikant widerstandsfähiger gegenüber mindestens einer Protease als eine normale Peptidbindung ist, steht und
der Träger ein lineares Peptid, das mindestens k Lysine umfasst, ist,
für die Herstellung eines Arzneimittels, das für die Behandlung einer Krankheit, an welcher eine Deregulation der Zellproliferation und/oder der Angiogenese beteiligt ist, bestimmt ist.

**2.** Verwendung nach Anspruch 1, **dadurch gekennzeichnet, dass** der Träger ein lineares Peptid ist, das ausgewählt ist unter:

- SEQ ID NO:1,
- SEQ ID NO:2,
- SEQ ID NO:3,
- SEQ ID NO:4,
- einer linearen Sequenz, die aus 2 bis 4 Motiven KAKPG (SEQ ID NO:12) gebildet wird, und
- einem linearen Peptid von helikoidaler Struktur, das aus 3 bis 8 Wiederholungen von peptidischen Motiven der Sequenz Aib-Lys-Aib-Gly (SEQ ID NO:6) bzw. Lys-Aib-Gly (SEQ ID NO:7) gebildet wird.

**3.** Verwendung nach Anspruch 2, **dadurch gekennzeichnet, dass** der Träger ein lineares Peptid ist, das unter den linearen Peptiden der Sequenz SEQ ID NO:1, SEQ ID NO:2, SEQ ID NO:3, SEQ ID NO:4, SEQ ID NO:8, SEQ ID NO:9, SEQ ID NO:13, SEQ ID NO:14, SEQ ID NO:15, SEQ ID NO:18 oder SEQ ID NO:19 ausgewählt ist.

**4.** Verwendung nach einem der Ansprüche 1 bis 3, **dadurch gekennzeichnet, dass** die pseudopeptidischen Motive direkt auf den Träger aufgepfropft sind.

**5.** Verwendung nach einem der Ansprüche 1 bis 4, **dadurch gekennzeichnet, dass** k zwischen 5 und 6 liegt.

**6.** Verwendung nach einem der Ansprüche 1 bis 5, **dadurch gekennzeichnet, dass** $Y_1$ ein Lysin (K) ist und $Y_2$ ein Arginin (R) ist.

**7.** Verwendung nach einem der Ansprüche 1 bis 6, **dadurch gekennzeichnet, dass** m gleich 0 ist.

**8.** Verwendung nach einem der Ansprüche 1 bis 7, **dadurch gekennzeichnet, dass** $\Psi$ für eine reduzierte Bindung ($-CH_2NH-$), eine retro-inverso-Bindung (-NHCO-), eine Oxymethylen-Bindung ($-CH_2-O-$), eine Thiomethylen-Bin-

dung ($-CH_2-S-$), eine Carba-Bindung ($-CH_2CH_2-$), eine Ketomethylen-Bindung ($-CO-CH_2-$), eine Hydroxyethylen-Bindung ($-CHOH-CH_2-$), eine Bindung (-N-N-), eine E-Alken-Bindung oder eine Bindung (-CH=CH-) steht.

9. Verwendung nach Anspruch 1, **dadurch gekennzeichnet, dass** die Verbindung ausgewählt ist unter den Verbindungen, deren Struktur in der Figur 2A (SEQ ID NO:5), der Figur 2C (SEQ ID NO:20), der Figur 2D (SEQ ID NO:21), der Figur 2E (SEQ ID NO:16) oder der Figur 2F (SEQ ID NO:17) beschrieben ist.

10. Verwendung nach einem der Ansprüche 1 bis 9, **dadurch gekennzeichnet, dass** die Krankheit, an welcher eine Deregulation der Zellproliferation und/oder der Angiogenese beteiligt ist, eine Krebserkrankung ist.

11. Verwendung nach einem der Ansprüche 1 bis 9, **dadurch gekennzeichnet, dass** die Krankheit, an welcher eine Deregulation der Zellproliferation und/oder der Angiogenese beteiligt ist,:

- eine nicht krebsartige Krankheit der Haut, ausgewählt unter den Epidermal- und Dermalzysten, Psoriasis und den Angiomen, oder
- eine Augenkrankheit, ausgewählt unter altersbedingter Makuladegeneration (AMD), diabetischer Retinopathie und neovaskulärem Glaukom,

ist.

12. Multivalente synthetische Verbindung, welche einen Träger, auf welchen 3 bis 8 pseudopeptidische Motive aufgepfropft sind, umfasst oder daraus gebildet wird, wobei die Verbindung die Formel aufweist:

$$[Y_1 \overset{\Psi}{-} P - Y_2 - (X)_m]_k - \text{Träger} \quad ,$$

worin
X für eine beliebige Aminosäure steht;
$Y_1$ und $Y_2$ unabhängig voneinander unter Arginin (R) und Lysin (K) ausgewählt sind;
P ein Prolin ist;
m einen Wert von 0 oder 1 aufweist;
k eine ganze Zahl zwischen 3 und 8 eingeschlossen ist;
$\Psi$ für eine modifizierte Peptidbindung, die signifikant widerstandsfähiger gegenüber mindestens einer Protease als eine normale Peptidbindung ist, steht und der Träger ein lineares Peptid, das mindestens k Lysine umfasst, ist, mit Ausnahme der Verbindungen, bei denen der Träger ein nicht-cyclisches Peptid, welches eine Aminosäuresequenz, ausgewählt unter KPG, KGP, KGC oder $KX_1KX_4KX_1K$, worin $X_1$ fakultativ ist und unter Lysin (K), Valin (V), Alanin (A), Glutaminsäure (E) und Isoleucin (I) ausgewählt ist und $X_4$ fakultativ ist und unter Valin (V), Alanin (A), Glutaminsäure (E) und Isoleucin (I) ausgewählt ist, umfasst, ist.

13. Verbindung nach Anspruch 12, **dadurch gekennzeichnet, dass** der Träger ein lineares Peptid ist, das aus 3 bis 8 Wiederholungen von peptidischen Motiven der Sequenz Aib-K-Aib-G (SEQ ID NO:6) bzw. K-Aib-G (SEQ ID NO:7) gebildet wird.

14. Verbindung nach Anspruch 13, **dadurch gekennzeichnet, dass** der Träger aus einer Aminosäuresequenz, ausgewählt unter SEQ ID NO:8, SEQ ID NO:9, SEQ ID NO:14, SEQ ID NO:15, SEQ ID NO:18 und SEQ ID NO:19, gebildet wird.

15. Verbindung nach einem der Ansprüche 12 bis 14, **dadurch gekennzeichnet, dass** $\Psi$ für eine reduzierte Bindung ($-CH_2NH-$), eine retro-inverso-Bindung (-NHCO-), eine Oxymethylen-Bindung ($-CH_2-O-$), eine Thiomethylen-Bindung ($-CH_2-S-$), eine Carba-Bindung ($-CH_2CH_2-$), eine Ketomethylen-Bindung ($-CO-CH_2-$), eine Hydroxyethylen-Bindung ($-CHOH-CH_2-$), eine Bindung (-N-N-), eine E-Alken-Bindung oder eine Bindung (-CH=CH-) steht.

16. Verbindung nach einem der Ansprüche 12 bis 15, **dadurch gekennzeichnet, dass** m gleich 0 ist.

17. Verbindung nach Anspruch 12, **dadurch gekennzeichnet, dass** sie unter den Verbindungen, deren Struktur in Figur 2C (SEQ ID NO:20), Figur 2D (SEQ ID NO:21), Figur 2E (SEQ ID NO:16) beschrieben ist, ausgewählt ist.

**18.** Verbindung, deren Struktur in Figur 2F (SEQ ID NO:17) beschrieben ist.

**19.** Verbindung nach einem der Ansprüche 12 bis 18 zu deren Verwendung als Arzneimittel.

**20.** Pharmazeutische Zusammensetzung, welche eine Verbindung nach einem der Ansprüche 12 bis 18 umfasst.

**Claims**

**1.** Use of a multivalent synthetic compound comprising or consisting of a support on which 3 to 8 pseudopeptide units are grafted, said compound being of formula:

$$[Y_1 \overset{\Psi}{-} P - Y_2 - (X)_m]_k - \text{Support},$$

where
X represents any amino acid;
$Y_1$ and $Y_2$ are selected independently from arginine (R) and lysine (K);
P is proline;
m is 0 or 1;
k is an integer between 3 and 8;
$\Psi$ represents a modified peptide bond significantly more resistant to at least one protease than a standard peptide bond, and
the support is a linear peptide comprising at least k lysines,
for the manufacture of a medicament intended for the treatment of a disorder involving deregulation of cell proliferation and/or angiogenesis.

**2.** Use according to claim 1, **characterized in that** said support is a linear peptide selected from:

- SEQ ID NO:1,
- SEQ ID NO:2,
- SEQ ID NO:3,
- SEQ ID NO:4,
- a linear sequence consisting of 2 to 4 KAKPG units (SEQ ID NO:12), and
- a linear peptide of helicoidal structure consisting of 3 to 8 repetitions of peptide units of sequence Aib-Lys-Aib-Gly (SEQ ID NO:6) or Lys-Aib-Gly (SEQ ID NO:7), respectively.

**3.** Use according to claim 2, **characterized in that** said support is a linear peptide selected from linear peptides of sequence SEQ ID NO:1, SEQ ID NO:2, SEQ ID NO:3, SEQ ID NO:4, SEQ ID NO:8, SEQ ID NO:9, SEQ ID NO:13, SEQ ID NO:14, SEQ ID NO:15, SEQ ID NO:18, or SEQ ID NO:19.

**4.** Use according to any one of claims 1 to 3, **characterized in that** said pseudopeptide units are grafted directly on said support.

**5.** Use according to any one of claims 1 to 4, **characterized in that** k is between 5 and 6.

**6.** Use according to any one of claims 1 to 5, **characterized in that** $Y_1$ is lysine (K) and $Y_2$ is arginine (R).

**7.** Use according to any one of claims 1 to 6, **characterized in that** m is equal to 0.

**8.** Use according to any one of claims 1 to 7, **characterized in that** $\Psi$ represents a reduced bond ($-CH_2NH-$), a retro-inverso bond (-NHCO-), a methylene-oxy bond ($-CH_2-O-$), a thiomethylene bond ($-CH_2-S-$), a carba bond ($-CH_2CH_2-$), a ketomethylene bond ($-CO-CH_2-$), a hydroxyethylene bond ($-CHOH-CH_2-$), a (-N-N-) bond, an E-alkene bond or a (-CH=CH-) bond.

**9.** Use according to claim 1, **characterized in that** the compound is selected from compounds whose structure is described in Figure 2A (SEQ ID NO:5), Figure 2C (SEQ ID NO:20), Figure 2D (SEQ ID NO:21), Figure 2E (SEQ ID NO:16) or Figure 2F (SEQ ID NO:17).

**10.** Use according to any one of claims 1 to 9, **characterized in that** said disease involving deregulation of cell proliferation and/or angiogenesis is cancer.

**11.** Use according to any one of claims 1 to 9, **characterized in that** said disease involving deregulation of cell proliferation and/or angiogenesis is:

- a non-cancerous disease of the skin selected from epidermal or dermal cysts, psoriasis and angiomas, or
- an ocular disease selected from age-related macular degeneration (ARMD), diabetic retinopathy and neovascular glaucoma.

**12.** Multivalent synthetic compound comprising or consisting of a support on which 3 to 8 pseudopeptide units are grafted, said compound being of formula:

$$[Y_1 \overset{\Psi}{-} P - Y_2 - (X)_m]_k - \text{ Support },$$

where
X represents any amino acid;
$Y_1$ and $Y_2$ are selected independently from arginine (R) and lysine (K);
P is proline;
m is 0 or 1;
k is an integer between 3 and 8;
Ψ represents a modified peptide bond significantly more resistant to at least one protease than a standard peptide bond,
and the support is a linear peptide comprising at least k lysines,
with the exception of compounds whose support is a non-cyclic peptide comprising an amino acid sequence selected from KPG, KGP, KGC, or $KX_1KX_4KX_1K$, where $X_1$ is optional and selected from lysine (K), valine (V), alanine (A), glutamic acid (E) and isoleucine (I), and $X_4$ is optional and selected from valine (V), alanine (A), glutamic acid (E) and isoleucine (I).

**13.** Compound according to claim 12, **characterized in that** the support is a linear peptide consisting of 3 to 8 repetitions of peptide units of sequence Aib-K-Aib-G (SEQ ID NO:6) or K-Aib-G (SEQ ID NO:7), respectively.

**14.** Compound according to claim 13, **characterized in that** the support consists of an amino acid sequence selected from SEQ ID NO:8, SEQ ID NO:9, SEQ ID NO:14, SEQ ID NO:15, SEQ ID NO:18 and SEQ ID NO:19.

**15.** Compound according to any one of claims 12 to 14, **characterized in that** Ψ represents a reduced bond ($-CH_2NH-$), a retro-inverso bond (-NHCO-), a methylene-oxy bond ($-CH_2-O-$), a thiomethylene bond ($-CH_2-S-$), a carba bond ($-CH_2CH_2-$), a ketomethylene bond ($-CO-CH_2-$), a hydroxyethylene bond ($-CHOH-CH_2-$), a (-N-N-) bond, an E-alkene bond or a (-CH=CH-) bond.

**16.** Compound according to any one of claims 12 to 15, **characterized in that** m is equal to 0.

**17.** Compound according to claim 12, **characterized in that** it is selected from compounds whose structure is described in Figure 2C (SEQ ID NO:20), Figure 2D (SEQ ID NO:21) and Figure 2E (SEQ ID NO:16).

**18.** Compound whose structure is described in Figure 2F (SEQ ID NO:17).

**19.** Compound according to any one of claims 12 to 18, for use as medicament.

**20.** Pharmaceutical composition comprising a compound according to any one of claims 12 to 18.

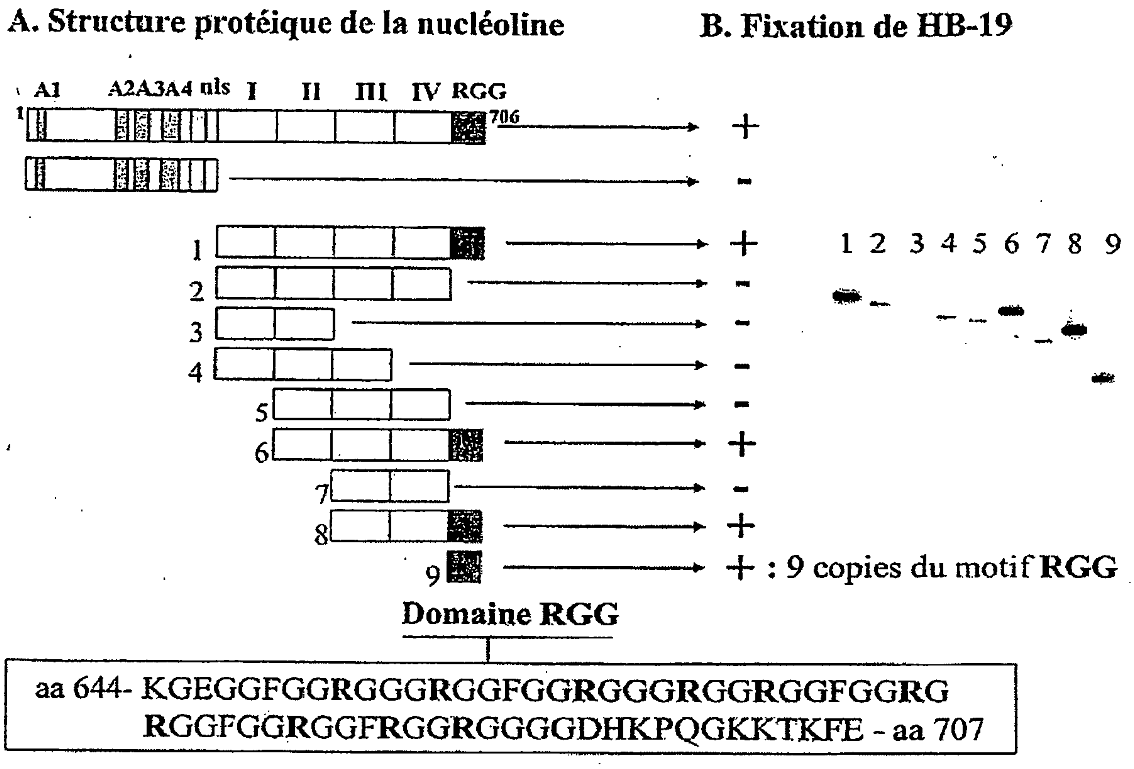
A. Structure protéique de la nucléoline
B. Fixation de HB-19
A1 A2A3A4 nls I II III IV RGG
1
706
+
-
1
+
2
-
3
-
4
-
5
-
6
+
7
-
8
+
9
+ : 9 copies du motif RGG
1 2 3 4 5 6 7 8 9
Domaine RGG
aa 644- KGEGGFGGRGGGRGGFGGRGGGRGGRGGFGGRG
RGGFGGRGGFRGGRGGGGDHKPQGKKTKFE - aa 707

**Figure 1**

A

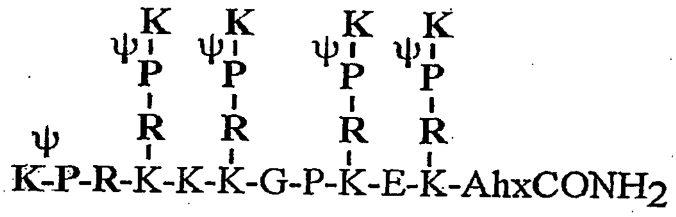

HB-19

B

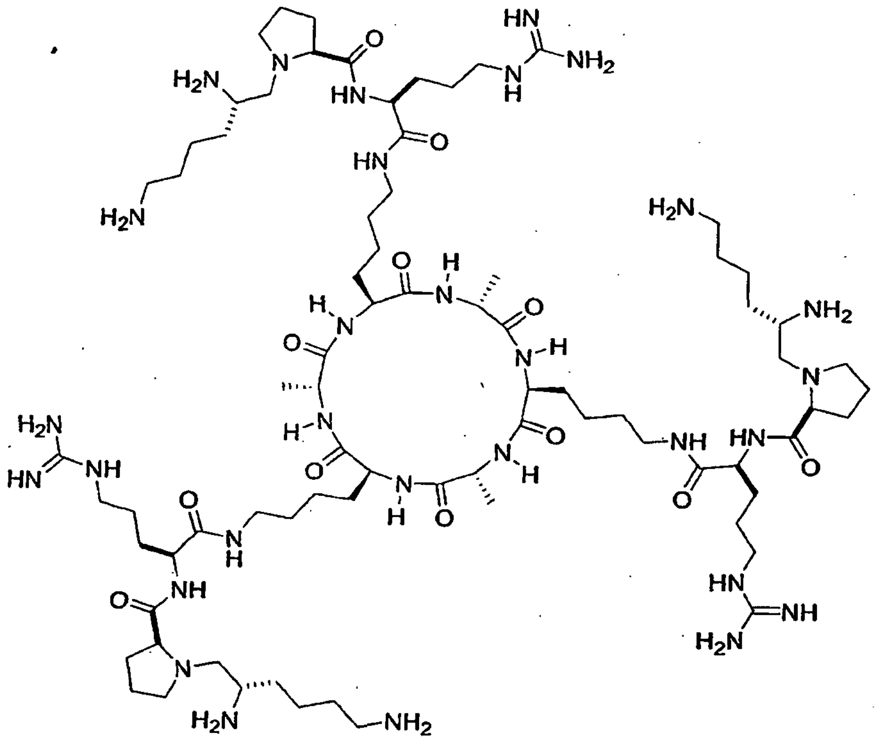

Nucant 01

Figure 2

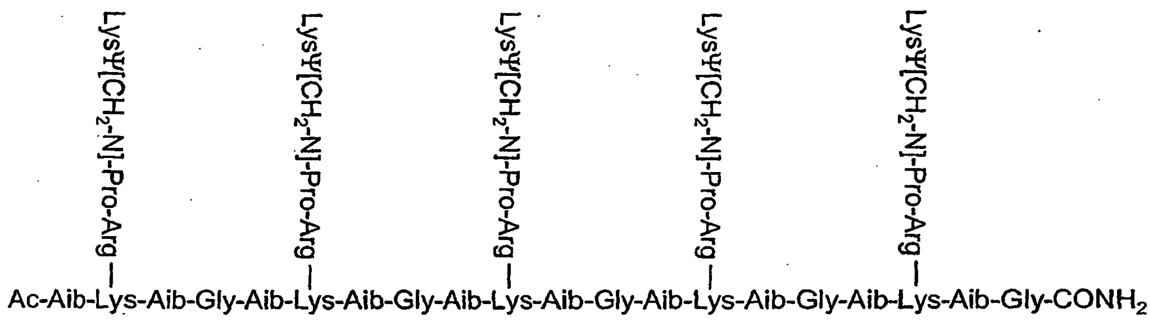

**Nucant 2**

D

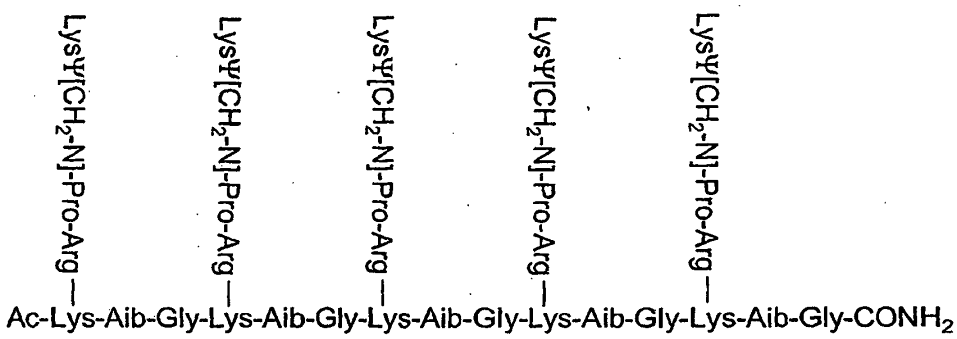

**Nucant 3**

**Figure 2 (suite)**

**E**

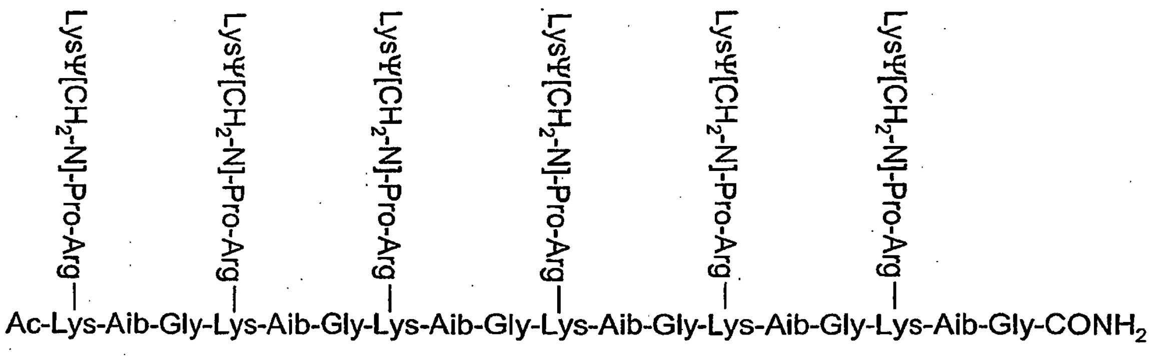

**Nucant 6**

**F**

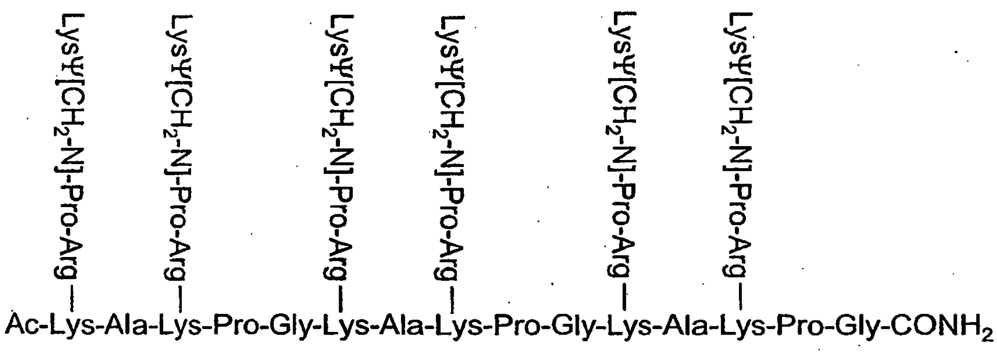

**Nucant 7**

**Figure 2 (suite)**

A

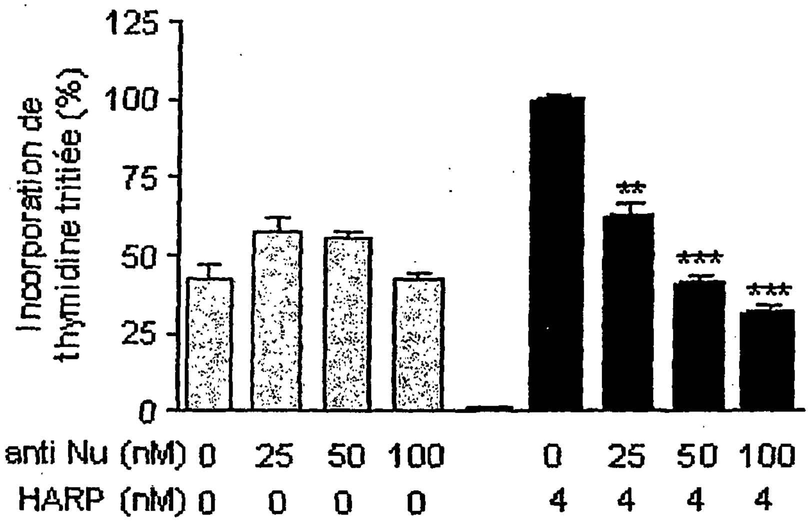
Incorporation de thymidine tritiée (%)
125
100
75
50
25
0
**
***
***
anti Nu (nM) 0 25 50 100 0 25 50 100
HARP (nM) 0 0 0 0 4 4 4 4

B

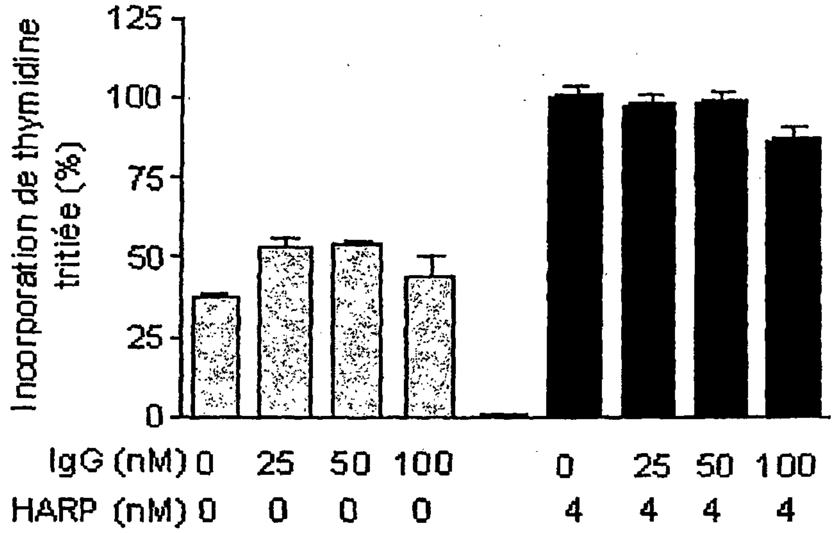
Incorporation de thymidine tritiée (%)
125
100
75
50
25
0
IgG (nM) 0 25 50 100 0 25 50 100
HARP (nM) 0 0 0 0 4 4 4 4

**Figure 3**

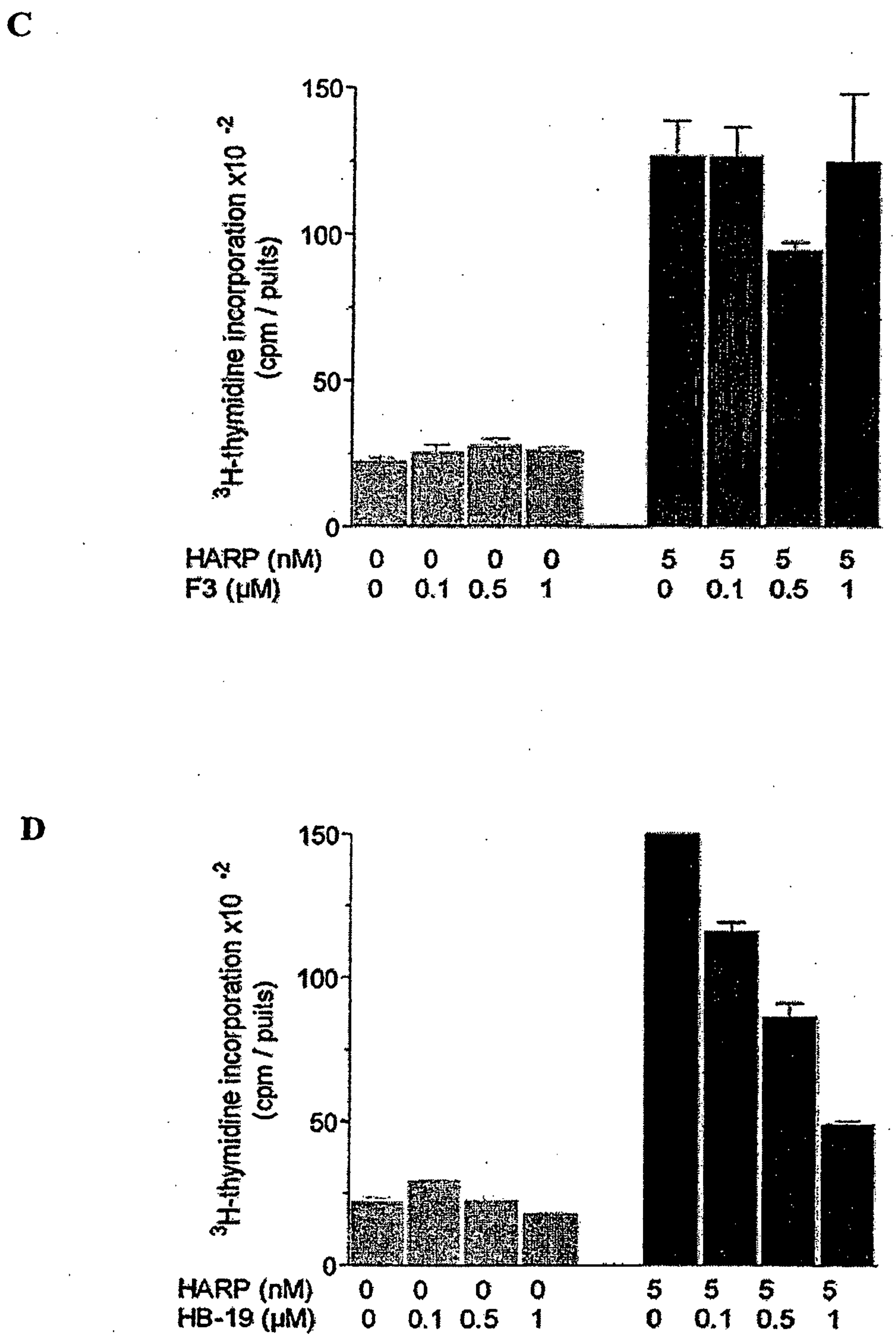


**Figure 3 (suite)**

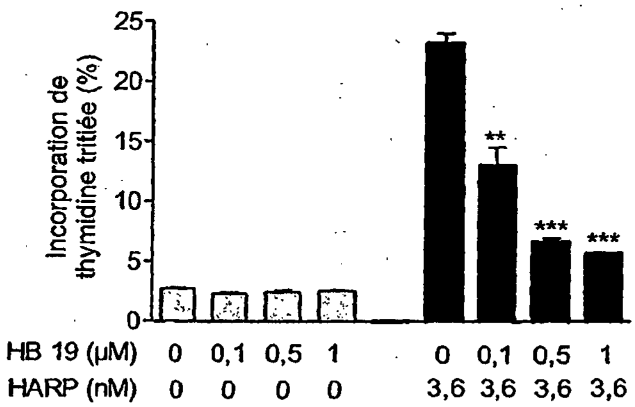


**Figure 4**

A

B

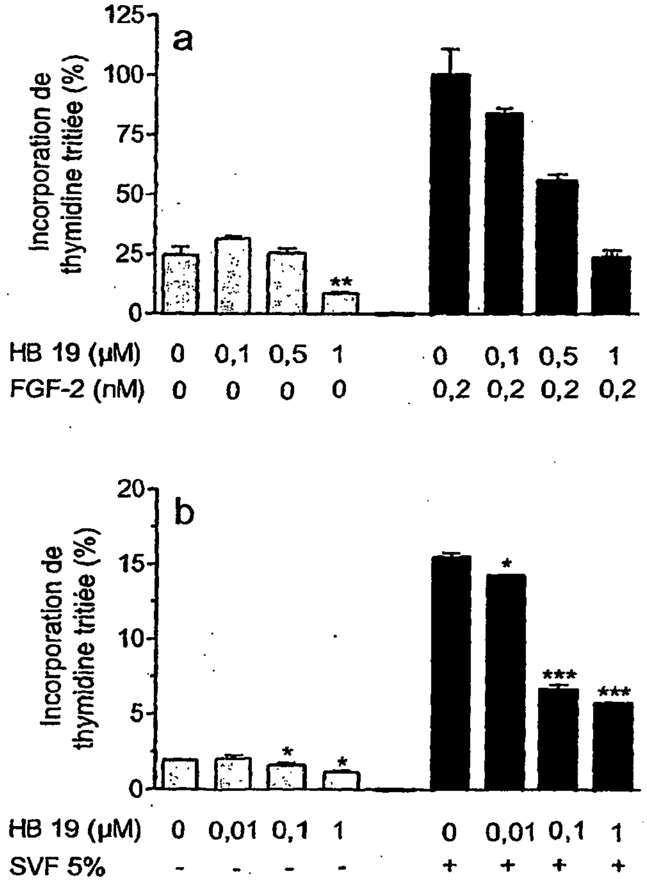


**Figure 5**

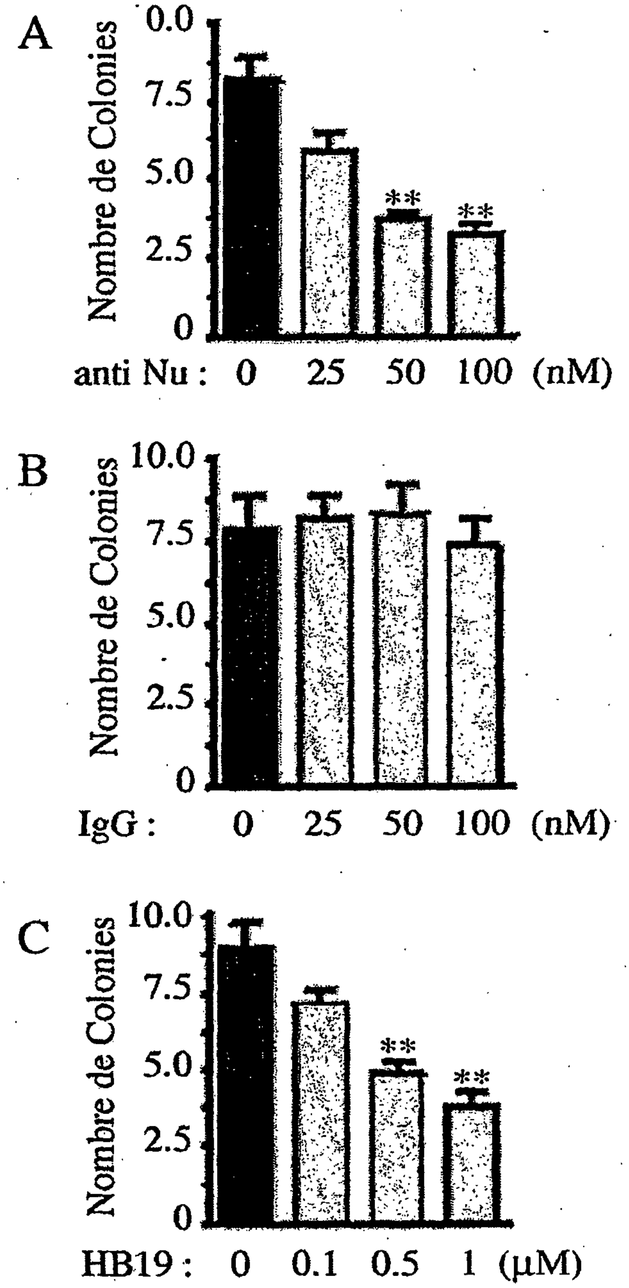
A
Nombre de Colonies
0.0
7.5
5.0
2.5
0
**
**
anti Nu : 0 25 50 100 (nM)
B
Nombre de Colonies
10.0
7.5
5.0
2.5
0
IgG : 0 25 50 100 (nM)
C
Nombre de Colonies
10.0
7.5
5.0
2.5
0
**
**
HB19 : 0 0.1 0.5 1 (µM)

**Figure 6**

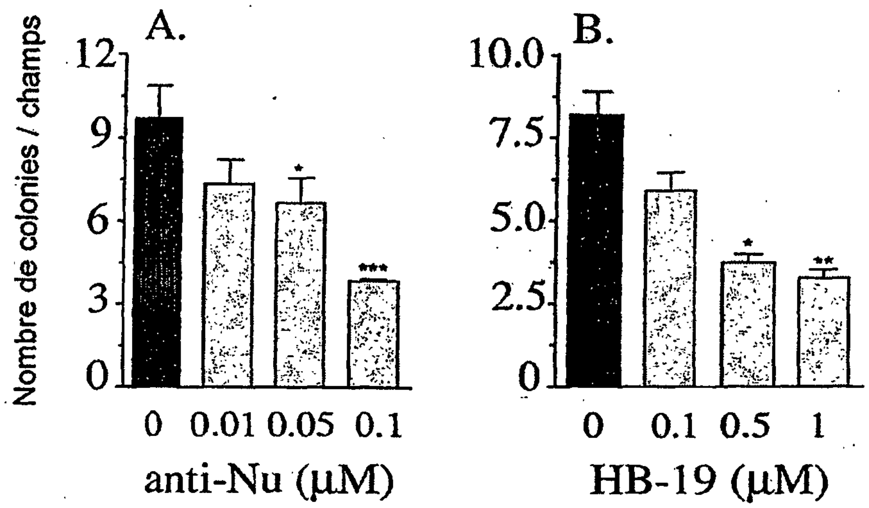
Nombre de colonies / champs
A.
12
9
6
3
0
0 0.01 0.05 0.1
anti-Nu (µM)
B.
10.0
7.5
5.0
2.5
0
0 0.1 0.5 1
HB-19 (µM)
*
***
**

**Figure 7**

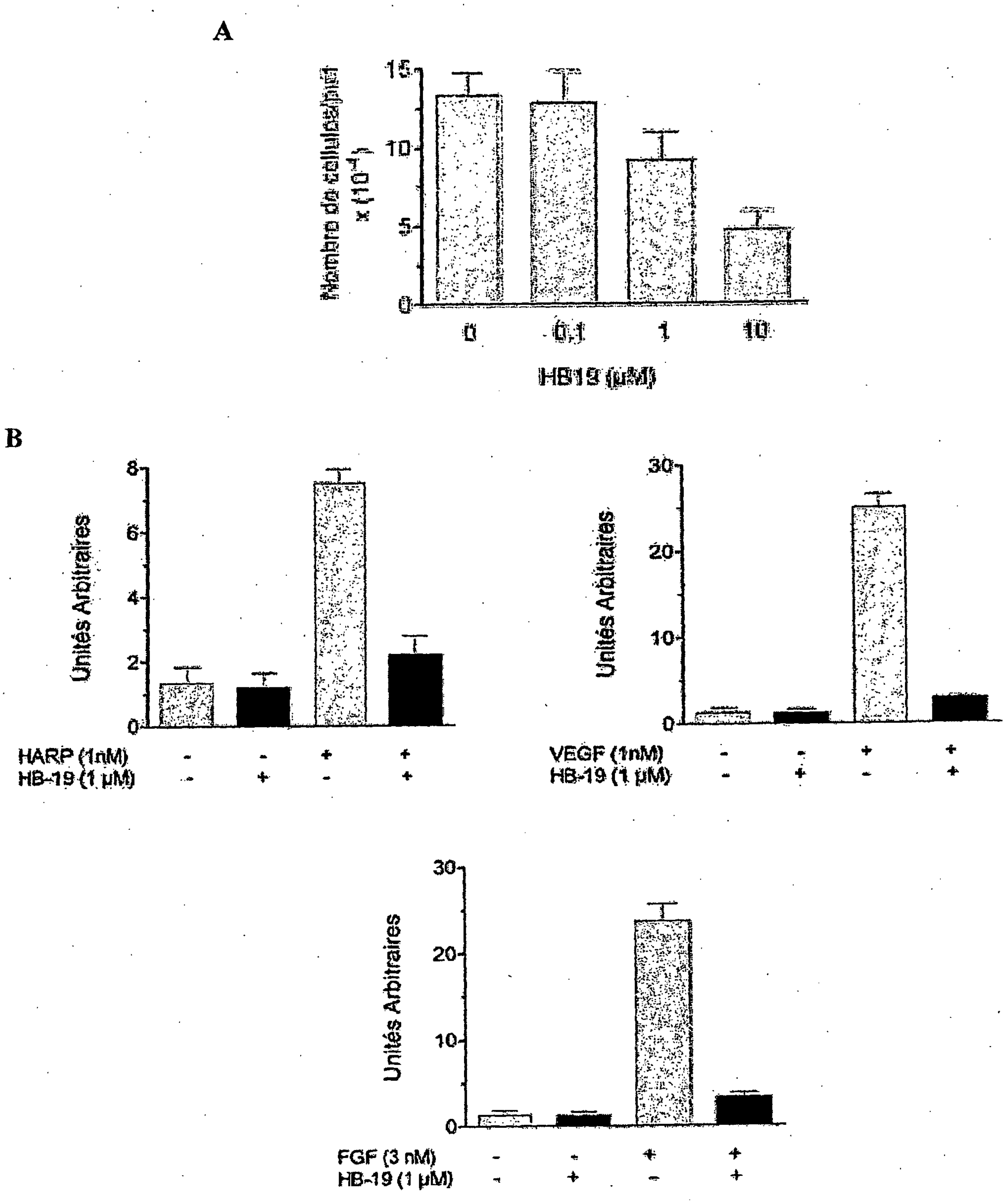
A
15
10
5
0
0
0,1
1
10
HB19 (µM)
B
Unités Arbitraires
8
6
4
2
0
HARP (1nM) - - + +
HB-19 (1 µM) - + - +
Unités Arbitraires
30
20
10
0
VEGF (1nM) - - + +
HB-19 (1 µM) - + - +
Unités Arbitraires
30
20
10
0
FGF (3 nM) - - + +
HB-19 (1 µM) - + - +

Figure 8

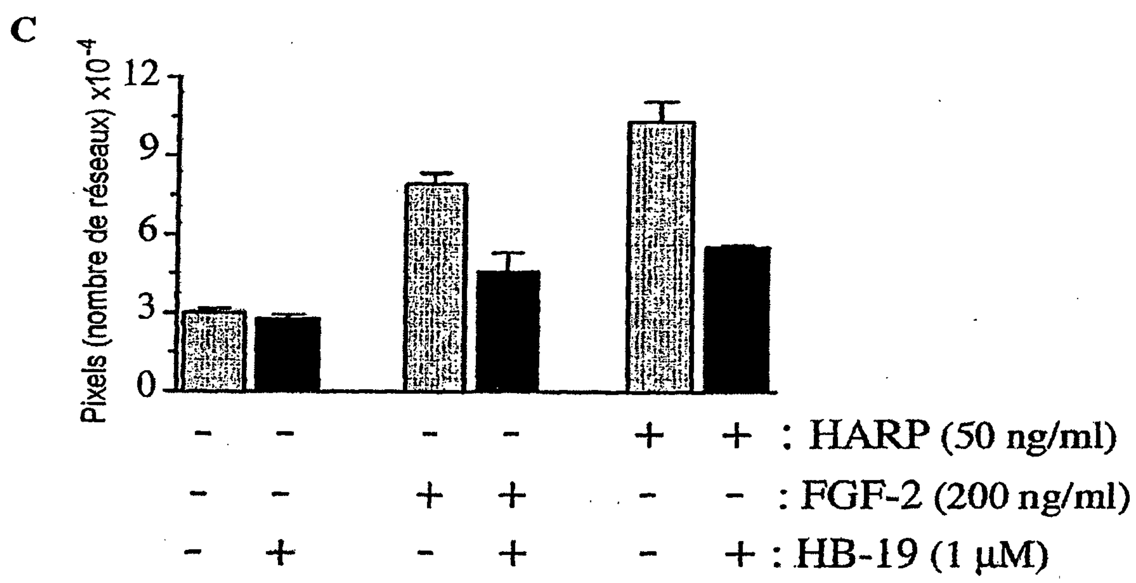


**Figure 8 (suite)**

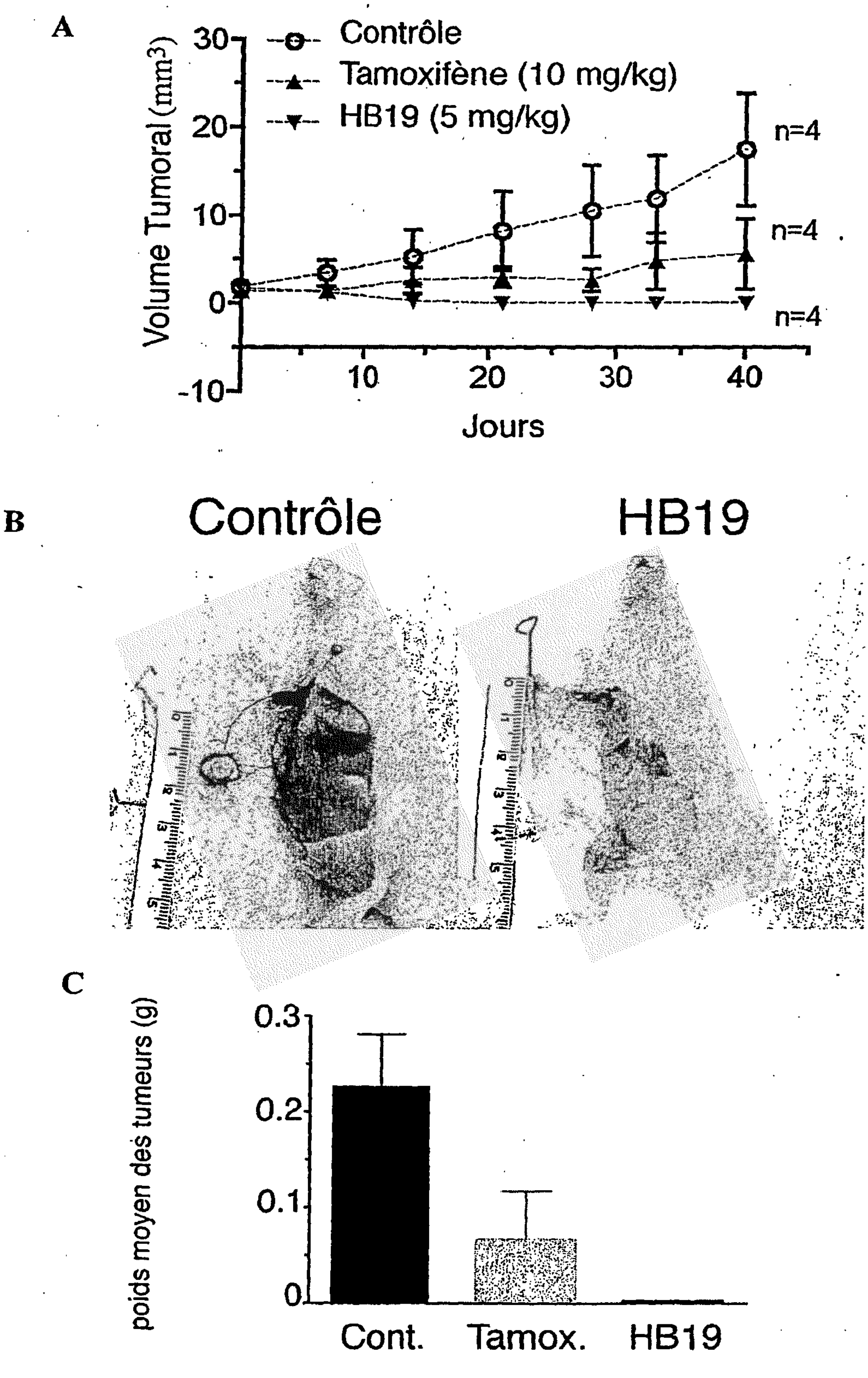
A
Contrôle
Tamoxifène (10 mg/kg)
HB19 (5 mg/kg)
Volume Tumoral (mm3)
30
20
10
0
-10
10
20
30
40
Jours
n=4
n=4
n=4
B
Contrôle
HB19
C
poids moyen des tumeurs (g)
0.3
0.2
0.1
0
Cont.
Tamox.
HB19

**Figure 9**

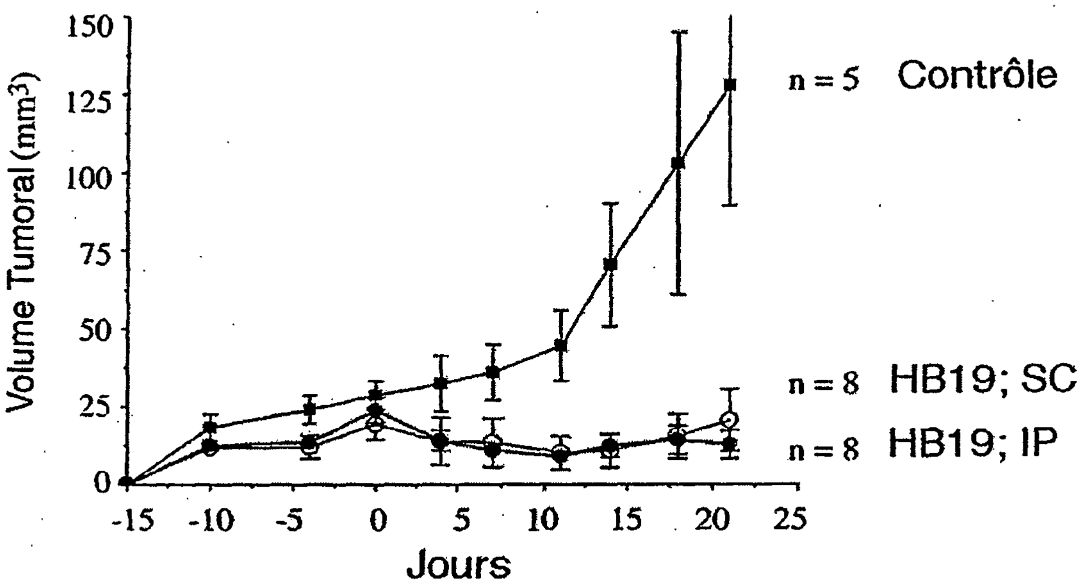
150
125
100
75
50
25
0
Volume Tumoral (mm³)
-15 -10 -5 0 5 10 15 20 25
Jours
n = 5 Contrôle
n = 8 HB19; SC
n = 8 HB19; IP

**Figure 10**

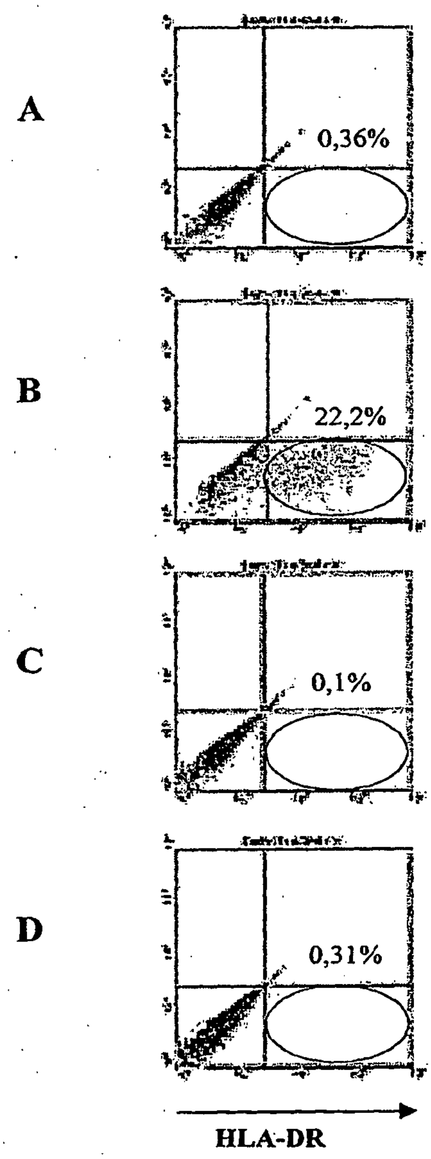


Figure 11

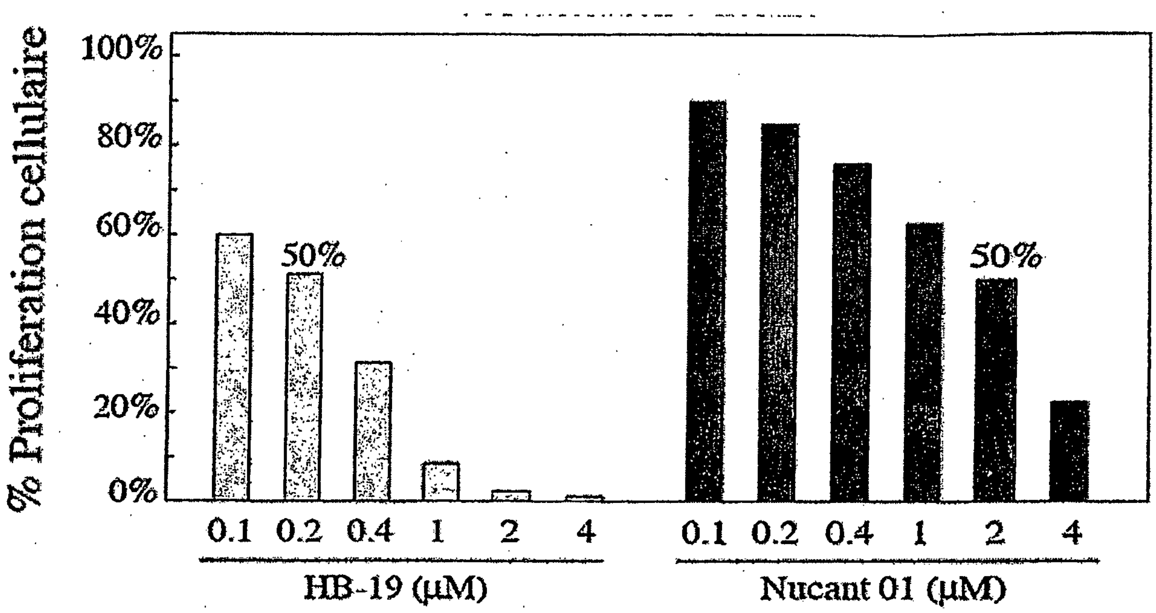


**Figure 12**

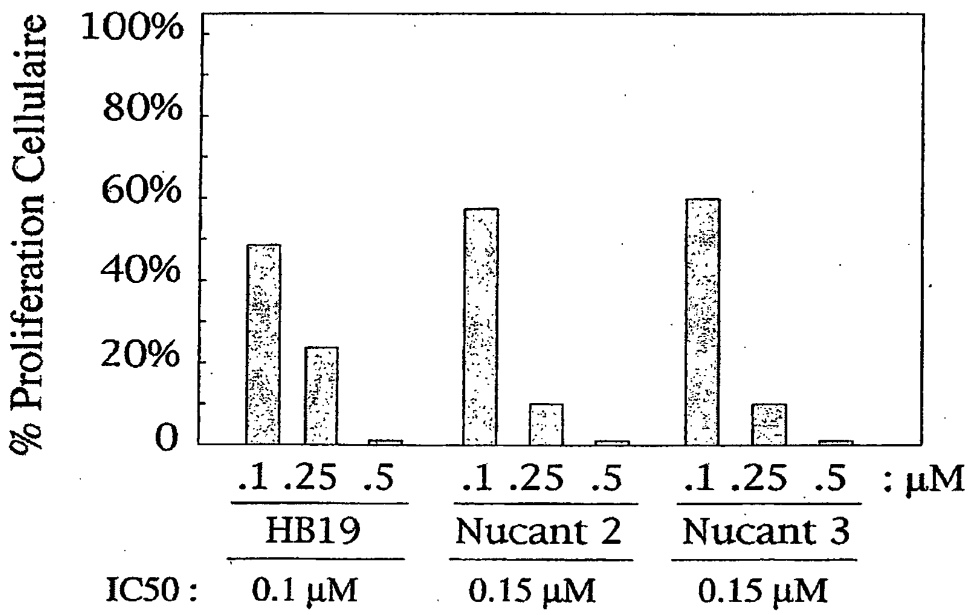


**Figure 13**

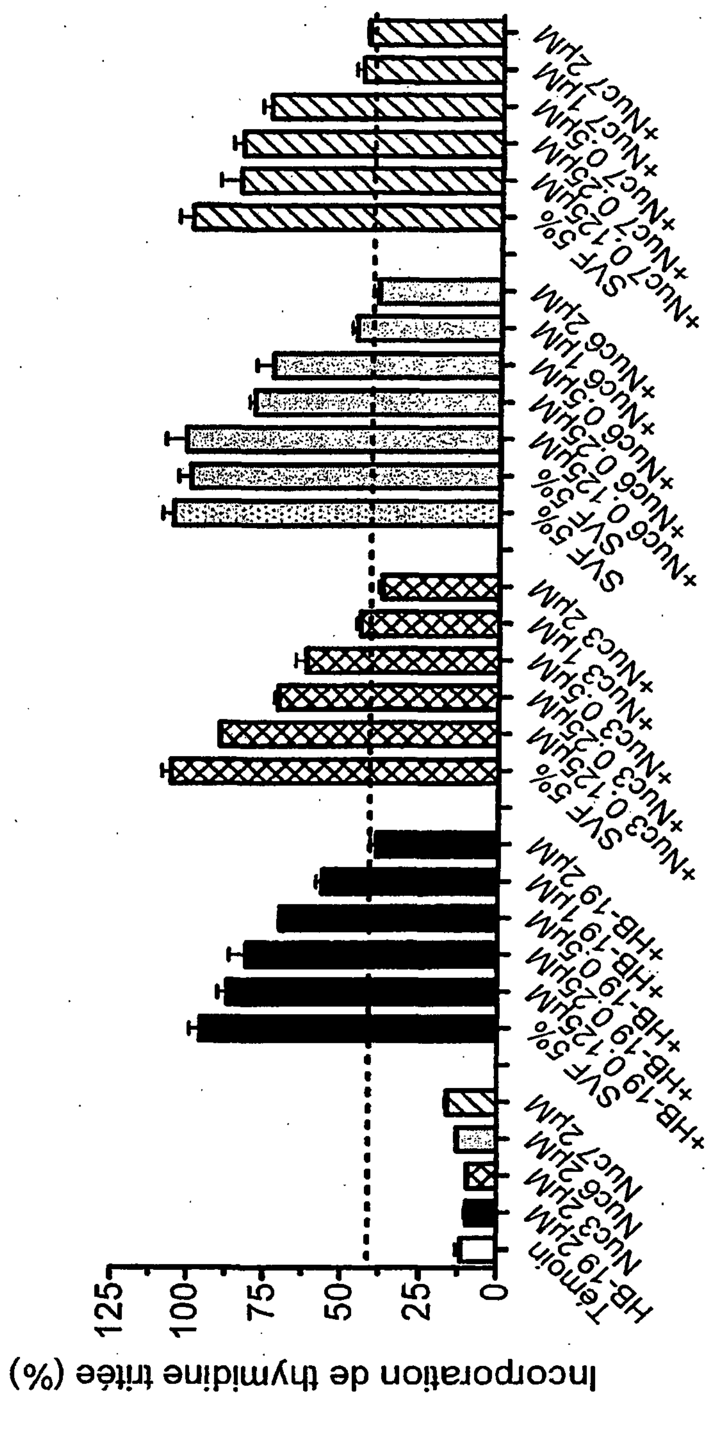
Incorporation de thymidine tritée (%)
125
100
75
50
25
0
Temoin
HB-19 2µM
Nuc3 2µM
Nuc6 2µM
Nuc7 2µM
SVF 5%
+HB-19 0.125µM
+HB-19 0.25µM
+HB-19 0.5µM
+HB-19 1µM
+HB-19 2µM
SVF 5%
+Nuc3 0.125µM
+Nuc3 0.25µM
+Nuc3 0.5µM
+Nuc3 1µM
+Nuc3 2µM
SVF 5%
+Nuc6 0.125µM
+Nuc6 0.25µM
+Nuc6 0.5µM
+Nuc6 1µM
+Nuc6 2µM
SVF 5%
+Nuc7 0.125µM
+Nuc7 0.25µM
+Nuc7 0.5µM
+Nuc7 1µM
+Nuc7 2µM

Figure 14

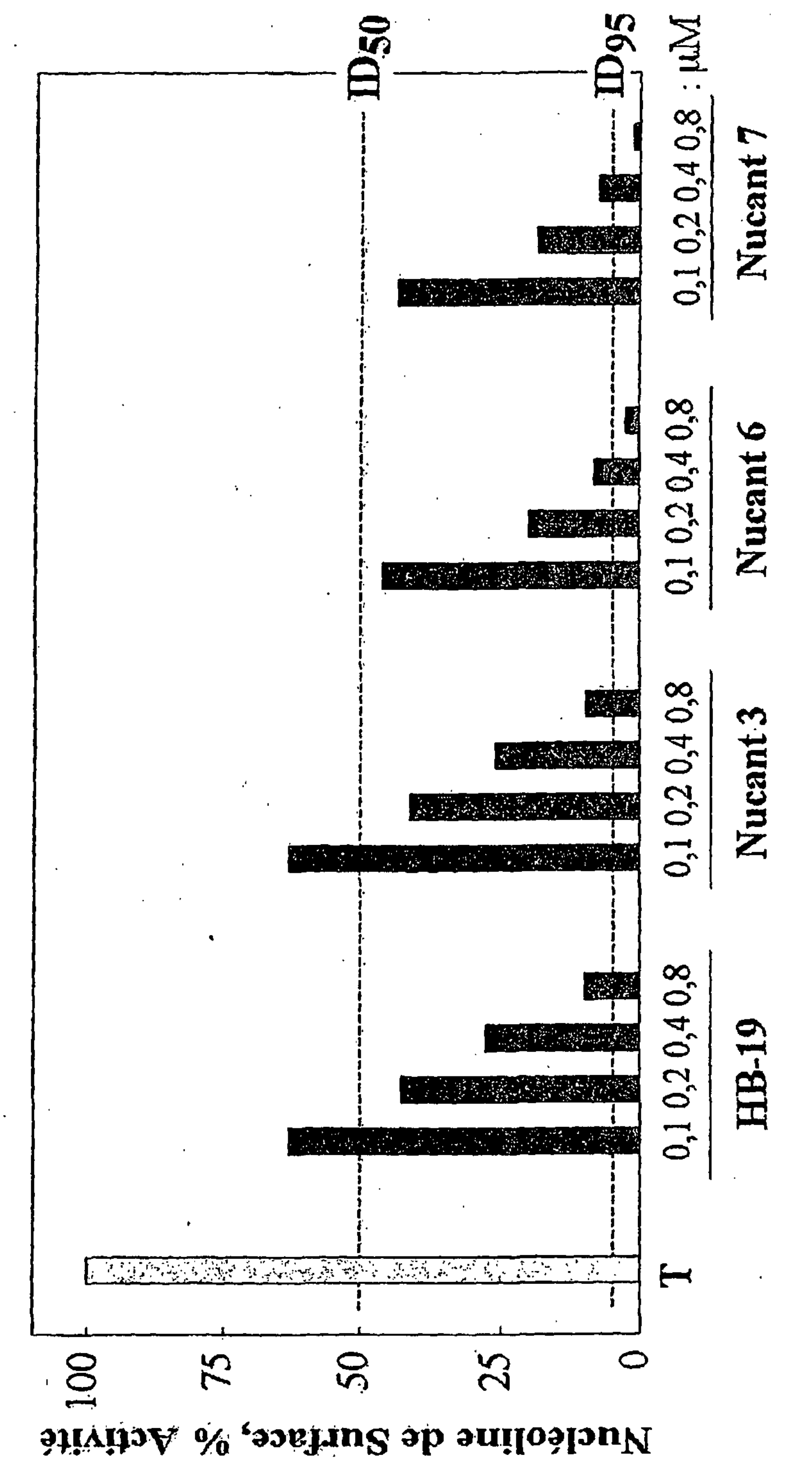
Nucléoline de Surface, % Activité
100
75
50
25
0
ID50
ID95
T
0,1 0,2 0,4 0,8
HB-19
0,1 0,2 0,4 0,8
Nucant 3
0,1 0,2 0,4 0,8
Nucant 6
0,1 0,2 0,4 0,8 : µM
Nucant 7

**Figure 15**

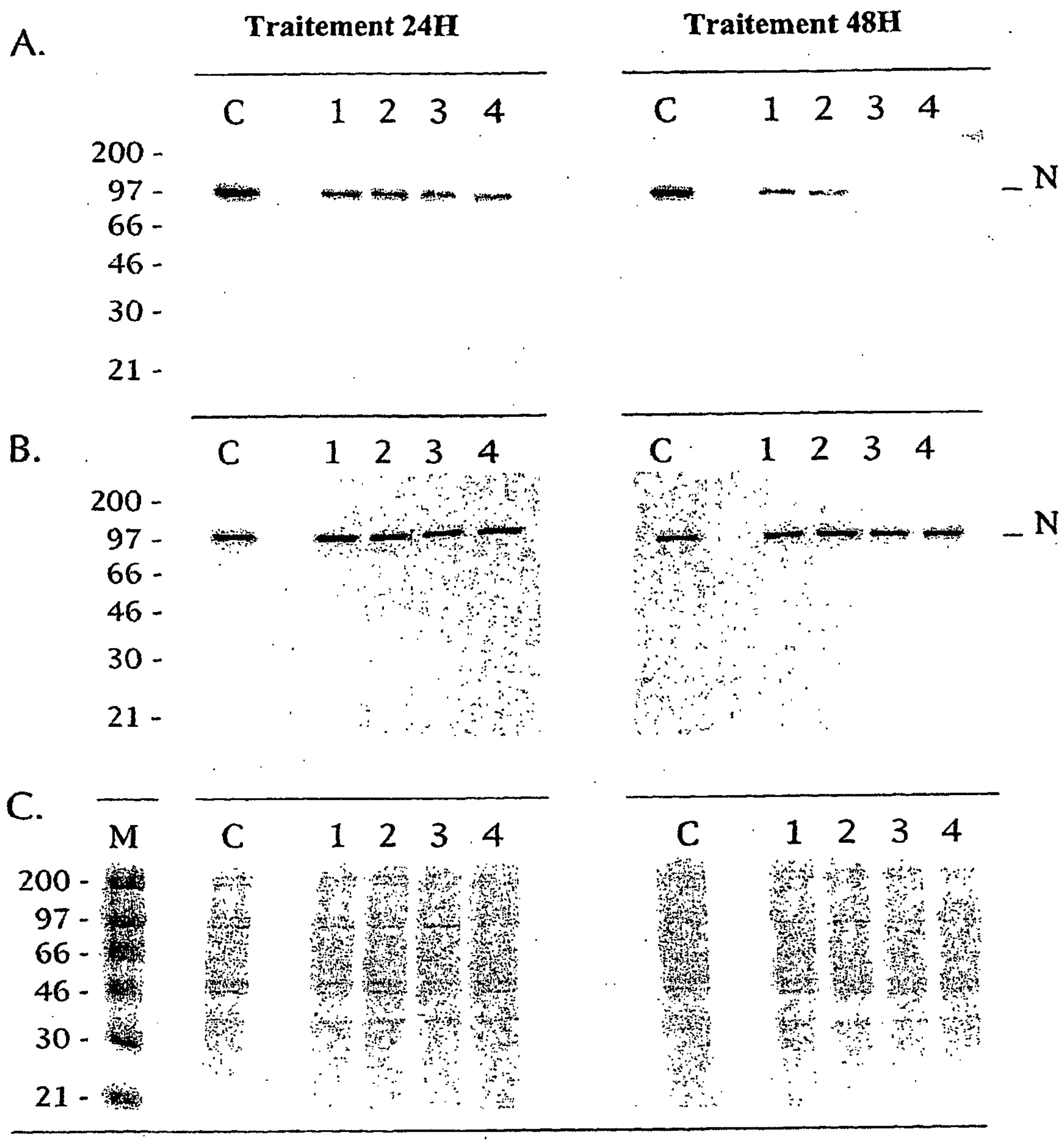
A.
Traitement 24H
Traitement 48H
C 1 2 3 4
C 1 2 3 4
200 -
97 -
66 -
46 -
30 -
21 -
N
B.
C 1 2 3 4
C 1 2 3 4
200 -
97 -
66 -
46 -
30 -
21 -
N
C.
M
C 1 2 3 4
C 1 2 3 4
200 -
97 -
66 -
46 -
30 -
21 -

**Figure 16**

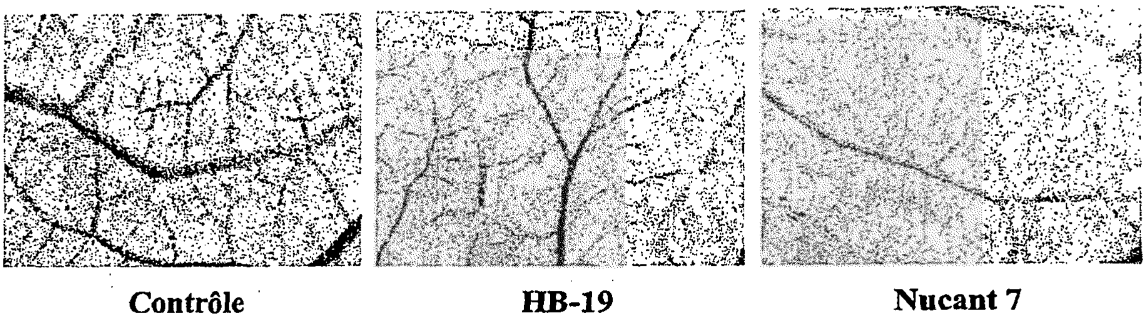

Contrôle HB-19 Nucant 7

Figure 17

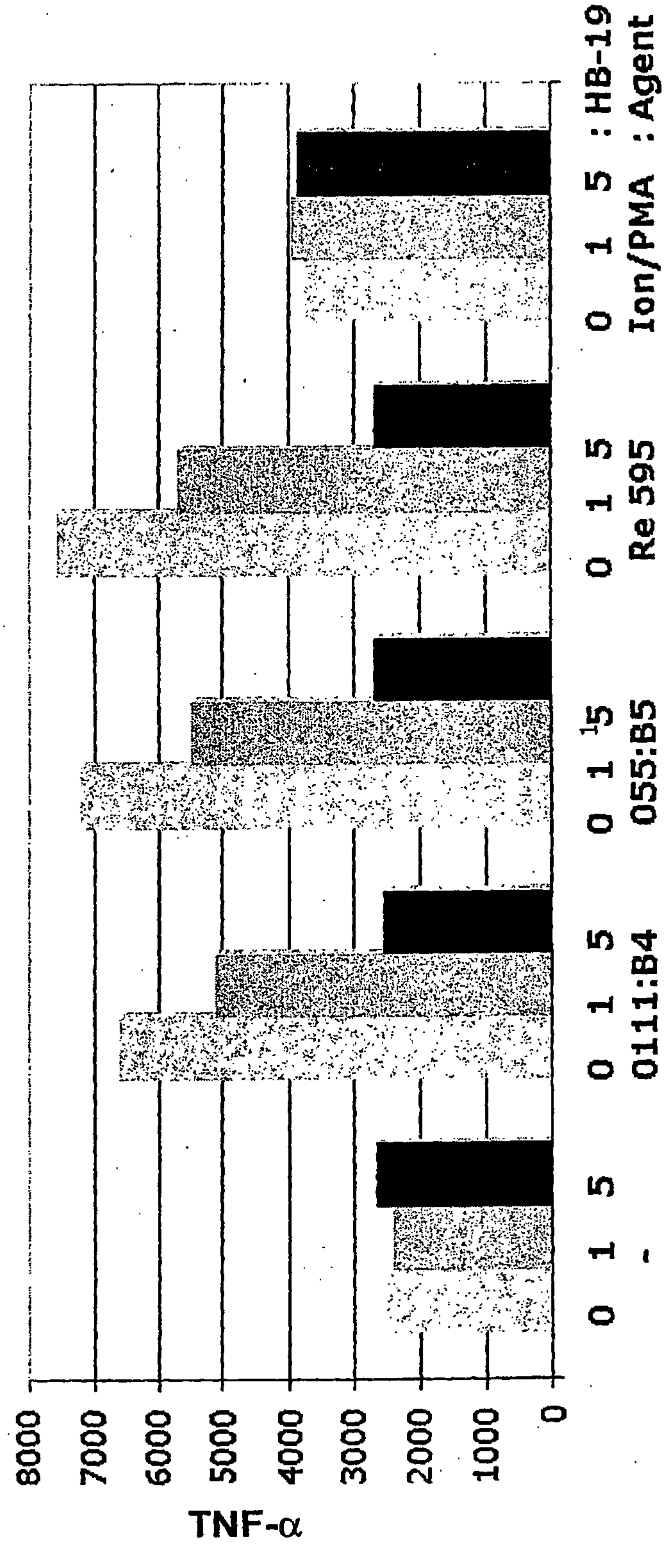
TNF-α
8000
7000
6000
5000
4000
3000
2000
1000
0
0 1 5
-
0 1 5
0111:B4
0 1 5
055:B5
0 1 5
Re 595
0 1 5 : HB-19
Ion/PMA : Agent

Figure 18

A

B

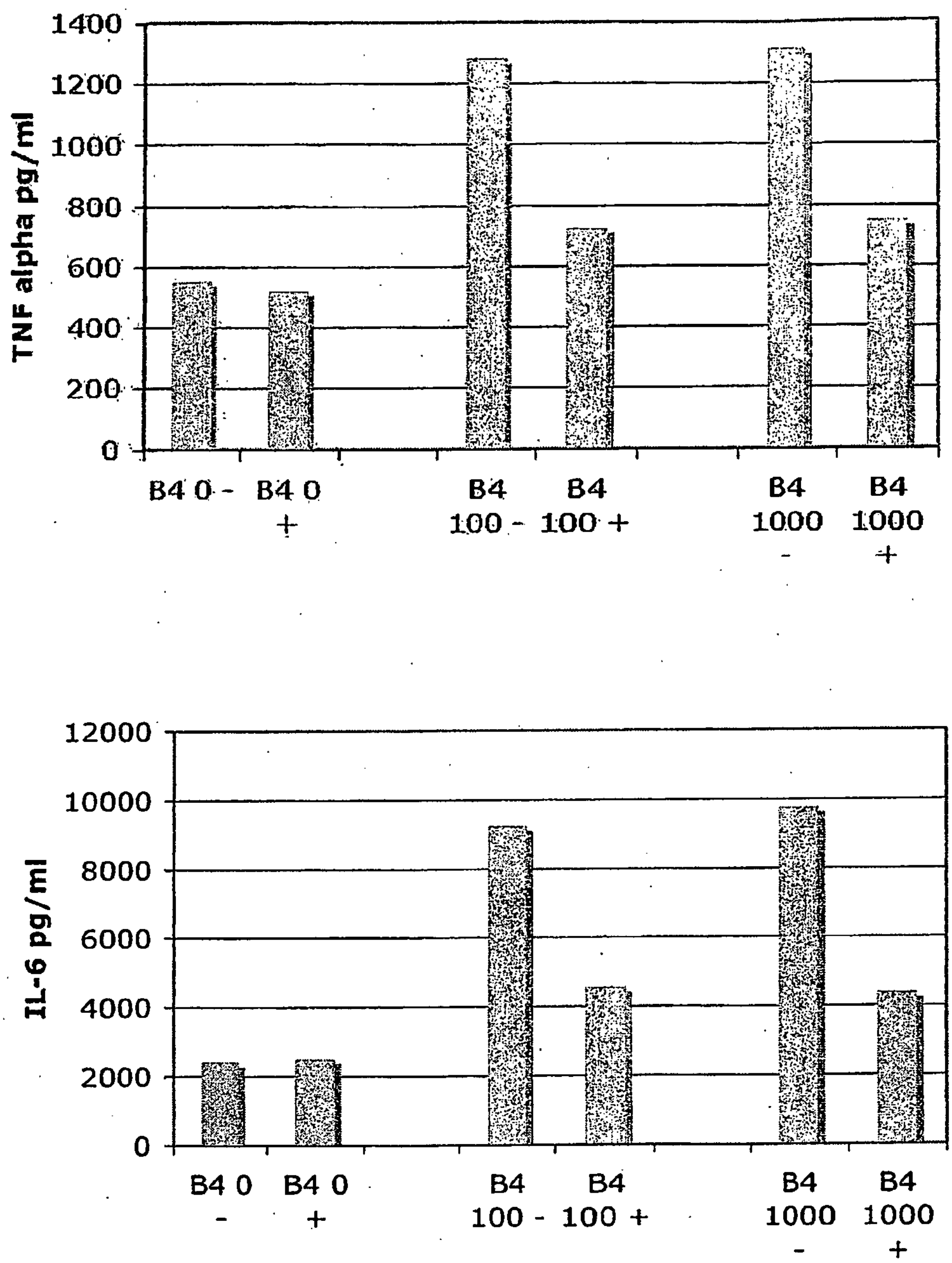
1400
1200
1000
800
600
400
200
0
TNF alpha pg/ml
B4 0 -
B4 0 +
B4 100 -
B4 100 +
B4 1000 -
B4 1000 +
12000
10000
8000
6000
4000
2000
0
IL-6 pg/ml
B4 0 -
B4 0 +
B4 100 -
B4 100 +
B4 1000 -
B4 1000 +

**Figure 19**

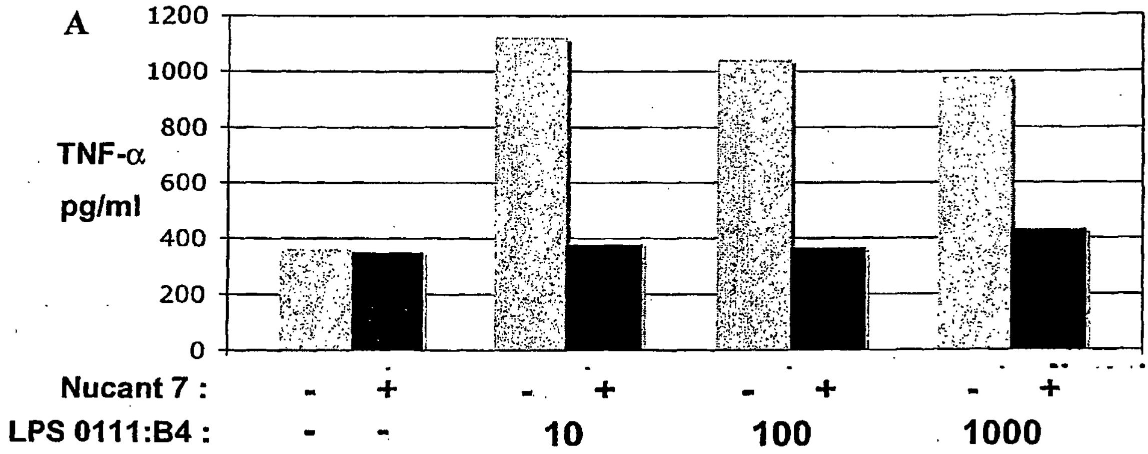
A
1200
1000
800
600
400
200
0
TNF-α pg/ml
Nucant 7 : - + - + - + - +
LPS 0111:B4 : - - 10 100 1000

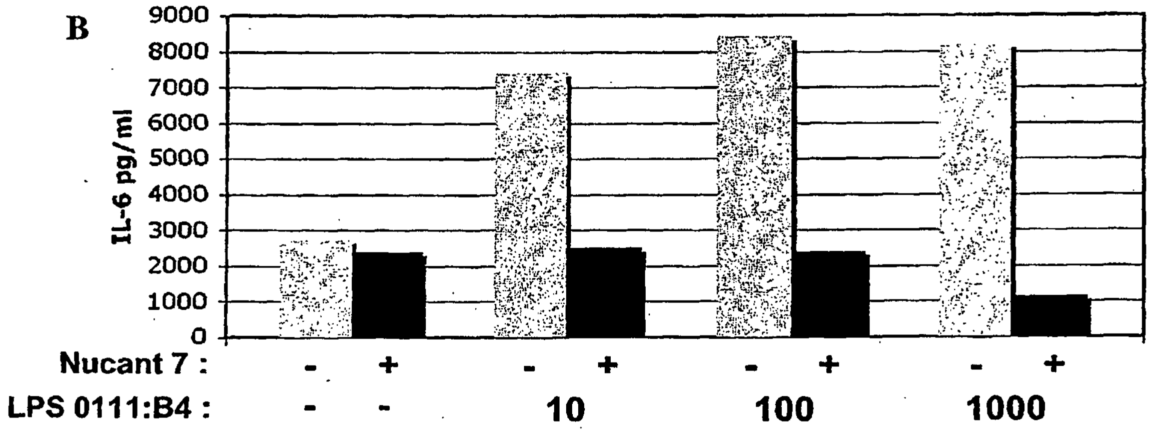
B
9000
8000
7000
6000
5000
4000
3000
2000
1000
0
IL-6 pg/ml
Nucant 7 : - + - + - + - +
LPS 0111:B4 : - - 10 100 1000

Figure 20

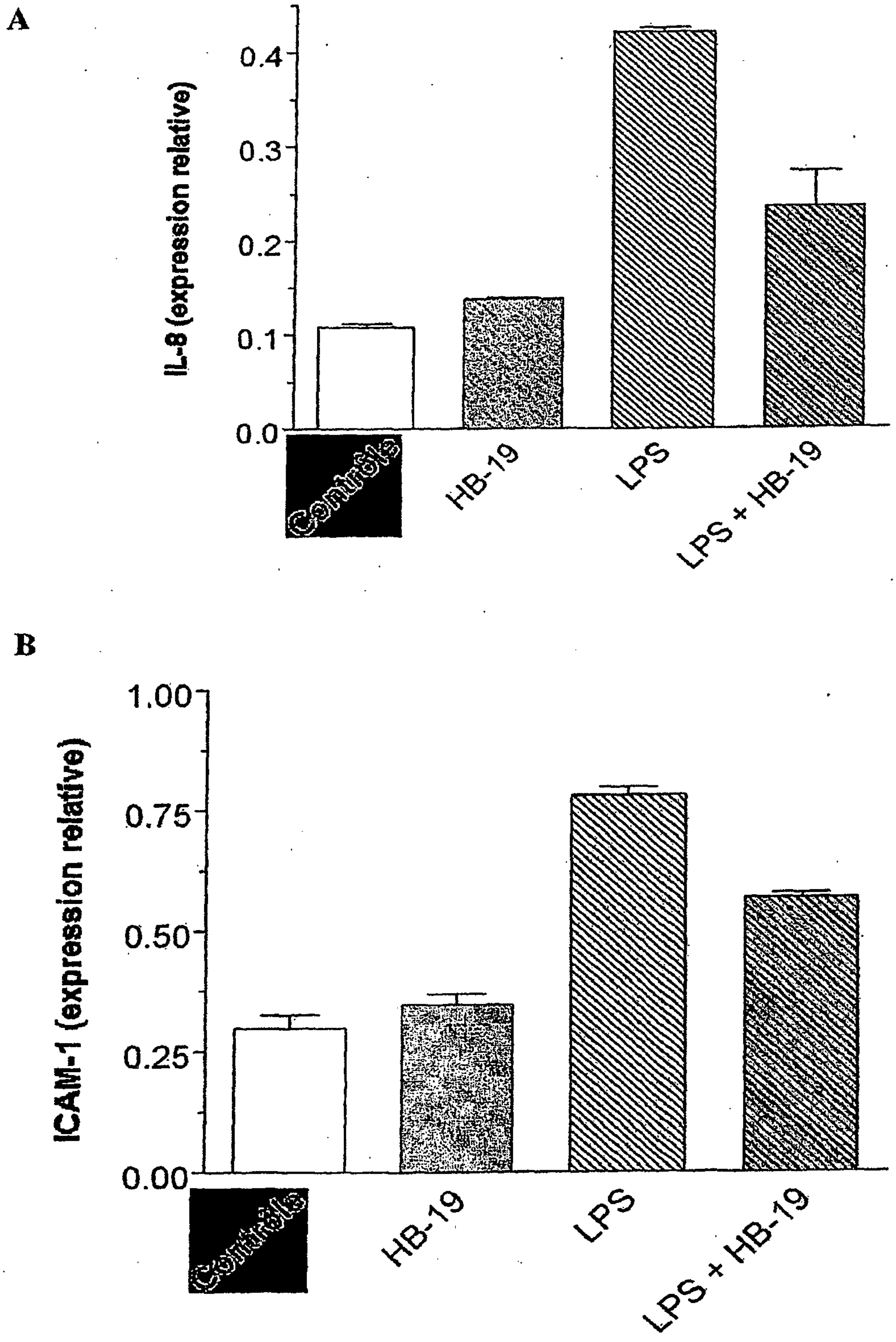
A
IL-8 (expression relative)
0.0
0.1
0.2
0.3
0.4
Controls
HB-19
LPS
LPS + HB-19
B
ICAM-1 (expression relative)
0.00
0.25
0.50
0.75
1.00
Controls
HB-19
LPS
LPS + HB-19

**Figure 21**

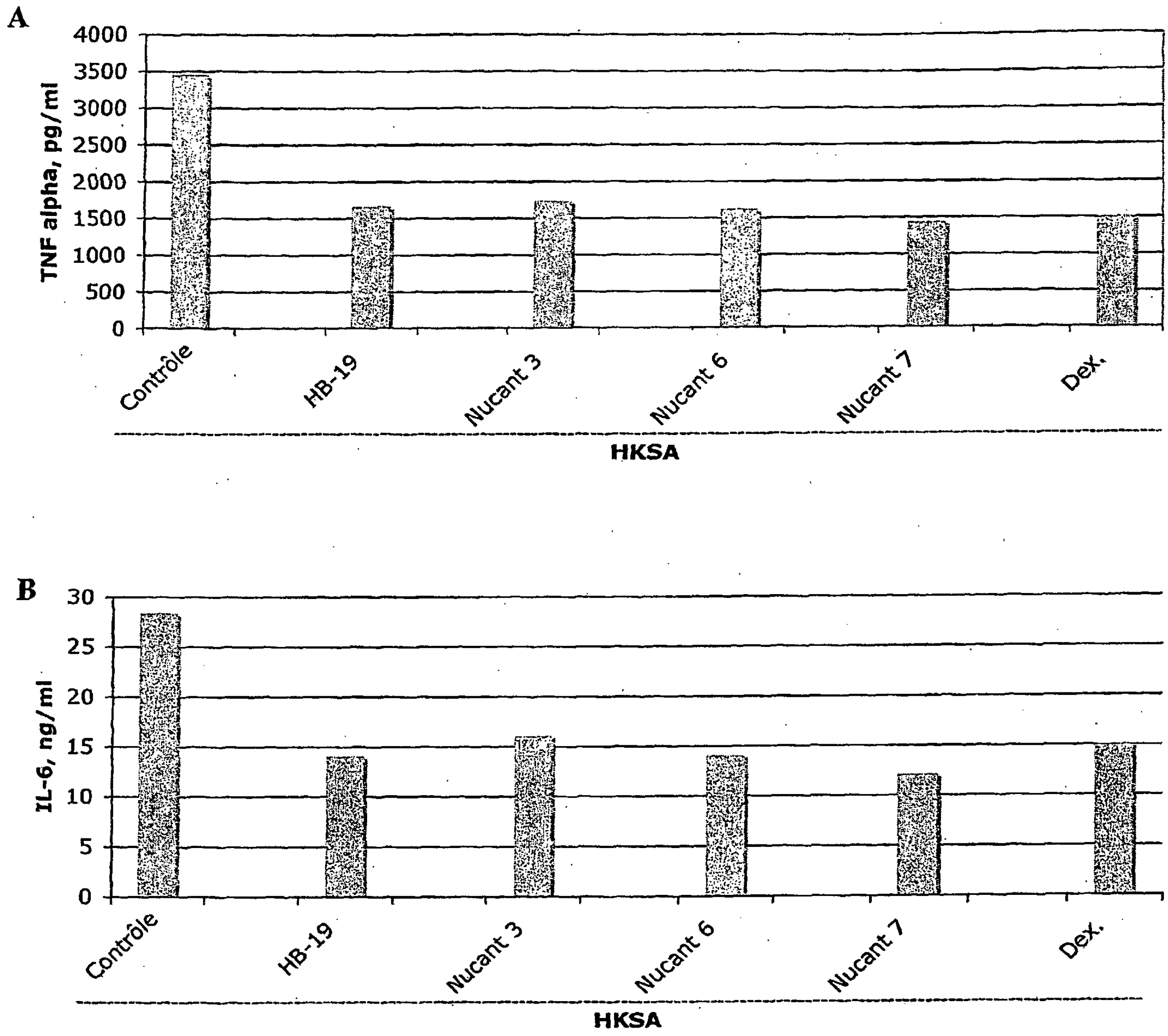
A
4000
3500
3000
2500
2000
1500
1000
500
0
TNF alpha, pg/ml
Contrôle
HB-19
Nucant 3
Nucant 6
Nucant 7
Dex.
HKSA
B
30
25
20
15
10
5
0
IL-6, ng/ml
Contrôle
HB-19
Nucant 3
Nucant 6
Nucant 7
Dex.
HKSA

Figure 22

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005035579 A
- US 20050026860 A
- WO 0061597 A
- US 2004186056 A
- WO 9529190 A
- FR 0603813


### Littérature non-brevet citée dans la description


- **HOVANESSIAN, A.G. ; PUVION-DUTILLEUL, F. ; NISOLE, S. ; SVAB, J. ; PERRET, E. ; DENG, J. S. ; KRUST, B.** The cell-surface-expressed nucleolin is associated with the actin cytoskeleton. *Exp. Cell Res.,* 2000, vol. 261, 312-328
- **NISOLE, S. ; KRUST, B. ; CALLEBAUT, C. ; GUICHARD, G. ; MULLER, S. ; BRIAND, J. P. ; HOVANESSIAN, A. G.** The anti-HIV composé HB-19 forms a complex with the cell-surface-expressed nucleolin independent of heparan sulfate proteoglycans. *J. Biol. Chem.,* 1999, vol. 274, 27875-27884
- **GINISTY, H. ; SICARD, H. ; ROGER, B. ; BOUVET, P.** Structure and functions of nucleolin. *J. Cell Science,* 1999, vol. 112, 761-772
- **SRIVASTAVA, M. ; POLLARD, H. B.** Molecular dissection of nucleolin's role in growth and cell prolifération: new insights. *FASEB J.,* 1999, vol. 13, 1911-1922
- **NISOLE, S. ; KRUST, B. ; HOVANESSIAN, A. G.** Anchorage of HIV on permissive cells leads to coaggregation of viral particles with surface nucleolin at membrane raft microdomains. *Exp. Cell Res.,* 2002, vol. 276, 155-173
- **SEMENKOVICH, C. F. ; OSTLUND, R. E. J. ; OLSON, M. O. ; YANG, J. W.** A protein partially expressed on the surface of HepG2 cells that binds lipoproteins specifically is nucleolin. *Biochemistry,* 1990, vol. 29, 9708-9713
- **CALLEBAUT, C. ; JACOTOT, E. ; KRUST, B. ; GUICHAR, G. ; BLANCO, J. ; SVAB, J. ; MULLER, S. ; BRIAND, J. P. ; HOVANESSIAN, A. G.** Composé TASP inhibitors of HIV entry bind specifically to a 95-kDa cell surface protein. *J. Biol. Chem.,* 1997, vol. 272, 7159-7166
- **CALLEBAUT, C. ; BLANCO, J. ; BENKIRANE, N. ; KRUST, B. ; JACOTOT, E. ; GUICHARD, G. ; SEDDIKI, N. ; SVAB, J. ; DAM, E. ; MULLER, S.** Identification of V3 loop-binding proteins as potential receptors implicated in the binding ofHIV particles to CD4(+) cells. *J. Biol. Chem.,* 1998, vol. 273, 21988-2199
- **CHRISTIAN, S. ; PILCH, J. ; AKERMAN, M. E. ; PORKKA, K. ; LAAKKONEN, P. ; RUOSLAHTI, E.** Nucleolin expressed at the cell surface is a marker of endothelial cells in angiogenic blood vessels. *J. Cell Biol.,* 2003, vol. 163, 871-878
- **SAID, A. E. ; KRUST, B. ; NISOLE, S. ; BRIAND, J. P. ; HOVANESSIAN, A. G.** The anti-HIV cytokine midkine binds the cell surface-expressed nucleolin as a low affinity receptor. *J. Biol. Chem.,* 2002, vol. 277, 37492-37502
- **SAID, E. A. ; COURTY, J. ; SVAB, J. ; DELBÉ, J. ; KRUST, B. ; HOVANESSIAN, A. G.** Pleiotrophin inhibits HIV infection by binding the cell surface-expressed nucleolin. *FEBS J.,* 2005, vol. 272, 4646-4659
- **LEGRAND, D. ; VIGIE, K. ; SAID, E. A. ; ELASS, E. ; MASSON, M. ; SLOMIANNY, M. C. ; CARPENTIER, M. ; BRIAND, J. P. ; MAZURIER, J. ; HOVANESSIAN, A. G.** Surface nucleolin participates in both the binding and endocytosis of lactoferrin in target cells. *Eur. J. Biochem.,* 2004, vol. 271, 303-317
- **NISOLE, S. ; KRUST, B. ; DAM, E. ; BLANCO, A. ; SEDDIKI, N. ; LOAEC, S. ; CALLEBAUT, C ; GUICHARD, G. ; MULLER, S. ; BRIAND, J. P.** The HB-19 composé 5[Kpsi(CH2N)PR]-TASP inhibits attachment of T lymophocyte- and macrophage-tropic HIV to permissive cells. *AIDS Res. Hum. Retroviruses,* 2000, vol. 16, 237-249
- **NISOLE, S. ; SAID, E. A. ; MISCHE, C. ; PREVOST, M. C. ; KRUST, B. ; BOUVET, P. ; BIANCO, A. ; BRIAND, J. P. ; HOVANESSIAN, A. G.** The anti-HIV pentameric composé HB-19 binds the C-terminal end of nucleolin and prevents anchorage of virus particles in the plasma membrane of target cells. *J. Biol. Chem.,* 2002, vol. 277, 20877-20886
- **BATES, P. J. ; KAHLON, J. B. ; THOMAS, S. D. ; TRENT, J. O. ; MILLER, D. M.** Antiproliferative activity of G-rich oligonucleotides correlates with protein binding. *J. Biol. Chem.,* 1999, vol. 274, 26369-26377

- **HUANG Y ; SHI H ; ZHOU H ; SONG X ; YUAN S ; LUO Y.** The angiogenesis function of nucleolin is mediated by vascular endothelial growth factor and non-muscle myosin. *Blood,* 2006, vol. 107, 3564-3571
- **SEKO Y ; COLE S ; KASPRZAK W ; SHAPIRO BA ; RAGHEB JA.** The role of cytokine mRNA stability in the pathogenesis of autoimmune disease. *Autoimmun Rev,* 2006, vol. 5, 299-305
- **ROT A.** Neutrophil attractant/activation protein-1 (intrleukin-8) induces in vitro neutrophil migration by haptotactic mechanism. *Eur J Immunol,* 1993, vol. 23, 303-06
- **ELASS E ; MASSON M ; MAZURIER J ; LEGRAND D.** Lactoferrin inhibits the lipposaccharaide-induced expression and proteoglycan-binding ability of IL-8 in human entothelial cells. *Infect Immun,* 2002, vol. 70, 1860-66
- **KEVIL CG ; PATEL RP ; BULLARD DC.** Essential role of ICAM-1 in mediating monocyte adhesion to aortic endothelial cells. *Am J Physiol Cell Physiol,* 2001, vol. 281, C1442-47
- **BUCKLIN SE ; SILVERSTEIN R ; MORRISON DC.** An interleukin-6-induced acute-phase response does not confer protection against lipopolysaccharide lethality. *Infect Immun,* 1993, vol. 61, 3184-89
- **KARIMA R ; MATSUMOTO S ; HIGASHI H ; MATSUSHIMA K.** The molecular pathogenesis of endotoxic shock and organ failure. *Mol Med Today,* 1999, vol. 5, 123-32
- **ZANOTTI S ; KULMAR A ; KUMAR A.** Cytokine modulation in sepsis and septic shock. *Expert Opin Investig Drugs,* 2002, vol. 11, 1061-75
- **ALTER, P ; HOESCHEN, J. ; RITTER, M. ; MAISCH, B.** Usefulness of cytokines interleukin-6 and interleukin-2R concentrations in diagnosing active infective endocarditis involving native valves. *Am. J. Cardiol.,* 2002, vol. 89, 1400-1404
- **SHUN, C.T. ; LU, S.Y. ; YEH, C.Y. ; CHAING, C.P. ; CHIA, J.S. ; CHEN, J.Y.** Glucosyltransferases of viridans streptococci are modulins of interleukin-6 induction in infective endocarditis. *Infec. Immun.,* 2005, vol. 73, 3261-3270
- **KANNAN K ; ORTMANN RA ; KIMPEL D.** Animal models of rheumatoid arthritis and their relevance to human disease. *Pathophysiology,* 2005, vol. 12, 167-81
- **PEIFER C ; WAGNER G ; LAUFER S.** New approaches to the treatment of inflammatory disorders small molecule inhibitors of p38 MAP kinase. *Curr Top Med Chem,* 2006, vol. 6 (2), 113-49
- **KABBINAVAR, F. ; HURWITZ, H. I. ; FEHRENBACHER, L. ; MEROPOL, N. J. ; NOVOTNY, W. F. ; LIEBERMAN, G. ; GRIFFING, S. ; BERGSLAND, E.** Phase II, randomized trial comparing bevacizumab plus fluorouracil (FU)/leucovorin (LV) with FU/LV alone in patients with metastatic colorectal cancer. *J Clin Oncol,* 2003, vol. 21, 60-65
- **HURWITZ, H. I.** New agents in colon cancer. *Clin Adv Hematol Oncol,* 2003, vol. 1, 404-405
- **FOURNEL, S. et al.** C3-symmetric peptide scaffolds are functional mimetics of trimeric CD40L. *Nat Chem Biol,* 2005, vol. 1, 377-382
- **KRUST B. et al.** The anti-HIV pentameric pseudopeptide HB-19 is preferentially taken up in vivo by lymphoid organs where it forms a complex with nucleolin. *PNAS,* 2001, vol. 98 (24), 14090-14095